(19) Europäisches Patentamt
European Patent Office
Office européen des brevets

(11) **EP 4 205 767 A1**

(12) **EUROPEAN PATENT APPLICATION**
published in accordance with Art. 153(4) EPC

(43) Date of publication:
**05.07.2023 Bulletin 2023/27**

(21) Application number: **21860418.9**

(22) Date of filing: **25.08.2021**

(51) International Patent Classification (IPC):
***A61K 47/64*** (2017.01) ***A61K 47/62*** (2017.01)
***A61P 35/00*** (2006.01) ***C08G 69/10*** (2006.01)
***A61K 47/60*** (2017.01) ***A61K 31/13*** (2006.01)
***A61K 31/195*** (2006.01)

(52) Cooperative Patent Classification (CPC):
**A61K 47/60; A61K 31/13; A61K 31/195; A61K 47/62; A61K 47/64; A61P 35/00**

(86) International application number:
**PCT/CN2021/114474**

(87) International publication number:
**WO 2022/042583 (03.03.2022 Gazette 2022/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.08.2020 CN 202010862995**

(71) Applicants:
- **Shanghai Senhui Medicine Co., Ltd.**
  **Shanghai 201203 (CN)**
- **Shanghai Shengdi Pharmaceutical Co., Ltd**
  **Shanghai 201210 (CN)**
- **Jiangsu Hengrui Pharmaceuticals Co., Ltd.**
  **Lianyungang, Jiangsu 222047 (CN)**

(72) Inventors:
- **HUANG, Jian**
  **Shanghai 201203 (CN)**
- **ZHU, Lingjian**
  **Shanghai 201203 (CN)**
- **GUAN, Zhongjun**
  **Shanghai 201203 (CN)**
- **LIANG, Shaonan**
  **Lianyungang, Jiangsu 222047 (CN)**
- **REN, Wenming**
  **Lianyungang, Jiangsu 222047 (CN)**
- **LIAO, Cheng**
  **Lianyungang, Jiangsu 222047 (CN)**

(74) Representative: **Regimbeau**
**20, rue de Chazelles**
**75847 Paris Cedex 17 (FR)**

# (54) DRUG-LOADED MACROMOLECULE AND PREPARATION METHOD THEREFOR

(57) A drug-loaded macromolecule and a preparation method therefor, the macromolecule specifically being a dentritic polymer loaded with a drug and a pharmacokinetic modifier, and relating in particular to connecting the drug to the dentritic polymer by means of a specific linker. The present macromolecule can be used to regulate the release speed of the drug, specifically by means of the selection of linker.

EP 4 205 767 A1

## Description

### TECHNICAL FIELD

The present disclosure belongs to the field of pharmaceutics, and relates to a dendrimer loaded with a drug and a pharmacokinetic modifier, and particularly to linking a drug to a dendrimer by a specific linker.

### BACKGROUND

Although drug research and development has now advanced considerably, it is still difficult to prepare suitable formulations of many drugs for administration in clinical trials due to their physical properties (such as solubility), or the formulations fail due to toxic effects that occur in the high-drug-concentration stage or poor therapeutic indexes after administration. In addition, the disadvantages of the formulations also include, for example, poor absorption, low bioavailability, poor *in vivo* stability, systemic side effects due to poor targeting, and inability to control their biodistribution, metabolism and renal or hepatic clearance in the body after administration. As drug research has advanced rapidly, some new areas of research have been opened up and some technical approaches with great potential have been developed to facilitate drug development. These approaches include, for example, formulating drug agents in liposomes, micelles or polymeric micelle formulations, covalently attaching drug agents to the backbones of hydrophilic polymers, etc. While these approaches can solubilize pharmaceutically active agents and in certain cases improve bioavailability and targeting, there is difficulty in the release of pharmaceutically active agents. In certain cases, the carrier degrades rapidly to release the pharmaceutically active agent before a drug molecule reaches the target organ. In many cases, the rate of release of a pharmaceutically active agent from the carrier is variable, thereby rendering the drug incapable of achieving a therapeutically effective dose *in vivo* or in the target organ.

In recent years, significant advances with respect to dendrimers have been made in biotechnology and drug application (Xiangyang Shi et al., Sci China Mater, 2018, 61(11), 1387-1403). Dendrimers are a specific class of polymers that have dense branched structures. They are tree-like macromolecules resulting from repeated outward branching of a core molecule. In other words, a branch of the core grows to a certain length and then splits into two branches, and this repeats until the molecule becomes so dense that it forms a spherical bush (V Gajbhiye et al., Journal of Pharmacy and Pharmacology, 2009, 61, 989-1003). Dendrimers are characterized by having a higher concentration of functional groups/unit molecular volume than common polymers. In particular, the unique properties of dendrimers, such as their high degrees of branching, multivalency, spherical structures and well-determined molecular weights, make them promising to be used for new scaffolds for drug delivery. Over the last decade, there has been increasing interest in research on the design and synthesis of biocompatible dendrimers and their application to many areas of bioscience, including drug delivery.

Starpahrma Inc. from Australia uses its own developed dendritic polylysine to perform loading and transport of anti-cancer drugs to strengthen the pharmacological properties of the drugs, ensuring that the drugs are delivered to appropriate body parts in due course. This approach is called "drug delivery", and this technique is launched under the trademark DEP®. DEP®-docetaxel, DEP®-cabazitaxel and DEP®-irinotecan are 3 major anti-cancer drugs developed by the company using the DEP® technique, which are now in the clinical research stage and hold great promise.

CN103796684A discloses macromolecules obtained by linking drugs to dendrimers by diacid linkers, especially by saturated branched or linear C1-C10 diacid linkers interrupted by oxygen, nitrogen or sulfur atoms.

### SUMMARY

The present disclosure provides a macromolecule, which comprises:

i) a dendrimer D having surface amino groups, wherein at least two different terminal groups are covalently linked to the surface amino groups of the dendrimer;
ii) a first terminal group, which is a pharmaceutically active agent comprising a hydroxy, amino or sulfhydryl group or is a residue A thereof; and
iii) a second terminal group, which is a pharmacokinetic modifier;

wherein the first terminal group is covalently linked to a surface amino group of the dendrimer by a linker $-X_1-L-X_2-$; $X_1$ is a linking group of the linker to the pharmaceutically active agent or residue A thereof, $X_2$ is a linking group of the linker to dendrimer D, and $X_1$ and $X_2$ are both -C(O)-; L is a linear or branched $C_{1-10}$ alkylene, wherein the linear or branched $C_{1-10}$ alkylene is substituted with one or more substituents selected from the group consisting of deuterium, hydroxy, $C_{3-7}$ cycloalkyl, $C_{3-7}$ cycloalkylene, $C_{1-6}$ alkoxy, haloalkyl, haloalkoxy, halogen, nitro, cyano, acyl, sulfhydryl, thioether group, sulfinyl, sulfonyl, $-NR_1R_2$, aryl, heteroaryl and heterocyclyl;

$R_1$ and $R_2$ are each independently selected from the group consisting of hydrogen, hydroxy, $C_{1-6}$ alkyl, cycloalkyl and $C_{1-6}$ alkoxy.

In some embodiments, L is linear or branched $C_{1-10}$ alkylene, wherein the linear or branched $C_{1-10}$ alkylene is substituted with one or more substituents selected from the group consisting of deuterium, halogen, $-OR_1$, $-SR_1$, $-NR_1R_2$ and $-C(O)R_3$;

wherein $R_1$ and $R_2$ are each independently selected from the group consisting of hydrogen, hydroxy, $C_{1-6}$ alkyl, $C_{3-7}$ cycloalkyl, $C_{1-6}$ alkoxy and $C(O)R_4$, and the $C_{1-6}$ alkyl, $C_{3-7}$ cycloalkyl and $C_{1-6}$ alkoxy are optionally substituted with one or more substituents selected from the group consisting of hydroxy, halogen, $C_{1-6}$ alkyl, $C_{1-6}$ alkoxy, $C_{2-6}$ alkenyl, $C_{2-6}$ alkynyl, $C_{1-6}$ haloalkyl, $C_{1-6}$ haloalkoxy, nitro, cyano, amino and $C_{1-6}$ alkylamino;

$R_3$ and $R_4$ are each independently selected from the group consisting of hydrogen, $C_{1-6}$ alkyl, $C_{1-6}$ haloalkyl and $C_{1-6}$ alkoxy.

In some embodiments, $R_1$ is not hydrogen.

In some embodiments, the linear or branched $C_{1-10}$ alkylene is substituted with one or more substituents selected from the group consisting of $-OR_1$, $-SR_1$ and $-NR_1R_2$, wherein $R_1$ and $R_2$ are each independently selected from the group consisting of hydrogen, $C_{1-6}$ alkyl, $C_{3-7}$ cycloalkyl, $C_{1-6}$ alkoxy and $C(O)R_4$, and the $C_{1-6}$ alkyl, $C_{3-7}$ cycloalkyl and $C_{1-6}$ alkoxy are optionally substituted with one or more substituents selected from the group consisting of hydroxy, halogen, $C_{1-6}$ alkyl, $C_{1-6}$ alkoxy, $C_{1-6}$ haloalkyl, $C_{1-6}$ haloalkoxy, amino and $C_{1-6}$ alkylamino; $R_4$ is selected from the group consisting of hydrogen, $C_{1-6}$ alkyl, $C_{1-6}$ haloalkyl and $C_{1-6}$ alkoxy.

In some embodiments, the linear or branched $C_{1-10}$ alkylene is substituted with one or more $-NR_1R_2$, wherein $R_1$ and $R_2$ are each independently selected from the group consisting of hydrogen, $C_{1-6}$ alkyl, $C_{3-7}$ cycloalkyl, $C_{1-6}$ alkoxy and $C(O)R_4$, and the $C_{1-6}$ alkyl, $C_{3-7}$ cycloalkyl and $C_{1-6}$ alkoxy are optionally substituted with one or more substituents selected from the group consisting of hydroxy, halogen, $C_{1-6}$ alkyl, $C_{1-6}$ alkoxy, $C_{1-6}$ haloalkyl, $C_{1-6}$ haloalkoxy, amino and $C_{1-6}$ alkylamino; $R_4$ is selected from the group consisting of hydrogen, $C_{1-6}$ alkyl, $C_{1-6}$ haloalkyl and $C_{1-6}$ alkoxy.

In some embodiments, the linear or branched $C_{1-10}$ alkylene is substituted with one or more $-NR_1R_2$, wherein $R_1$ and $R_2$ are each independently selected from the group consisting of hydrogen, $C_{1-6}$ alkyl and $C(O)R_4$, and $R_4$ is selected from the group consisting of hydrogen, $C_{1-6}$ alkyl, $C_{1-6}$ haloalkyl and $C_{1-6}$ alkoxy; preferably $R_1$ and $R_2$ are each independently selected from the group consisting of hydrogen and $C_{1-6}$ alkyl, and $R_1$ and $R_2$ are not both hydrogen.

In some embodiments, the linear or branched $C_{1-10}$ alkylene is linear or branched $C_{1-6}$ alkylene.

In some embodiments, in linker $-X_1-L-X_2-$, $X_1$ is -C(O)- and is linked to the pharmaceutically active agent or residue A thereof; $X_2$ is -C(O)- and is linked to a surface amino group of the dendrimer D to form an amide bond.

In some embodiments, the pharmaceutically active agent or residue A thereof comprises a hydroxy group, which forms an ester bond with X1.

In some specific embodiments, the macromolecule has a structure selected from the group consisting of:

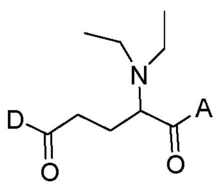 , 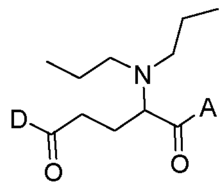 , 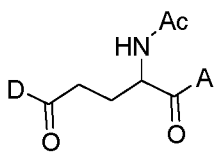 , 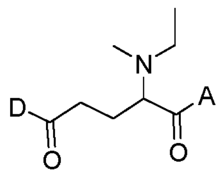 ,

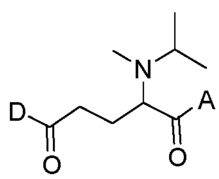 , 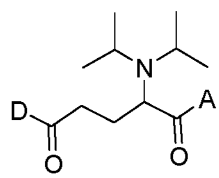 , 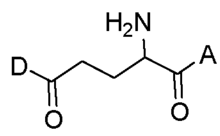 , 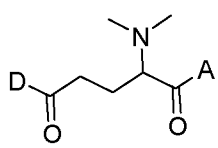

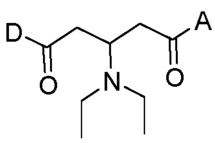 , 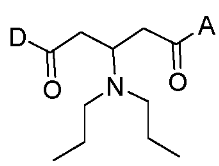 , 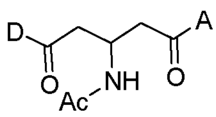 , 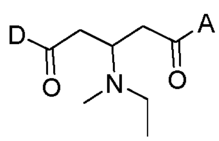 ,

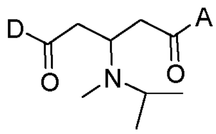 , 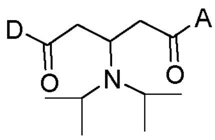 , 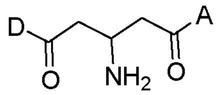 , 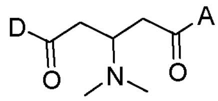

, 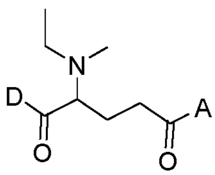 , 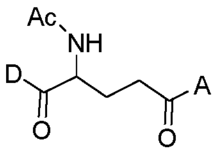 , 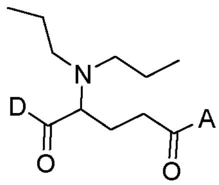 , 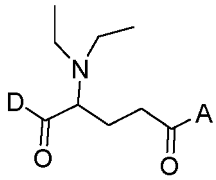

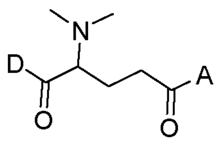 , 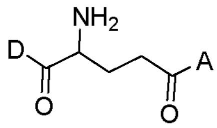 , 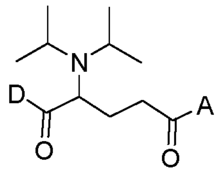 , 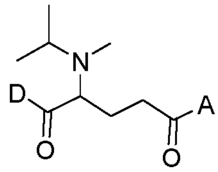

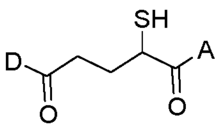 , 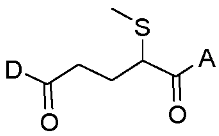 , 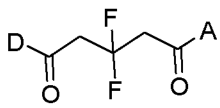 , 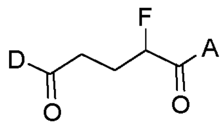 , ,

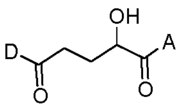 , 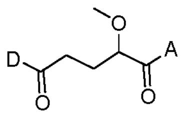 , 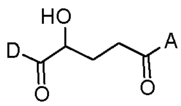 , 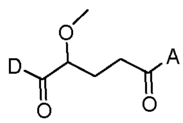

and

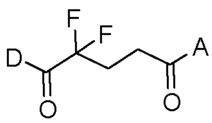 .

In some embodiments, the linker of the present disclosure can be selected to provide a desired rate of drug release, e.g., rapid release or slow release.

In some embodiments, the pharmaceutically active agent of the macromolecule is released faster, possibly at least 2 times faster, than is delivered independently of the macromolecule. In some embodiments, the pharmaceutically active agent of the macromolecule is released slower, possibly two, three, four, five, six, seven, eight, nine, ten or more, fifteen or more, twenty or more, thirty or more times slower, than is delivered independently of the macromolecule. A low-release-rate macromolecule is suitable for being formulated into a drug that enables a slow release over a long period of time, e.g., 1 week to 3 months, 1 month to 6 months, or more than 6 months. A rapid release is preferably a release of more than 50% of the pharmaceutically active agent over 0-8 h, especially 0-4 h, particularly 0-2 h, and more particularly 5-60 min. An intermediate release is preferably a release of more than 50% of the pharmaceutically active agent over 1-72 h, especially over 2-48 h. The rate of release of the pharmaceutically active agent can be controlled by selecting an appropriate linker. The rate of release also depends on the properties of the pharmaceutically active agent. In some embodiments, the pharmaceutically active agent is linked to the dendrimer by identical linkers. In some other embodiments, the pharmaceutically active agent is linked to the dendrimer by two or more types of linker, so that the pharmaceutically active agent can be released from the macromolecule at different rates.

In some embodiments, the first terminal group and the second terminal group are present in a ratio of 1:2 to 2:1, especially 1:2, 1:1 or 2:1. In some embodiments, the macromolecule comprises a third terminal group that is a blocking group, a drug or a targeting group. The blocking group may be an acyl group. In some embodiments, the first terminal group, the second terminal group and the third terminal group are in a ratio of 1:1:1 to 1:2:2, especially 1:2:1.

In some embodiments, at least 50% of terminal groups include one of the first terminal group and the second terminal group. In particular embodiments, the pharmaceutically active agent binds to more than 14%, 25%, 27%, 30%, 39%, 44% or 48% of the surface amino groups. In some embodiments, the pharmacokinetic modifier binds to more than 15%, 25%, 30%, 33% or 46% of the surface amino groups.

The pharmaceutically active agent of the present disclosure can be selected from the group consisting of anesthetics, antacids, antibodies, anti-infectives, biologics, cardiovascular drugs, contrast agents, diuretics, hematinics, immunosuppressants, hormones and analogs, nutraceuticals, ophthalmic drugs, pain-treating agents, respiratory drugs, adjuvants, anabolics, antiarthritics, anticonvulsants, antihistamines, anti-inflammatory drugs, antiulcer drugs, behavior-modifying drugs, oncology drugs, central nervous system drugs, contraceptives, diabetes-treating drugs, fertility drugs, growth promoters, hemostatics, immunostimulants, muscle relaxants, obesity-treating agents, osteoporosis drugs, peptides, sedatives and tranquilizers, urinary tract acidifiers and vitamins.

In some embodiments, the pharmaceutically active agent is an oncology drug, steroid, opioid analgesic, respiratory drug, central nervous system (CNS) drug, hypercholesterolemia drug, antihypertensive drug, antibacterial agent, immunosuppressive drug, antibiotic, luteinizing hormone-releasing hormone (LHRH) agonist, LHRH antagonist, antiviral drug, antiretroviral drug, estrogen receptor modulator, somatostatin analog, anti-inflammatory drug, vitamin D2 analog, synthetic thyroxine, antihistamine, antifungal agent or non-steroidal anti-inflammatory drug (NSAID), preferably an oncology drug.

In some embodiments, the oncology drug includes taxanes (such as paclitaxel, cabazitaxel and docetaxel), camptothecins and analogs thereof (such as irinotecan and topotecan), nucleosides (such as gemcitabine, cladribine, fludarabine, capecitabine, decitabine, azacitidine, clofarabine and nelarabine), kinase inhibitors (such as dasatinib (sprycel), temsirolimus (temisirolimus), AZD6244, AZD1152, PI-103, R-roscovitine, olomoucine and purvalanol A) and epothilone B analogs (such as ixabepilone), anthracyclines (anthrocyclines) (such as amrubicin, doxorubicin, epirubicin and valrubicin), ecteinascidin derivatives (such as trabectedin (trabectecin) and lurbinectedin), proteasome inhibitors (such as bortezomib) and other topoisomerase inhibitors, intercalators and alkylating agents, microtubule inhibitors (such as eribulin and vinflunine), etc., or structural modifications of these drug molecules.

In some embodiments, the pharmaceutically active agent is selected from the group consisting of taxanes, camptothecin derivatives, nucleosides, anthracyclines, ecteinascidin derivatives, proteasome inhibitors, microtubule inhibitors, BCL-2 inhibitors, BCL-$X_L$ inhibitors, selective inhibitor of nuclear export, antimetabolites, tyrosine kinase inhibitors, PLK1 inhibitors, CDK4/6 inhibitors, BTK inhibitors, non-steroidal hormone receptor antagonists and steroids, preferably from the group consisting of taxanes, camptothecin derivatives, BCL-2 inhibitors and BCL-$X_L$ inhibitors.

In some embodiments, the pharmaceutically active agent is selected from the group consisting of docetaxel, irinotecan, gemcitabine, capecitabine, decitabine, azacitidine, doxorubicin, epirubicin, trabectedin, lurbinectedin, bortezomib, eribulin, selinexor, venetoclax, tesetaxel, pemetrexed, cabazitaxel, cabozantinib, onvansertib, compound 2 and compound 3 below, and structural modifications of these drug molecules,

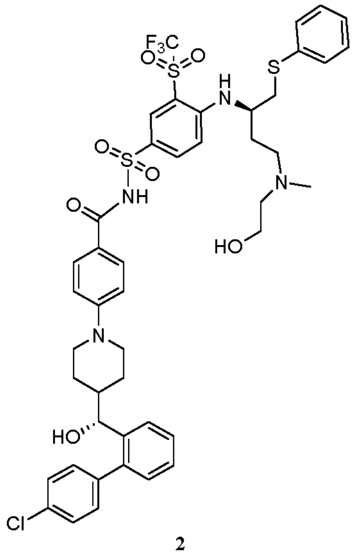

2

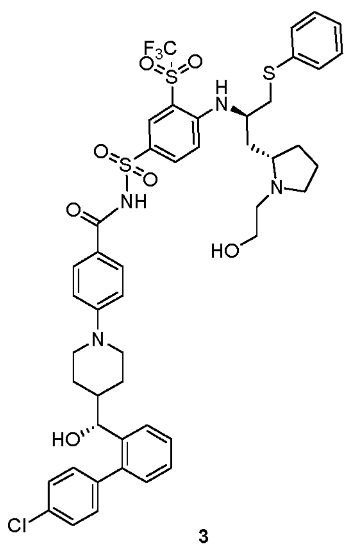

3

.

In some embodiments, the steroids include synthetic steroids (such as testosterone, dihydrotestosterone, and ethinyl estradiol) and corticosteroids (such as cortisone, prednisolone (prednisilone), budesonide, triamcinolone, fluticasone, mometasone, amcinonide, fluocinolone (flucinolone), fluocinonide (fluocinanide), desonide, halcinonide, prednicarbate, fluocortolone, dexamethasone, betamethasone and fluprednidene (fluprednidine)).

In some embodiments, the opioid analgesic includes morphine, oxymorphone, naloxone, codeine, oxycodone, methylnaltrexone, hydromorphone, buprenorphine and etorphine. In some embodiments, the respiratory drug includes bronchodilators, inhaled steroids and decongestants and particularly salbutamol, ipratropium bromide, montelukast and formoterol. In some embodiments, the CNS drug includes antipsychotics (such as quetiapine) and antidepressants (such as venlafaxine). In some embodiments, the hypercholesterolemia drug includes ezetimibe and statins such as simvastatin, lovastatin, atorvastatin, fluvastatin, pitavastatin, pravastatin and rosuvastatin. In some embodiments, the antihypertensive drug includes losartan, olmesartan, medoxomil, metoprolol (metrolol), travoprost and bosentan. In some embodiments, the immunosuppressive drug includes glucocorticoids, cytostatics, antibody fragments, anti-immunophilins, interferons, TNF binding proteins and particularly calcineurin (cacineurin) inhibitors such as tacrolimus, mycophenolic acid and derivatives thereof such as mycophenolate mofetil and cyclosporine. In some embodiments, the antibacterial agent includes antibiotics such as amoxicillin, meropenem, and clavulanic acid. In some embodiments, the LHRH agonist includes goserelin acetate, deslorelin and leuprorelin. In some embodiments, the LHRH antagonist includes cetrorelix, ganirelix, abarelix and degarelix. In some embodiments, the antiviral agent includes nucleoside analogs such as lamivudine, zidovudine, abacavir and entecavir, and the antiretroviral drug includes protease inhibitors such as atazanavir, lapinavir and ritonavir. In some embodiments, the estrogen receptor modulator includes raloxifene and fulvestrant. In some embodiments, the somatostatin analog includes octreotide. In some embodiments, the anti-inflammatory drug includes mesalazine and suitable NSAIDs include paracetamol (acetaminophen). In some embodiments, the vitamin D2 analog includes paricalcitol. In some embodiments, the synthetic thyroxine includes levothyroxine. In some embodiments, the antihistamine includes fexofenadine. In some embodiments, the antifungal agent includes azoles such as voriconazole (viriconazole).

In some embodiments, the pharmaceutically active agent is eribulin. In some embodiments, the pharmaceutically active agent is docetaxel. In some embodiments, the pharmaceutically active agent is trabectedin. In some embodiments, the pharmaceutically active agent is lurbinectedin.

In some embodiments, the pharmaceutically active agent is sparingly soluble or insoluble in aqueous solution.

The second terminal group is a pharmacokinetic modifier that can modify or modulate the pharmacokinetic properties of the pharmaceutically active agent or macromolecule, including absorption, distribution, metabolism and/or excretion. In particular embodiments, the pharmacokinetic modifier prolongs the plasma half-life of the pharmaceutically active agent, such that the pharmaceutically active agent linked to the macromolecule has a longer half-life than the pharmaceutically active agent alone or the pharmaceutically active agent on a non-dendrimer carrier. Preferably, the macromolecule or composition has a half-life that is at least 3 times longer, more preferably, at least 11 times longer, than that of the pharmaceutically active agent alone or the pharmaceutically active agent on a non-dendrimer carrier.

The pharmacokinetic modifier can be selected from the group consisting of polyethylene glycol, polyethyloxazoline, polyvinylpyrrolidone, polypropylene glycol, a folate and a folate derivative of a ligand for a cell surface receptor. In some embodiments, the pharmacokinetic modifier is polyethylene glycol. In some embodiments, the polyethylene glycol has a molecular weight in the range of 220-5500 Da; for example, the molecular weight can be 220-2500 Da, 570-2500 Da, 220-1100 Da, 570-1100 Da, 1000-5500 Da, 1000-2500 Da or 1000-2300 Da. In some embodiments, the pharmacokinetic modifier forms an amide bond with a surface amino group of the dendrimer.

The targeting group is an agent that binds to a biological target cell, organ or tissue with some selectivity, thereby helping to direct the macromolecule to a particular target in the body and allowing it to accumulate in that target cell, organ or tissue. In addition, the targeting group can provide a mechanism for the macromolecule to be actively taken into the cell or tissue by receptor-mediated endocytosis. Particular examples include lectins and antibodies and other ligands (including small molecules) for cell surface receptors. The interaction may occur through any type of bonding or association (including covalent, ionic and hydrogen bonding and Van der Waals forces). Suitable targeting groups include those that bind to cell surface receptors, for example, the folate receptor, adrenergic receptor, growth hormone receptor, luteinizing hormone receptor, estrogen receptor, epidermal growth factor receptor, fibroblast growth factor receptor (e.g., FGFR2), IL-2 receptor, CFTR and vascular epithelial growth factor (VEGF) receptor.

In some embodiments, the targeting group is luteinizing hormone-releasing hormone (LHRH) or a derivative thereof that binds to a luteinizing hormone-releasing hormone receptor. In some embodiments, the targeting group is LYP-1, a peptide that targets the lymphatic system of tumors but not the lymphatic system of normal tissue. In some embodiments, the targeting group may be an RGD peptide. RGD peptides are peptides comprising a sequence of -Arg-Gly-Asp-, which is the primary integrin recognition site in extracellular matrix proteins. In some embodiments, the targeting group may be folic acid. Estrogens can also be used for target cells expressing estrogen receptors.

In some embodiments, the targeting group can bind to the dendrimer core directly or preferably through a linking group. The linking group may be any divalent group capable of binding to the functional group of the core and the functional group on the targeting group.

The macromolecule of the present disclosure comprises a dendrimer in which the outermost generation of the structural unit has surface amino groups. The properties of the dendrimer of the macromolecule are not particularly

important, provided that it has surface amino groups. For example, the dendrimer can be a polylysine, polylysine analog, polyamidoamine (PAMAM), polyethyleneimine (PEI) or polyether hydroxylamine (PEHAM) dendrimer. In some embodiments, the dendrimer is a polylysine or a polylysine analog. The polylysine or polylysine analog comprises a core and 2-7 generations of lysine or a lysine analog, e.g., 2, 3, 4, 5, 6 or 7 generations of lysine or a lysine analog.

In some embodiments, the lysine has the structure shown in 1:

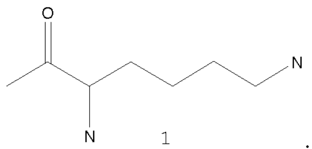

1 .

In some embodiments, the lysine analog has the structure shown in 2:

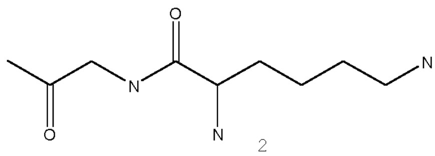

2 .

In some embodiments, the lysine analog has the structure shown in 3:

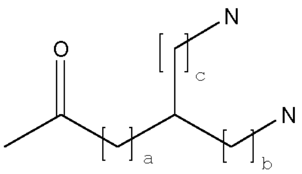

3 ,

wherein a is 1 or 2; b and c are identical or different and are integers from 1 to 4.

In some embodiments, the lysine analog has the structure shown in 4:

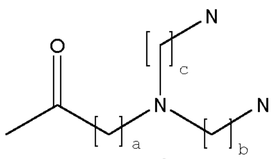

4 ,

wherein a is an integer from 0 to 2; b and c are identical or different and are integers from 2 to 6.

In some embodiments, the lysine analog has the structure shown in 5:

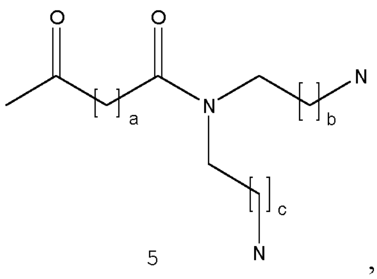

5 ,

wherein a is an integer from 0 to 5; b and c are identical or different and are integers from 1 to 5.

In some embodiments, the lysine analog has the structure shown in 6:

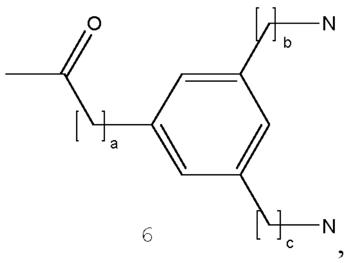

6

wherein a is an integer from 0 to 5; b and c are identical or different and are integers from 0 to 5.

In some embodiments, the lysine analog has the structure shown in 7:

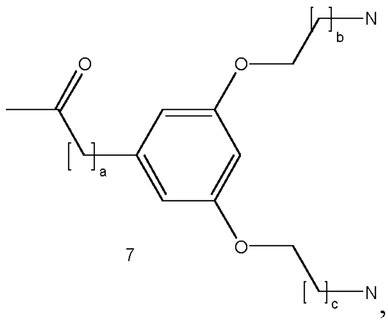

7

wherein a is an integer from 0 to 5; b and c are identical or different and are integers from 1 to 5.

In some embodiments, the lysine analog has the structure shown in 8:

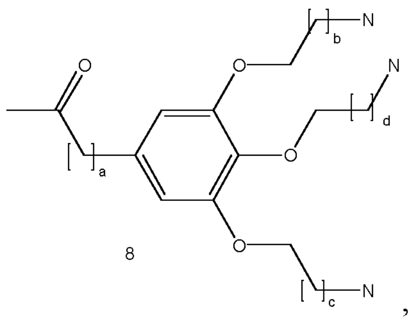

8

wherein a is an integer from 0 to 5; b, c and d are identical or different and are integers from 1 to 5.

In some embodiments, the lysine analog has the structure shown in 9:

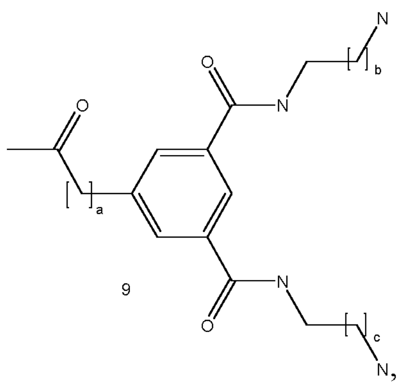

9

wherein a is an integer from 0 to 5; b and c are identical or different and are integers from 1 to 5.

The core of the dendrimer, in particular the polylysine or polylysine analog described in the present disclosure, can be selected from the group consisting of benzhydrylamine (BHA), a benzhydrylamide of lysine (BHALys) or a lysine analog or:

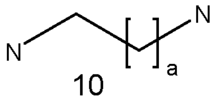

10 ,

wherein a is an integer from 1 to 9, preferably from 1 to 5;

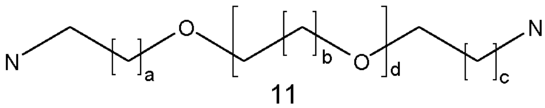

11 ,

wherein a, b and c may be identical or different and are integers from 1 to 5, and d is an integer from 0 to 100, preferably from 1 to 30;

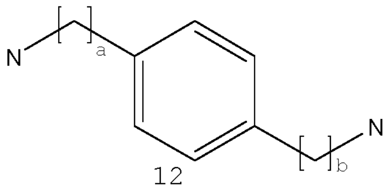

12 ,

wherein a and b may be identical or different and are integers from 0 to 5;

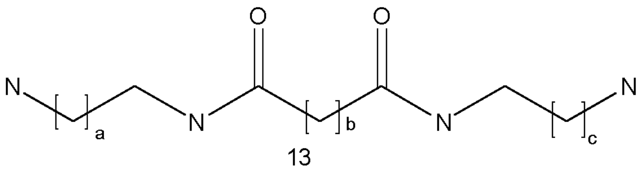

13 ,

wherein a and c may be identical or different and are integers from 1 to 6, and b is an integer from 0 to 6;

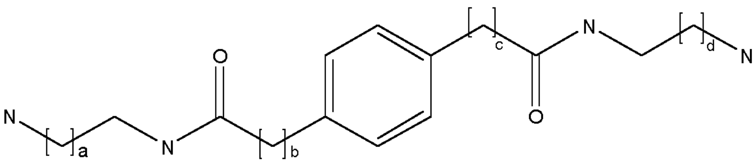

14 ,

wherein a and d may be identical or different and are integers from 1 to 6, and b and c may be identical or different and are integers from 0 to 6;

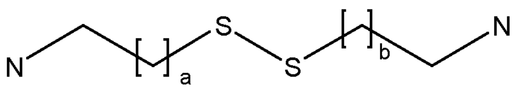

15 ,

wherein a and b are identical or different and are integers from 1 to 5, particularly from 1 to 3, and are especially 1;

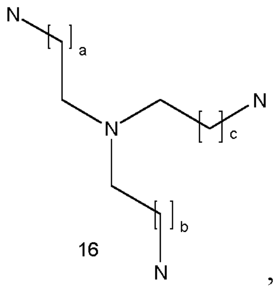

16 ,

wherein a, b and c are identical or different and are integers selected from the group consisting of 1 to 6;

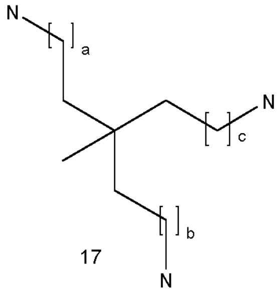

17 ,

wherein a, b and c are identical or different and are integers selected from the group consisting of 0 to 6;

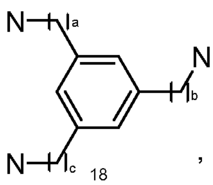

18 ,

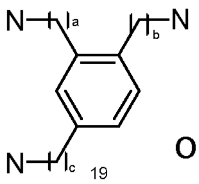

19 or

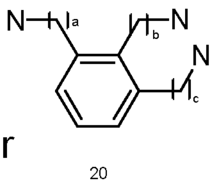

20 ,

wherein a, b and c are identical or different and are integers selected from the group consisting of 0 to 6;

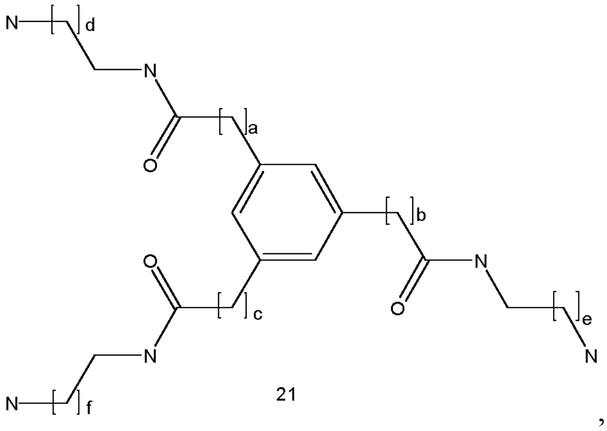

21 ,

wherein a, b and c may be identical or different and are integers from 0 to 6, and d, e and f may be identical or different and are integers from 1 to 6;

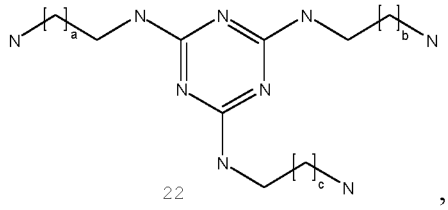

22 ,

wherein a, b and c may be identical or different and are integers from 1 to 6;

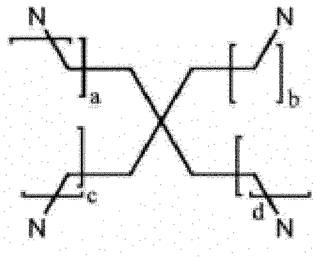

23,

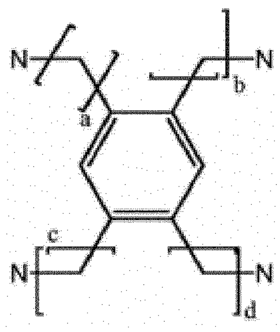

24,

wherein a, b, c and d may be identical or different and are integers from 0 to 6;

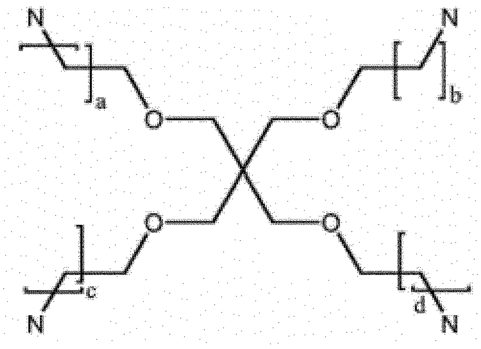

25,

wherein a, b, c and d may be identical or different and are integers from 1 to 6; or

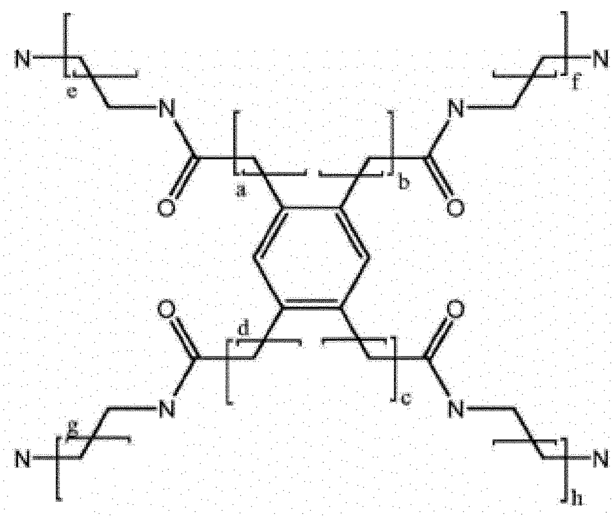

26,

wherein a, b, c and d may be identical or different and are integers from 0 to 6, and e, f, g and h may be identical or

different and are integers from 1 to 6.

In certain embodiments, the macromolecule comprises:

i) a dendrimer D having surface amino groups, wherein at least two different terminal groups are covalently linked to the surface amino groups of the dendrimer;

ii) a first terminal group, which is a pharmaceutically active agent comprising a hydroxy, amino or sulfhydryl group or is a residue A thereof; and

iii) a second terminal group, which is a pharmacokinetic modifier polyethylene glycol; wherein the first terminal group is covalently linked to a surface amino group of the dendrimer by a linker selected from the group consisting of

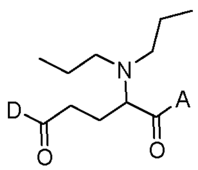 , 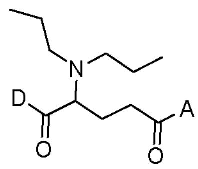 , 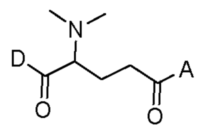 , 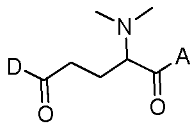 ,

The dendrimer D is selected from the group consisting of BHALys$[Lys]_{16}$, BHALys$[Lys]_{32}$ and BHALys$[Lys]_{64}$.

The polyethylene glycol has a molecular weight in the range of 1000 to 2500 Da.

The present disclosure also relates to a pharmaceutical composition, which comprises the macromolecule of the present disclosure and a pharmaceutically acceptable carrier. In some embodiments, the pharmaceutical composition is free of solubilizing excipients, such as polyethoxylated castor oils and polysorbates. In some embodiments, the pharmaceutical composition is administered transdermally, orally, by injection, etc.

The macromolecule of the present disclosure is formulated in compositions including those suitable for oral, rectal, topical, nasal, inhalation, aerosol, ophthalmic or parenteral (including intraperitoneal, intravenous, subcutaneous or intramuscular injection) administration. The composition can be conveniently presented in unit dosage form and can be prepared using any of the methods well known in the art of pharmacy. All methods include the step of bringing the macromolecule into association with a carrier that constitutes one or more accessory ingredients. In general, the composition is prepared by bringing the macromolecule into association with a liquid carrier to form a solution or a suspension, or bringing the macromolecule into association with formulation components suitable for forming a solid, optionally a particulate product, and then, if necessary, shaping the product into a desired delivery form. The solid formulation of the present disclosure, when particulate, will typically comprise particles with sizes ranging from about 1 nanometer to about 500 microns. In general, for solid formulations intended for intravenous administration, particles will typically range from about 1 nm to about 10 microns in diameter. The composition may comprise the macromolecule of the present disclosure which is a nanoparticle having a particle diameter of less than 1000 nm, for example, 5 to 1000 nm, particularly 5 to 500 nm, especially 5 to 400 nm (such as 5 to 50 nm and particularly 5 to 20 nm). In particular embodiments, the composition comprises macromolecules with a mean size of 5 to 20 nm. In some embodiments, the macromolecule is polydispersed in the composition, with PDI of between 1.01 and 1.8, particularly between 1.01 and 1.5, especially between 1.01 and 1.2. In particular embodiments, the macromolecule is monodispersed in the composition. Particularly preferred are sterile, lyophilized compositions, which are reconstituted in an aqueous vehicle prior to injection.

In some embodiments, the composition comprises macromolecules with a mean size of 5 to 20 nm. In some embodiments, the macromolecules have a particle size $D_{90}$ or $D_{50}$ of less than 1000 nm, e.g., 5 to 1000 nm, particularly 5 to 500 nm, especially 5 to 400 nm (e.g., 5 to 50 nm, particularly 5 to 20 nm). In particular embodiments, the composition comprises macromolecules having a $D_{50}$ of 5 to 20 nm.

The macromolecule of the present disclosure can also be used to provide controlled-release and/or sustained-release formulations of the pharmaceutically active agent. In a sustained-release formulation, formulation ingredients are selected to release the macromolecule from the formulation over a prolonged period of time (e.g., days, weeks or months). Such formulations include transdermal patches or are in implantable devices that may be deposited subcutaneously or are injected intravenously, subcutaneously, intramuscularly, intraepidurally or intracranially. In controlled-release formulations, the diacid linker is selected to release a majority of its pharmaceutically active agent in a given time window. For example, when the time taken for a majority of the macromolecule to accumulate in a target organ, tissue or tumor is known, the linker may be selected to release a majority of its pharmaceutically active agent after the accumulation time has elapsed. This can allow a high drug load to be delivered at a given time point at the site where its action is desired. Alternatively, the linker is selected to release the pharmaceutically active agent at a therapeutic level over a prolonged period of time. In some embodiments, the formulation may have multiple controlled-release characteristics. For example, the formulation comprises macromolecules in which the drug is linked by different linkers; this allows a burst release of a fast-release drug followed by a slower release at low but constant therapeutic levels over a prolonged period of time. In some embodiments, the formulation may have sustained-release and controlled-release

characteristics. For example, the formulation ingredients may be selected to release the macromolecule over a prolonged period of time and the linker is selected to deliver a constant low therapeutic level of pharmaceutically active agent. In some embodiments, the pharmaceutically active agent is linked to the same molecule by different linkers. In some embodiments, each drug-linker combination is linked to different macromolecules in the same formulation.

In some embodiments, in the pharmaceutical composition, the macromolecule is formulated to release more than 50% of the pharmaceutically active agent in between 5 minutes to 60 minutes. In some embodiments, in the pharmaceutical composition, the macromolecule is formulated to release more than 50% of the pharmaceutically active agent in between 2 hours to 48 hours. In some embodiments, in the pharmaceutical composition, the macromolecule is formulated to release more than 50% of the pharmaceutically active agent in between 5 days to 30 days.

Another aspect of the present disclosure provides a method for treating or inhibiting tumor growth, which comprises administering an effective amount of the macromolecule or pharmaceutical composition of the present disclosure, wherein the pharmaceutically active agent of the first terminal group is an oncology drug. The tumor described in the present disclosure is selected from the group consisting of melanoma, brain tumor, esophageal cancer, gastric cancer, liver cancer, pancreatic cancer, colorectal cancer, lung cancer, kidney cancer, breast cancer, ovarian cancer, prostate cancer, skin cancer, neuroblastoma, sarcoma, osteochondroma, osteoma, osteosarcoma, seminoma, testicular tumor, uterine cancer, head and neck tumor, multiple myeloma, malignant lymphoma, polycythemia vera, leukemia, thyroid tumor, ureteral tumor, bladder cancer, gallbladder cancer, bile duct cancer, chorionic epithelioma and pediatric tumors (Ewing familial sarcoma, Wilms tumor, rhabdomyosarcoma, angiosarcoma, embryonal testicular cancer, neuroblastoma, retinoblastoma, hepatoblastoma, nephroblastoma, etc.).

In another aspect of the present disclosure, provided is a method for reducing hypersensitivity upon treatment with an oncology drug, which comprises administering the pharmaceutical composition of the present disclosure, wherein the composition is substantially free of solubilizing excipients such as Cremophor EL and polysorbate 80. In another aspect of the present disclosure, provided is a method for reducing the toxicity of an oncology drug or a formulation of an oncology drug, which comprises administering the macromolecule of the present disclosure, wherein the oncology drug is the first terminal group of the macromolecule. In some embodiments, the toxicity that is reduced is hematologic toxicity, neurological toxicity, gastrointestinal toxicity, cardiotoxicity, hepatotoxicity, nephrotoxicity, ototoxicity or encephalotoxicity.

In another aspect of the present disclosure, provided is a method for reducing side effects associated with an oncology drug or a formulation of an oncology drug, which comprises administering the macromolecule of the present disclosure, wherein the oncology drug is the first terminal group of the macromolecule. In some embodiments, the side effects that are reduced are selected from the group consisting of neutropenia, leukopenia, thrombocytopenia, myelotoxicity, myelosuppression, neuropathy, fatigue, non-specific neurocognitive problems, vertigo, encephalopathy, anemia, dysgeusia, dyspnea, constipation, anorexia, nail disorders, fluid retention, asthenia, pain, nausea, vomiting, mucositis, alopecia, skin reactions, myalgia and hypersensitivity.

In some embodiments, the macromolecule or pharmaceutical composition comprising the macromolecule of the present disclosure can reduce or eliminate the need for presurgical medication with agents such as corticosteroids and antihistamines.

Methods of preparing dendrimers are known in the art. For example, the dendrimer of the macromolecule can be prepared using a divergent method or a convergent method or a mixture thereof.

In the divergent method, each generation of structural units is sequentially added to the core or the previous generation. The surface generation having one or two surface amino groups is protected. If one of the amino groups is protected, the free amino group is reacted with one of the linker, the linker-pharmaceutically active agent and the pharmacokinetic modifier. If both amino groups are protected, they are protected with different protecting groups, one of which can be removed without removing the other. One of the amino protecting groups is removed and a reaction with one of the linker, the linker-pharmaceutically active agent and the pharmacokinetic modifier is conducted. Once the initial terminal group has been linked to the dendrimer, the other amino protecting group is removed and other first and second terminal groups are added. These groups are linked to the surface amino groups by amide formation that is known in the art.

In the convergent method, each generation of structural units is built on the previous generation to form a dendron. The first and second terminal groups can be linked to the surface amino groups as described above before or after linking of the dendron to the core.

In a mixed approach, each generation of structural units is added to the core or the previous generation of structural units. However, before the last generation is added to the dendrimer, the surface amino groups are functionalized with terminal groups (e.g., first and second terminal groups, first and third terminal groups or second and third terminal groups). The functionalized final generation is then added to the subsurface layer of structural units and the dendron is linked to the core.

The pharmaceutically active agent is reacted with one of the carboxylic acids of the linker by ester formation that is known in the art. For example, an activated carboxylic acid is formed. For example, an acid chloride or an anhydride

is used and reacted with the hydroxy group of the pharmaceutically active agent. If the pharmaceutically active agent has more than one hydroxy group, other hydroxy groups can be protected.

Where the targeting agent is linked to the core, a functional group on the core can be protected during formation of the dendrimer, then deprotected and reacted with the targeting agent, the linking group or the targeting agent-linking group. Alternatively, the core can be reacted with the linking group or the targeting agent-linking group before the dendrimer is formed.

Suitable protecting groups and methods of introducing and removing them are described in Greene&Wuts, Protecting Groups in Organic Synthesis, Third Edition, 1999.

The present disclosure also includes various deuterated forms of the macromolecule or pharmaceutically acceptable salts thereof, wherein each available hydrogen atom in the macromolecule can be independently replaced with a deuterium atom. Those skilled in the art will know how to synthesize deuterated forms of the macromolecule of the present disclosure or pharmaceutically acceptable salts thereof.

The present disclosure also includes macromolecules that are isotopically labeled. One or more atoms in the macromolecules are replaced by atoms with an atomic mass or mass number that is different from the most common atomic mass or mass number in nature. Examples of isotopes for the macromolecule of the present disclosure include isotopes of hydrogen, carbon, nitrogen, oxygen, fluorine, iodine, and chlorine, such as $^{3}H$, $^{11}C$, $^{14}C$, $^{18}F$, $^{123}I$ or $^{125}I$.

The macromolecule of the present disclosure can be present in the form of pharmaceutically acceptable salts. However, it will be appreciated that non-pharmaceutically acceptable salts also fall within the scope of the present disclosure as these may be useful as intermediates in the preparation of pharmaceutically acceptable salts or may be useful during storage or transport. Suitable pharmaceutically acceptable salts include, but are not limited to, salts of pharmaceutically acceptable inorganic acids (such as hydrochloric acid, sulphuric acid, phosphoric acid, nitric acid, carbonic acid, boric acid, sulfamic acid and hydrobromic acid), or salts of pharmaceutically acceptable organic acids (such as acetic acid, propionic acid, butyric acid, tartaric acid, maleic acid, hydroxymaleic acid, fumaric acid, citric acid, lactic acid, mucic acid, gluconic acid, benzoic acid, succinic acid, oxalic acid, phenylacetic acid, methanesulphonic acid, toluenesulphonic acid, benezenesulphonic acid, salicylic acid, sulphanilic acid, aspartic acid, glutamic acid, ethylenediaminetetraacetic acid, stearic acid, palmitic acid, oleic acid, lauric acid, pantothenic acid, tannic acid, ascorbic acid and valeric acid). Base salts include, but are not limited to, those formed with pharmaceutically acceptable cations (such as sodium, potassium, lithium, calcium, magnesium, ammonium and alkylammonium).

Unless otherwise stated, the following terms used in the specification and claims have the following meanings.

"Halogen" refers to fluorine, chlorine, bromine or iodine.

"Halo" means being substituted with one or more atoms selected from the group consisting of fluorine, chlorine, bromine and iodine.

"Alkyl" refers to a linear or branched saturated hydrocarbon group having 1 to 10 carbon atoms. Where appropriate, alkyl may have the specified number of carbon atoms, for example, $C_{1-4}$ alkyl which includes alkyl groups having 1, 2, 3 or 4 carbon atoms in a linear or branched arrangement. Examples of suitable alkyl groups include, but are not limited to, methyl, ethyl, n-propyl, isopropyl, n-butyl, isobutyl, tert-butyl, n-pentyl, 2-methylbutyl, 3-methylbutyl, 4-methylbutyl, n-hexyl, 2-methylpentyl, 3-methylpentyl, 4-methylpentyl, 5-methylpentyl, 2-ethylbutyl, 3-ethylbutyl, heptyl, octyl, nonyl and decyl.

"Alkylene" refers to a linear or branched divalent alkyl group having 1 to 10 carbon atoms.

The term "alkenyl" includes branched and linear alkenes having 2 to 12 carbon atoms or alkenes containing aliphatic hydrocarbon groups; if the number of carbon atoms is specified, the specified number is meant. For example, "$C_{2-6}$ alkenyl" refers to an alkenyl group having 2, 3, 4, 5 or 6 carbon atoms. Examples of alkenyl groups include, but are not limited to, ethenyl, allyl, 1-propenyl, 1-butenyl, 2-butenyl, 3-butenyl, 2-methylbut-2-enyl, 3-methylbut-1-enyl, 1-pentenyl, 3-pentenyl, and 4-hexenyl.

The term "alkynyl" includes branched and linear alkynyl having 2 to 12 carbon atoms or alkynyl containing aliphatic hydrocarbon groups, or alkynyl having a particular number of carbon atoms (if the particular number is specified), e.g., ethynyl, propynyl (e.g., 1-propynyl, 2-propynyl), 3-butynyl, pentynyl, hexynyl and 1-methylpent-2-ynyl.

"Alkenylene" and "alkynylene" refer to a partially unsaturated branched or linear divalent hydrocarbon group derived from alkenyl or alkynyl. In certain embodiments, such alkenylene groups are optionally substituted. Non-limiting examples of alkenylene groups include ethenylene, propenylene, butenylene, pentenylene, hexenylene, heptenylene, octenylene, nonenylene, decenylene, and the like; non-limiting examples of alkynylene groups include ethynylene, propynylene, butynylene, pentynylene, hexynylene, and the like.

"Cycloalkyl" refers to a saturated or unsaturated cyclic hydrocarbon. A cycloalkyl ring may include a specified number of carbon atoms. For example, a 3- to 8-membered cycloalkyl group includes 3, 4, 5, 6, 7 or 8 carbon atoms. Examples of suitable cycloalkyl groups include, but are not limited to, cyclopropyl, cyclobutyl, cyclopentanyl, cyclopentenyl, cyclohexanyl, cyclohexenyl, 1,4-cyclohexadienyl, cycloheptanyl and cyclooctanyl.

"Cycloalkylene" refers to a divalent cyclic hydrocarbon group derived from cycloalkyl, e.g.,

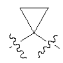 , 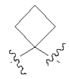 , 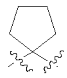 , 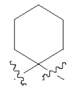,

etc.

The term "alkoxy" refers to -O-(alkyl) and -O-(unsubstituted cycloalkyl), wherein the alkyl is as defined above. Non-limiting examples of alkoxy groups include: methoxy, ethoxy, propoxy, butoxy, cyclopropyloxy, cyclobutoxy, cyclopentyloxy and cyclohexyloxy. The alkoxy may be substituted or unsubstituted. When substituted, the alkoxy may be substituted with one or more substituents preferably independently selected from the group consisting of H, D, halogen, alkyl, alkoxy, haloalkyl, hydroxy, hydroxyalkyl, cyano, amino, nitro, cycloalkyl, heterocyclyl, aryl and heteroaryl.

"Aryl" means any stable, monocyclic or bicyclic carbon ring of up to 7 atoms in each ring, wherein at least one ring is aromatic. Examples of such aryl groups include, but are not limited to, phenyl, naphthyl, tetrahydronaphthyl, indanyl, biphenyl and binaphthyl.

The term "heterocycloalkyl" or "heterocyclyl" refers to a cyclic hydrocarbon in which one to four carbon atoms have been replaced by heteroatoms independently selected from the group consisting of N, N(R), S, S(O), $S(O)_2$ and O. A heterocyclic ring may be saturated or unsaturated. Examples of suitable heterocyclyl groups include tetrahydrofuranyl, tetrahydrothienyl, pyrrolidinyl, pyrrolinyl, pyrazolinyl, pyranyl, piperidinyl, pyrazolinyl, dithiolyl, oxathiolyl, dioxanyl, dioxinyl, morpholino and oxazinyl.

"Heteroaryl" represents a stable monocyclic or bicyclic ring of up to 7 atoms in each ring, wherein at least one ring is aromatic and at least one ring contains 1 to 4 heteroatoms selected from the group consisting of O, N and S. Heteroaryl groups within the scope of this definition include, but are not limited to, acridinyl, carbazolyl, cinnolinyl, quinoxalinyl, quinazolinyl, pyrazolyl, indolyl, benzotriazolyl, furanyl, thienyl, thiophenyl, 3,4-propylenedioxythiophenyl, benzothienyl, benzofuranyl, benzodioxane, benzodioxin, quinolinyl, isoquinolinyl, oxazolyl, isoxazolyl, imidazolyl, pyrazinyl, pyridazinyl, pyridinyl, pyrimidinyl, pyrrolyl, tetrahydroquinoline, thiazolyl, isothiazolyl, 1,2,4-triazolyl, 1,2,3-triazolyl, 1,2,4-oxadiazolyl, 1,2,4-thiadiazolyl, 1,3,5-triazinyl, 1,2,4-triazinyl, 1,2,4,5-tetrazinyl and tetrazolyl.

"Dendrimer" refers to a molecule containing a core and at least one dendron linked to the core. Each dendron consists of at least one layer or generation of branched structural units, resulting in a branched structure with an increasing number of branches with each generation of structural units. The maximum number of dendrons linked to the core is limited by the number of functional groups on the core.

"Structural unit" refers to a branched molecule having at least three functional groups, one for linking to the core or the previous generation of structural units and at least two functional groups for linking to the next generation of structural units or forming the surface of the dendrimer.

"Generation" refers to the number of layers of structural units that constitute a dendron or dendrimer. For example, a one-generation dendrimer will have one layer of branched structural units linked to the core, for example, core-[[structural unit]]u, where u is the number of dendrons linked to the core. A two-generation dendrimer has two layers of structural units in each dendron linked to the core, and when the structural unit has one branching point, the dendrimer may be: core[[structural unit][structural unit]2]u. A three-generation dendrimer has three layers of structural units in each dendron linked to the core, for example, core-[[structural unit][structural unit]2[structural unit]4]u. A 6-generation dendrimer has six layers of structural units linked to the core, for example, core-[[structural unit][structural unit]2[structural unit]4[structural unit]8[structural unit] 16[structural unit]32]u, and the like. The last generation of structural units (the outermost generation) provides the surface functionalization of the dendrimer and the number of functional groups available for binding to terminal groups. For example, in a dendrimer having two dendrons linked to the core (u=2), if each structural unit has one branching point and there are 6 generations, the outermost generation has 64 structural units and 128 functional groups available for binding to terminal groups.

"Sparingly soluble" refers to a drug or pharmaceutically active agent that has a solubility in water of 1 mg/mL to 10 mg/mL. Drugs with a solubility in water of less than 1 mg/mL are considered insoluble.

"Solubilizing excipient" refers to a formulation additive that is used to solubilize an insoluble or sparingly soluble pharmaceutically active agent into an aqueous formulation. Examples include surfactants such as polyethoxylated castor oils including Cremophor EL, Cremophor RH40 and Cremophor RH60, D-α-tocopherol-polyethylene glycol 1000 succinate, polysorbate 20, polysorbate 80, solutol HS15, sorbitan monoleate, poloxamer 407, Labrasol, etc.

The term "optionally" or "optional" means that the event or circumstance subsequently described may, but not necessarily, occur, and that the description includes instances where the event or circumstance occurs or does not occur. For example, "L is a linear $C_{1-10}$ alkylene optionally interrupted by one or more oxygen, sulfur or nitrogen atoms" means that the linear $C_{1-10}$ alkylene can be, but not necessarily, interrupted by oxygen, sulfur or nitrogen atoms, and this description includes the case where the linear $C_{1-10}$ alkylene is interrupted by oxygen, sulfur or nitrogen atoms and the case where the linear $C_{1-10}$ alkylene is not interrupted by oxygen, sulfur or nitrogen atoms.

The term "substituted" means that one or more, preferably up to 5, more preferably 1 to 3 hydrogen atoms in

the group are independently substituted with a corresponding number of substituents. It goes without saying that a substituent is only in its possible chemical position, and those skilled in the art will be able to determine (experimentally or theoretically) possible or impossible substitution without undue effort.

In the chemical structure of the compound described herein, a "" bond is not specified with a configuration, that is, a "" bond may be "" or "", or includes both "" and " -"* " configurations. In the chemical structure of the compound described herein, a "" bond is not specified with a configuration-that is, it may be in a Z configuration or an E configuration, or includes both configurations.

Any isotopically-labeled derivative of the compound or the pharmaceutically acceptable salt or the isomer thereof described herein is encompassed by the present disclosure. Atoms that can be isotopically labeled include, but are not limited to, hydrogen, carbon, nitrogen, oxygen, phosphorus, fluorine, chlorine, iodine, etc. They may be replaced by isotopes $^{2}H(D)$, $^{3}H$, $^{11}C$, $^{13}C$, $^{14}C$, $^{15}N$, $^{18}F$, $^{31}P$, $^{32}P$, $^{35}S$, $^{36}Cl$, $^{125}I$, etc. Unless otherwise stated, when a position is specifically designated as deuterium (D), that position shall be understood to be deuterium having an abundance that is at least 3000 times greater than the natural abundance of deuterium (which is 0.015%) (i.e., incorporating at least 45% deuterium).

The pharmaceutically active agent or its residue, pharmaceutically active agent, and pharmaceutically active agent or its residue A are used interchangeably in the present disclosure and all refer to a molecule or group with pharmaceutical activity.

## BRIEF DESCRIPTION OF THE DRAWINGS

FIG. 1 shows PK profiles of drugs in the plasma of rats in Test Example 1.

FIG. 2 shows PK profiles in the plasma of beagle dogs in Test Example 2 (0-168 hours).

FIG. 3 shows PK profiles in the plasma of beagle dogs in Test Example 2 (0-24 hours).

FIG. 4 shows tumor volume growth curves of groups of mice in the subcutaneous tumor graft model of human lung cancer cell A549 in Test Example 3.

FIG. 5 shows PK profiles in the plasma of beagle dogs in Test Example 4.

FIG. 6 shows the therapeutic effect of the compound of Test Example 6 on subcutaneously grafted tumors in mice bearing human acute lymphoblastic leukemia RS4;11.

## DETAILED DESCRIPTION

The present disclosure is further described and explained below with reference to examples, but these examples are not intended to limit the scope of the present disclosure.

Experimental procedures without conditions specified in the examples of the present disclosure were generally conducted according to conventional conditions, or according to conditions recommended by the manufacturers of the starting materials or commercial products. Reagents without specific origins indicated were commercially available conventional reagents.

The structures of the compounds were determined by nuclear magnetic resonance (NMR) or mass spectrometry (MS). NMR spectra were measured using a Bruker AVANCE-400 nuclear magnetic resonance instrument, with deuterated dimethyl sulfoxide (*DMSO-d6*), deuterated chloroform ($CDCl_3$) and deuterated methanol ($CD_3OD$) as determination solvents and tetramethylsilane (TMS) as internal standard. Chemical shifts were given in unit of $10^{-6}$ (ppm).

MS analysis was performed using a FINNIGAN LCQAd (ESI) mass spectrometer (manufacturer: Thermo, model: Finnigan LCQ advantage MAX).

High performance liquid chromatography (HPLC) analysis was performed using an Agilent 1200DAD high pressure liquid chromatograph (Sunfire C18 150 $\times$ 4.6 mm chromatography column) and a Waters 2695-2996 high pressure liquid chromatograph (Gimini C18 150 $\times$ 4.6 mm chromatography column).

Yantai Huanghai HSGF254 or Qingdao GF254 silica gel plates, 0.15-0.2 mm layer thickness, were adopted for thin-layer chromatography (TLC) analysis and 0.4-0.5 mm layer thickness for TLC separation and purification.

Yantai Huanghai silica gel of 200-300 mesh was generally used as a carrier in column chromatography.

Known starting materials described herein may be synthesized using or according to methods known in the art, or may be purchased from ABCR GmbH & Co.KG, Acros Organnics, Aldrich Chemical Company, Accela ChemBio Inc., Chembee Chemicals, and other companies.

In the examples, the reactions were performed under argon atmosphere or nitrogen atmosphere unless otherwise stated.

The argon atmosphere or nitrogen atmosphere means that the reaction flask was connected to a balloon

containing about 1 L of argon or nitrogen.

The hydrogen atmosphere means that the reaction flask was connected to a balloon containing about 1 L of hydrogen.

Parr 3916EKX hydrogenator, Qinglan QL-500 hydrogenator or HC2-SS hydrogenator was used in the pressurized hydrogenation reactions.

The hydrogenation reactions usually involved 3 cycles of vacuumization and hydrogen purge.

A CEM Discover-S 908860 microwave reactor was used in the microwave reactions.

In the examples, the solution in the reaction was an aqueous solution unless otherwise stated.

In the examples, the reaction temperature was room temperature unless otherwise stated.

The room temperature was the optimum reaction temperature, ranging from 20 °C to 30 °C.

Preparation of PBS buffer having a pH of 6.5 in Examples: 8.5 g of $KH_2PO_4$, 8.56 g of $K_2HPO_4.3H_2O$, 5.85 g of NaCl and 1.5 g of EDTA were added to a flask, the solution was brought to a volume of 2 L, the components were all dissolved by ultrasonication, and the solution was well shaken.

The eluent system for column chromatography and the developing solvent system for thin layer chromatography used for compound purification included: A: dichloromethane and isopropanol system, B: dichloromethane and methanol system, and C: petroleum ether and ethyl acetate system. The volume ratio of solvents was adjusted according to the polarity of the compound, or by adding a small amount of triethylamine and acidic or basic reagent.

Some of the compounds of the present disclosure were characterized by Q-TOF LC/MS. Q-TOF LC/MS analysis was performed using an Agilent 6530 accurate-mass quadrupole time-of-flight mass spectrometer and an Agilent 1290-Infinity ultra-high performance liquid chromatograph (Agilent Poroshell 300SB-C8 5 μm, 2.1 × 75 mm chromatography column).

A dendrimer as shown below was synthesized with reference to the synthesis method of the patent CN 110312531A. Its first amino terminal group is used for linking to a pharmaceutically active agent, and its second terminal group is used for linking to a pharmacokinetic modifier PEG.

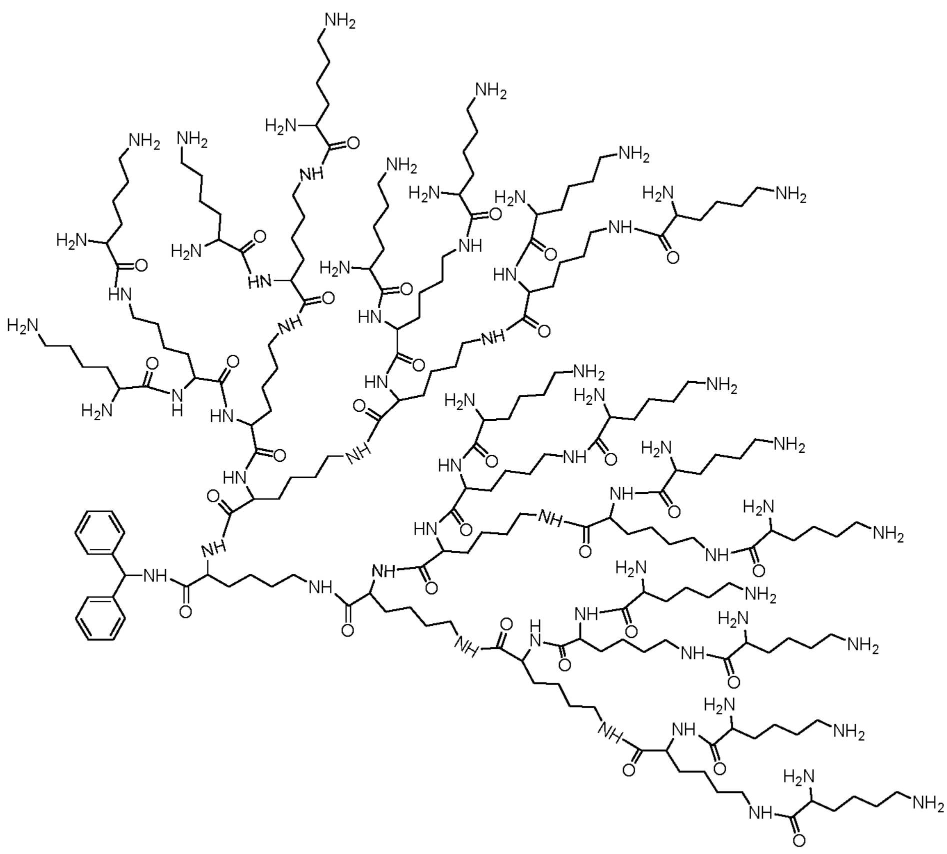

The dendrimers represented in the examples below include reference to the core and the structural units in the outermost generation of the dendrimer. The 1st generation to subsurface generations are not depicted. The dendrimer $BHAL_ys[Lys]_{32}$ represents a 5-generation dendrimer having the formula BHALys $[Lys]_2[Lys]_4[Lys]_8[Lys]_{16}[Lys]_{32}$; the 64 surface amino groups are available for binding to terminal groups.

Preparation of the dendrimer scaffolds BHALys $[Lys]_{32}[\alpha\text{-}NH_2.TFA]_{32}[\varepsilon\text{-}PEG_{570}]_{32}$, BHALys$[Lys]_{32}[\alpha$-

$NH_2.TFA]_{32}[\varepsilon\text{-}PEG_{1100}]_{32}$, $BHALys[Lys]_{32}[\alpha\text{-}NH_2.TFA]_{32}[\varepsilon\text{-t-}PEG_{2300}]_{32}$, $BHALys[Lys]_{32}[\alpha\text{-4-HSBA}]_{32}[\varepsilon\text{-}PEG_{1100}]_{32}$, $BHALys[Lys]_{32}[\alpha\text{-GILGVP-}NH_2.TFA]_{32}[\varepsilon\text{-}PEG_{1100}]_{32}$ and $BHALys[Lys]_{32}[\alpha\text{-GILGVP-}NH_2.TFA]_{32}[\varepsilon\text{-t-}PEG_{1100}]_{32}$ can be found in Kaminskas et al., J Control. Release (2011) (doi: 10.1016/j.jconrel.2011.02.005). Preparation of the dendrimer scaffold 4-azidobenzamide-$PEG_{12}$-NEOEOEN$[Su(NPN)_2][Lys]_{16}[NH_2.TFA]_{32}$ can be found in WO08/017122.

1-A00, 1-B00 and 1-C00 were synthesized according to the patent WO2012167309, and the pharmaceutically active agent to which they were linked was docetaxel. 2-A00 was synthesized according to the patent WO2018154004A, and the pharmaceutically active agent to which it was linked was compound 2 (synthesized according to the patent WO2012017251). The dendrimer scaffold $BHALys[Lys]_{32}[\alpha\text{-}NH_2.TFA]_{32}[\varepsilon\text{-}PEG_{1100}]_{32}$ was denoted in the examples by dendrimer 1, and $BHALys[Lys]_{32}[\alpha\text{-}NH_2.TFA]_{32}[\varepsilon\text{-}PEG_{2100}]_{32}$ was denoted in the examples by dendrimer 1-PEG2K. They were synthesized according to the method of WO2018154004A:

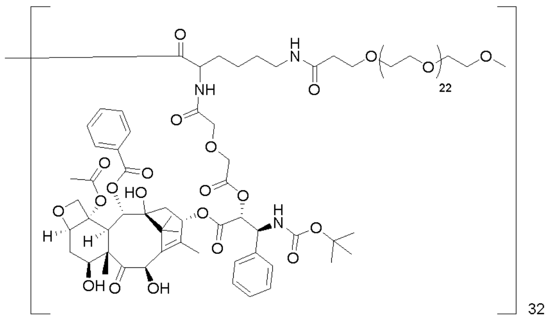

1-A00 ,

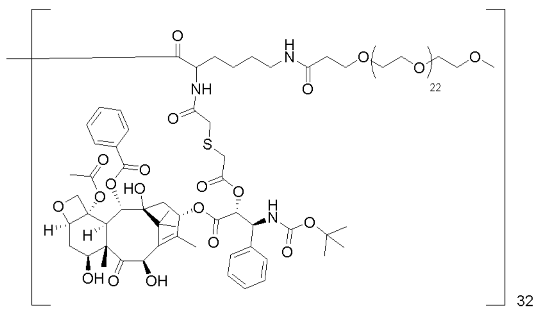

1-B00 ,

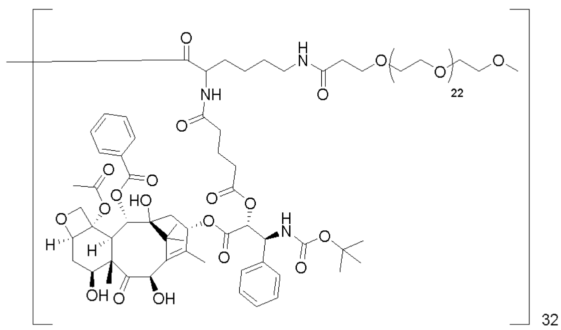

1-C00 ,

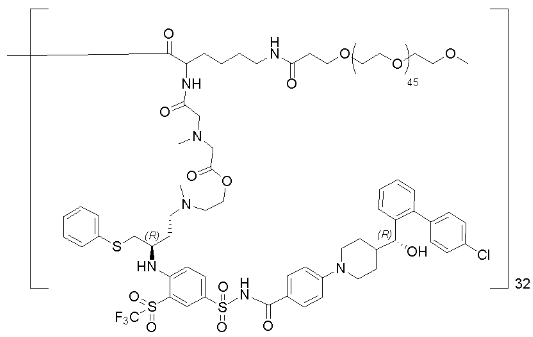

2-A00 .

General procedure

General procedure A

Installation of linkers to drugs

To a magnetically stirred solution of carboxylic acid linker (0.2-0.5 mmol) in solvent DMF or acetonitrile (1-5 mL) at 0 °C was added coupling agent EDC or DCC (1.2 equivalents). The mixture was left to be stirred for 5 min, and then a solution of solvent (1 mL) containing a mixture of drug (0.4-1 equivalents) and DMAP (0.4-1 equivalents) was added dropwise. The mixture was kept at 0 °C for 1 h and then warmed to ambient temperature. The volatiles were then removed *in vacuo* and the residue was purified by preparative HPLC (BEH300 Waters XBridge C18, 5 μM, 30 × 150 mm, 40-80% ACN/water (5-40 min), no buffer) to give the desired product.

General procedure B

Installation of linkers to drugs

To a magnetically stirred solution of drug (0.3-1.0 mmol) and anhydride (2 equivalents) in DMF (3-5 mL) was added DIPEA (3 equivalents). The mixture was stirred at ambient temperature overnight. The volatiles were then removed *in vacuo* and the residue was purified by preparative HPLC (BEH300 Waters XBridge C18, 5 μM, 30 × 150 mm, 40-70%

ACN/water (5-40 min), no buffer, RT = 34 min). The appropriate fractions were concentrated *in vacuo* to give the desired product.

General procedure C

Dendrimer loaded with drug-linker

To a magnetically stirred mixture of BHALys[Lys]$_{32}$[$\alpha$-$NH_2$.TFA]$_{32}$[$\varepsilon$-PEG$_{1100}$]$_{32}$ (0.5-1.0 $\mu$mol) and DIPEA (1.2 equivalents/amine) in DMF at room temperature was added linker-drug (1.2 equivalents/amine) and PyBOP (1.2 equivalents/amine). After 1.5 h of stirring at room temperature, the volatiles were removed and the residue was purified by SEC (sephadex, LH20, MeOH). The appropriate fractions (as judged by HPLC) were combined and concentrated to give the desired product.

General procedure D

Click reaction

To a magnetically stirred solution of dendrimer (0.5-1.0 mmol) in 1:1 $H_2O$/t-BuOH (about 0.5 mL) was added alkyne reagent (2 equivalents), sodium ascorbate solution (2 equivalents) and $CuSO_4$ solution (20 mol%). The solution was heated to 80 °C and monitored by HPLC. Additional amounts of sodium ascorbate and $CuSO_4$ were added as required to drive the reaction to completion. After the reaction was judged complete, the reaction mixture was concentrated *in vacuo* and then purified.

The macromolecule of the present disclosure can be synthesized by following a route selected from the group consisting of the following, with PG as a carboxylic acid protecting group:

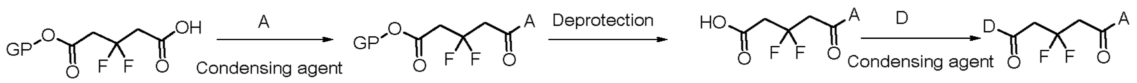


;

or

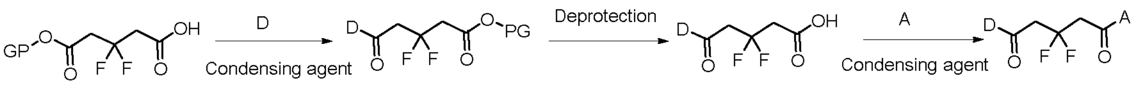


; or

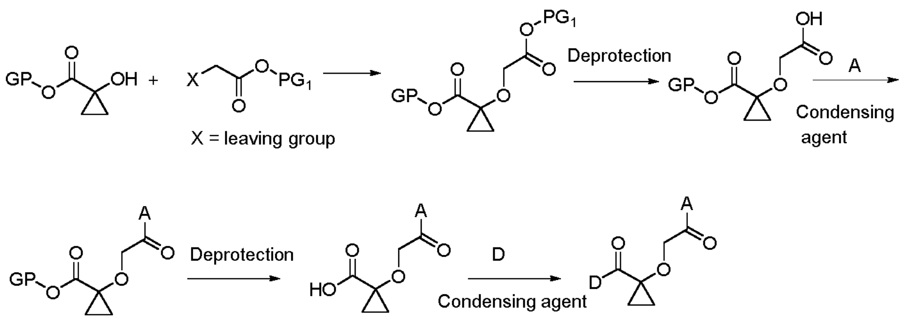


;

or

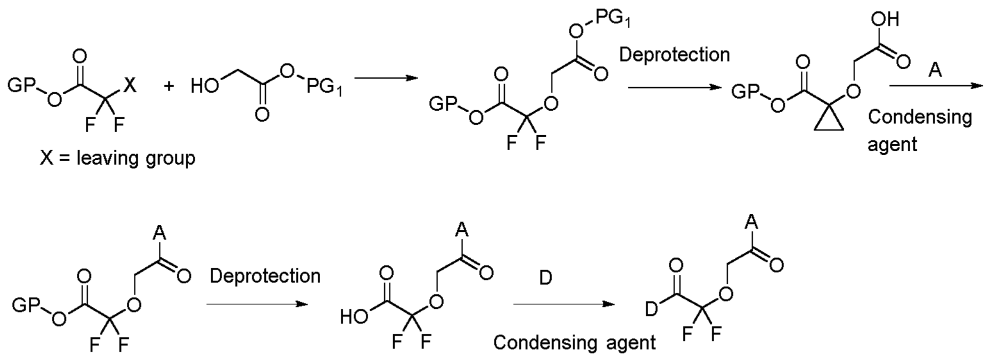
X = leaving group
Deprotection
A
Condensing agent
Deprotection
D
Condensing agent

;

or

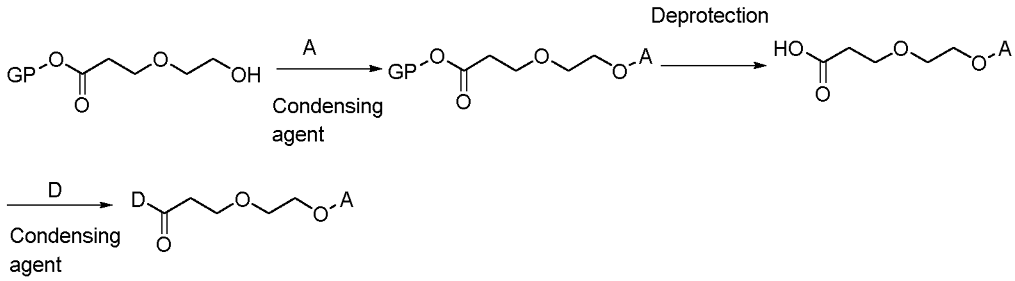
A
Condensing agent
Deprotection
D
Condensing agent

;

or

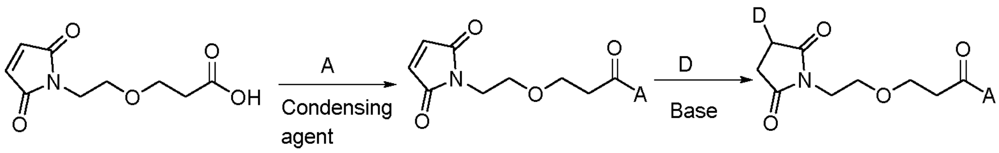
A
Condensing agent
D
Base

;

or

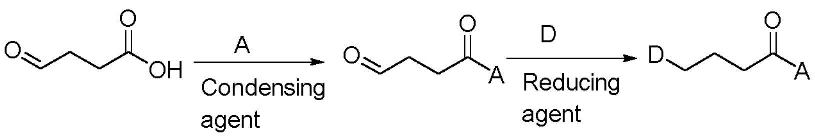
A
Condensing agent
D
Reducing agent

;

or

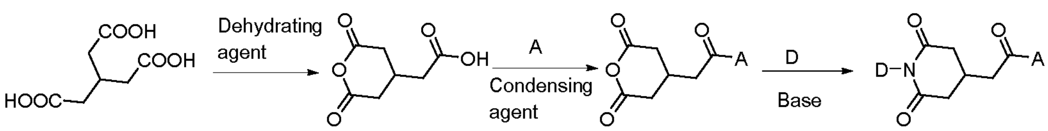
Dehydrating agent
A
Condensing agent
D
Base

;

or

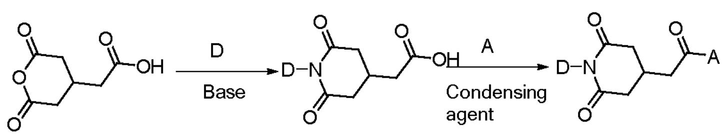
D
Base
A
Condensing agent

.

Example 1: Preparation of Compound 3

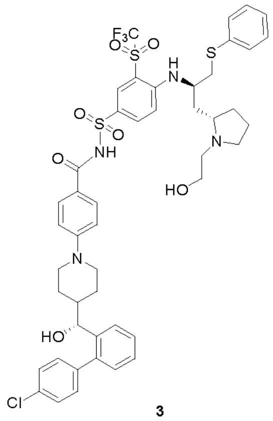

3

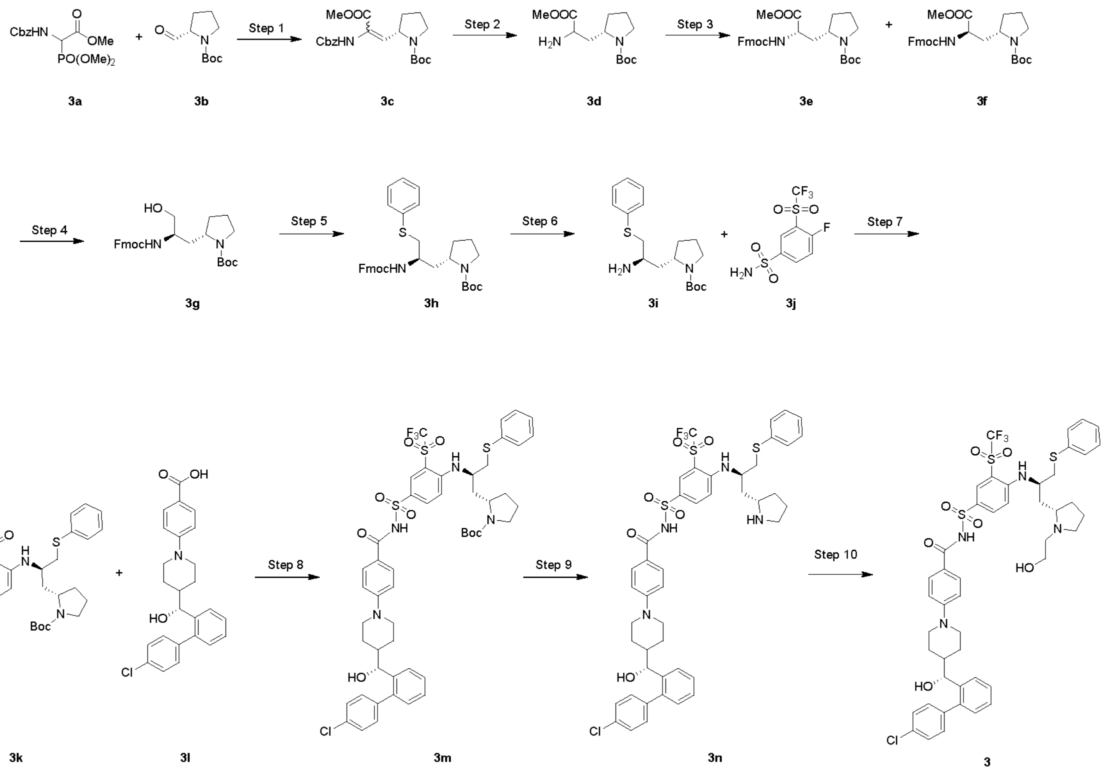


3a 3b 3c 3d 3e 3f

3g 3h 3i 3j

3k 3l 3m 3n 3

Step 1

tert-Butyl (*S*)-2-(2-(((benzyloxy)carbonyl)amino)-3-methoxy-3-oxoprop-1-en-1-yl)pyrrolidine-1-c arboxylate **3c**

**3a** (2.49 g, 7.53 mmol, Accela ChemBio) was dissolved in tetrahydrofuran (20 mL). The solution was cooled to 0 °C under ice bath conditions. *N*,*N*-lithium diisopropylamide (661 mg, 6.17 mmol) was added dropwise. After the mixture was stirred at 0 °C for 1 h, the reaction system was cooled to -78 °C, and a solution of tert-butyl (*S*)-2-formylpyrrolidine-1-carboxylate **3b** (1.00 g, 5.02 mmol, PharmaBlock) in tetrahydrofuran (20 mL) was added dropwise thereto. After the dropwise addition, the dry ice-acetone bath was removed, and the mixture was warmed to room temperature and reacted for 12 h. The mixture was quenched with saturated ammonium chloride solution (20 mL) and extracted with ethyl acetate (20 mL × 3). The organic phase was dried over anhydrous sodium sulfate and then filtered. The filtrate was concentrated under reduced pressure. The resulting residue was purified by silica gel column chromatography with eluent system B to give the title product **3c** (1.90 g, yield: 94.1%).

MS m/z (ESI): 305.0 [M+H-100].

Step 2

tert-Butyl (2*S*)-2-(2-amino-3-methoxy-3 -oxopropyl)pyrrolidine-1-carboxylate **3d**

**3c** (1.90 g, 4.7 mmol) was dissolved in isopropanol (50 mL). Palladium on carbon (380 mg, 10% loading, dry basis) was added. The system was purged 3 times with hydrogen. The mixture was stirred at room temperature for 8 h. The reaction mixture was filtered through celite. The filter cake was rinsed with ethyl acetate (20 mL) and methanol (20 mL). The filtrate was concentrated to give the crude title product **3d** (1.28 g). The product was directly used in the next step without being purified.

Step 3

tert-Butyl (*S*)-2-((*S*)-2-((((9*H*-fluoren-9-yl)methoxy)carbonyl)amino)-3-methoxy-3-oxopropyl)pyr rolidine-1-carboxylate **3e**

tert-Butyl (*S*)-2-((*R*)-2-((((9*H*-fluoren-9-yl)methoxy)carbonyl)amino)-3-methoxy-3-oxopropyl)pyr rolidine-1-carboxylate **3f**

The crude **3d** (1.28 g, 4.7 mmol) was dissolved in 1,4-dioxane (40 mL). Water (10 mL), sodium bicarbonate (1.17 g, 14.1 mmol) and fluorenylmethoxycarbonyl chloride (1.21 g, 4.7 mmol) were added. The mixture was stirred at room temperature for 3 h. The mixture was quenched with water (30 mL) and extracted with ethyl acetate (20 mL × 3). The organic phase was dried over anhydrous sodium sulfate, filtered and concentrated. The resulting residue was purified by silica gel column chromatography with eluent system A to give the title product **3e** (1.03 g, 44.2%, peak 1) and **3f** (513 mg, 22.1%, peak 2).

MS m/z (ESI): 495.2 [M+1].

Peak 1: UPLC analysis: retention time: 2.77 min (column: ACQUITY UPLC BEHC18 50 × 2.1 mm, 1.7 μm; mobile phase: acetonitrile/water/formic acid = 10/90/0.1 (v/v/v) to 95/5/0.1 (v/v/v) gradient elution).

Peak 2: UPLC analysis: retention time: 2.69 min (column: ACQUITY UPLC BEHC18 50 × 2.1 mm, 1.7 μm; mobile phase: acetonitrile/water/formic acid = 50/50/0.1 (v/v/v) to 95/5/0.1 (v/v/v) gradient elution).

Step 4

tert-Butyl (*S*)-2-((R)-2-((((9*H*-fluoren-9-yl)methoxy)carbonyl)amino)-3-hydroxypropyl)pyrrolidin e-1-carboxylate **3g**

**3f** (169 mg, 0.34 mmol) was dissolved in tetrahydrofuran (8 mL). Ethanol (8 mL), sodium borohydride (77 mg, 2.05 mmol) and lithium chloride (94 mg, 2.22 mmol) were added. The mixture was stirred at 28 °C for 2.5 h, and saturated ammonium chloride (5 mL) was added dropwise to quench the reaction. The solution was concentrated under reduced pressure. The residue was dissolved in ethyl acetate (10 mL) and washed sequentially with water (10 mL) and saturated sodium chloride (10 mL). The organic phase was dried over anhydrous sodium sulfate, filtered and concentrated. The resulting residue was purified by silica gel column chromatography with eluent system A to give the title product **3g** (125 mg, 78.4%).

MS m/z (ESI): 467.2 [M+1].

Step 5

tert-Butyl (*S*)-2-((*R*)-2-((((9*H*-fluoren-9-yl)methoxy)carbonyl)amino)-3-(phenylthio)propyl)pyrroli dine-1-carboxylate **3h**

**3g** (120 mg, 0.26 mmol) was dissolved in toluene (5 mL). Diphenyl disulfide (168 mg, 0.77 mmol) and tributylphosphine (156 mg, 0.77 mmol) were added. The reaction system was heated to 80 °C and stirred for 12 h under nitrogen atmosphere. The solution was concentrated under reduced pressure. The resulting residue was purified by silica gel column chromatography with eluent system A to give the title product **3h** (135 mg, 93.9%).

MS m/z (ESI): 559.2 [M+1].

Step 6

tert-Butyl (*S*)-2-((*R*)-2-amino-3-(phenylthio)propyl)pyrrolidine-1-carboxylate **3i**

**3h** (135 mg, 0.24 mmol) was dissolved in dichloromethane (4 mL). Diethylamine (4 mL) was added. The mixture was stirred at room temperature for 4 h. The reaction mixture was concentrated under reduced pressure to remove the organic solvent to give the title product **3i** (81.6 mg). The product was directly used in the next step without being purified.

Step 7

tert-Butyl (*S*)-2-((*R*)-4-(phenylthio)-3-((4-sulfamoyl-2-((trifluoromethyl)sulfonyl)phenyl)amino)b utyl)pyrrolidine-1-carboxylate **3k**

The crude **3i** (81.6 mg, 0.24 mmol) was dissolved in *N*,*N*-dimethylformamide (5 mL). 4-Fluoro-3-(trifluoromethylsulfonyl)benzenesulfonamide **3j** (82 mg, 0.27 mmol) and *N*,*N*-diisopropylethylamine (314 mg, 2.4 mmol) were added. The reaction system was heated to 50 °C and stirred for 12 h under nitrogen atmosphere. The mixture was quenched with water (10 mL) and extracted with ethyl acetate (5 mL × 3). The organic phase was dried over anhydrous sodium sulfate and filtered. The filtrate was concentrated under reduced pressure. The resulting residue was purified by silica gel column chromatography with eluent system A to give the title product **3k** (105 mg, 69.3%).

MS m/z (ESI): 622.1 [M-1].

Step 8

tert-Butyl (*S*)-2-((*R*)-2-((4-(*N*-(4-(4-((*R*)-(4'-chloro-[1,1'-biphenyl]-2-yl)(hydroxy)methyl)piperidin -1-yl)benzoyl)sulfamoyl)-2-((trifluoromethyl)sulfonyl)phenyl)amino)-3-(phenylthio)pro pyl)pyrrolidine-1-carboxylate **3m**

**3k** (30 mg, 0.048 mmol) was dissolved in dichloromethane (3 mL). **31** (24 mg, 0.058 mmol, synthesized using the method provided for intermediate 40 on page 79 of the specification in the patent "CN103153954 B") was added, and then 1-(3-dimethylaminopropyl)-3-ethylcarbodiimide hydrochloride (19 mg, 0.096 mmol) and 4-dimethylaminopyridine (12 mg, 0.096 mmol) were sequentially added. After the addition, the mixture was stirred at room temperature for 4 h under nitrogen atmosphere and supplemented with **31** (12 mg, 0.038 mmol), 1-(3-dimethylaminopropyl)-3-ethylcarbodiimide hydrochloride (19 mg, 0.096 mmol) and 4-dimethylaminopyridine (12 mg, 0.096 mmol). After the supplementation, the mixture was stirred at room temperature overnight. The reaction mixture was washed sequentially with water (5 mL) and saturated brine (5 mL), dried over anhydrous sodium sulfate, filtered and then concentrated under reduced pressure. The resulting residue was purified by thin-layer chromatography with developing solvent system A to give the title product **3m** (30 mg, yield: 60.7%).

MS m/z (ESI): 1027.2 [M+1].

Step 9

4-(4-((*R*)-(4'-chloro-[1,1'-biphenyl]-2-yl)(hydroxy)methyl)piperidin-1-yl)-*N*-((4-(((*R*)-1-(phenylthio)-3-((*S*)-pyrrolidin-2-yl)propan-2-yl)amino)-3-((trifluoromethyl)sulfonyl)ph enyl)sulfonyl)benzamide **3n**

**3m** (30 mg, 0.029 mmol) was dissolved in dichloromethane (2 mL). A 4 *M* solution of hydrogen chloride in 1,4-dioxane (2 mL) was added. The mixture was stirred at room temperature for 20 min. The reaction mixture was concentrated under reduced pressure to remove the organic solvent. The residue was dissolved in dichloromethane and then concentrated under reduced pressure to dryness; the process was repeated three times. The resulting residue was purified by high performance liquid chromatography (separation conditions: column: Sharpsil-T Prep C18; mobile phase: ammonium bicarbonate, water, acetonitrile). The corresponding fraction was collected and concentrated under reduced pressure to give the title product **3n** (25 mg, yield: 92.3%).

MS m/z (ESI): 927.1 [M+1].

$^1$H NMR (500 MHz, $CD_3OD$) δ 8.24 (d, 1H), 8.05-8.01 (m, 1H), 7.82 (d, 2H), 7.60 (d, 1H), 7.45-7.39 (m, 3H), 7.37 (d, 2H), 7.34-7.28 (m, 3H), 7.23 (t, 2H), 7.20-7.14 (m, 2H), 6.80 (d, 2H), 6.72 (d, 1H), 4.42 (d, 1H), 3.97 (br, 1H), 3.84-3.74 (m, 1H), 3.27-3.21 (m, 2H), 3.19-3.07 (m, 3H), 3.06-2.97 (m, 1H), 2.69-2.61 (m, 1H), 2.55-2.48 (m, 1H), 2.16-2.00 (m, 5H), 1.98-1.89 (m, 2H), 1.88-1.80 (m, 1H), 1.78-1.69 (m, 1H), 1.63-1.57 (m, 2H), 1.54-1.49 (m, 1H), 1.26-1.20 (m, 1H), 1.15-1.04 (m, 1H), 1.01-0.94 (m, 1H).

Step 10

4-(4-((*R*)-(4'-chloro-[1,1'-biphenyl]-2-yl)(hydroxy)methyl)piperidin-1-yl)-*N*-((4-(((A)-1 - ((*S*)-1-(2-hydroxyethyl)pyrrolidin-2-yl)-3-(phenylthio)propan-2-yl)amino)-3-((trifluoro methyl)sulfonyl)phenyl)sulfonyl)benzamide **3**

**3n** (15 mg, 0.016 mmol) was dissolved in acetonitrile (2 mL). Triethylamine (16 mg, 0.16 mmol) and bromoethanol (20 mg, 0.16 mmol) were sequentially added. The reaction system was heated to 70 °C and reacted for 12 h under nitrogen atmosphere. The reaction mixture was purified by high performance liquid chromatography (separation conditions: column: Sharpsil-T Prep C18; mobile phase: ammonium bicarbonate, water, acetonitrile). The corresponding fraction was collected and concentrated under reduced pressure to give the title product **3** (10 mg, yield: 63.4%).
MS m/z (ESI): 971.2 [M+1].
$^1$H NMR (500 MHz, $CD_3OD$) δ 8.25 (d, 1H), 8.06 (dd, 1H), 7.80 (d, 2H), 7.61 (dd, 1H), 7.49-7.35 (m, 5H), 7.35-7.28 (m, 3H), 7.24 (dd, 2H), 7.20-7.14 (m, 2H), 6.79 (t, 3H), 4.42 (d, 1H), 4.02 (t, 1H), 3.84-3.70 (m, 3H), 3.61 (d, 2H), 3.28-3.14 (m, 2H), 3.06 (br, 1H), 2.96 (br, 1H), 2.71-2.59 (m, 1H), 2.56-2.45 (m, 1H), 2.45-2.35 (m, 1H), 2.32-2.22 (m, 1H), 2.10-1.97 (m, 4H), 1.96-1.86 (m, 1H), 1.85-1.69 (m, 2H), 1.60 (t, 2H), 1.26-1.18 (m, 1H), 1.06-1.08 (m, 1H), 1.01-0.92 (m, 1H), 0.92-1.86 (m, 1H).

Example 2: Preparation of Compound **3-TMS**

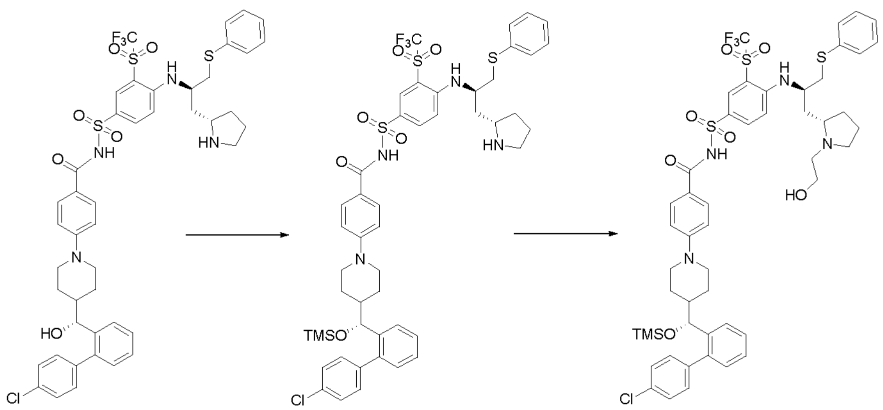

3n 3o 3-TMS

Step 1

**3n** (6 g, 6.47 mmol) was dissolved in DCM (120 mL). Triethylamine (3.22 g, 32.35 mmol) was added. The mixture was cooled to 0 °C in an ice-water bath. Trimethylsilyl trifluoromethanesulfonate (7.2 g, 32.35 mmol) was slowly added dropwise to the reaction mixture. The mixture was stirred in the ice-water bath for 2 h. The mixture was quenched by adding dropwise methanol (12 mL) under ice-water bath conditions, stirred in the ice-water bath for 10 min and concentrated under reduced pressure to remove the solvent to give crude **3o** (16.5 g), which was used in the next step without being purified.
MS-ESI: m/z 999.1 $[M+1]^+$.

Step 2

**3o** (16.5 g, calculated as 6.47 mmol) was dissolved in acetonitrile (90 mL). Triethylamine (7.86 g, 77.64 mmol) and bromoethanol (9.7 g, 77.64 mmol) were added. The system was purged three times with nitrogen. The mixture was heated to 70 °C and reacted overnight (about 16 h). The solvent was removed under reduced pressure, and then EA (120 mL) and water (120 mL) were added. The mixture was stirred. The aqueous phase was separated and extracted with EA (60 mL × 2). The organic phases were combined, washed with water (120 mL), washed with saturated brine (120 mL), dried and concentrated under reduced pressure. The resulting residue was purified by silica gel column chromatography (DCM:$CH_3OH$ = 20:1 to 10:1) to give compound **3-TMS** (5.03 g, yield over two steps: 74.5%, purity: 95.9%).
MS-ESI: m/z 1043.05 $[M+1]^+$.

$^1H$ NMR (400 MHz, $CDCl_3$) δ 8.29 (s, 1H), 8.05 (s, 1H), 7.74 (d, 2H), 7.55 (t, 1H), 7.43-7.33 (m, 3H), 7.29 (dd, 3H), 7.24-7.06 (m, 6H), 6.97 (d, 1H), 6.72 (d, 2H), 6.63 (d, 1H), 4.54 (d, 1H), 3.98 (s, 1H), 3.82-3.46 (m, 5H), 3.02 (d, 5H), 2.82-2.44 (m, 4H), 2.26 (dd, 1H), 2.07 (d, 1H), 1.90 (dd, 3H), 1.74 (d, 2H), 1.58-1.41 (m, 1H), 1.40-1.15 (m, 4H), -0.06 (s, 9H).

Example 3: Preparation of Compound **1-Z00**

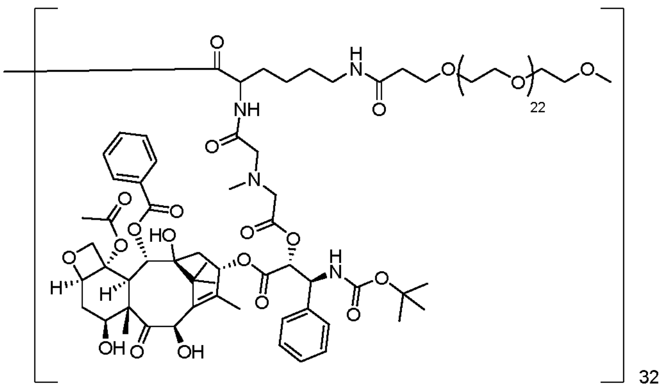

**1-Z00**

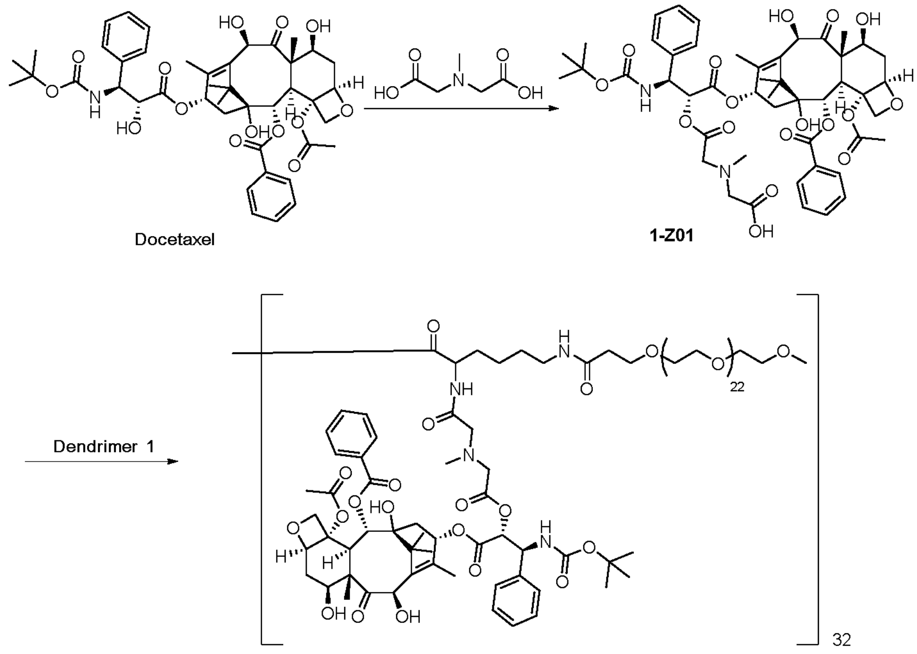


Docetaxel

**1-Z01**

**1-Z00**

Step 1

To a reaction flask were sequentially added DMF (4 mL), methyliminodiacetic acid (218 mg, 1.48 mmol) and DCC (245 mg, 1.18 mmol). The mixture was cooled in an ice-water bath. Docetaxel (400 mg, 0.495 mmol) and DMAP (60 mg, 0.495 mmol) were added. The mixture was stirred at room temperature for 1.5 h. Additional DCC (102 mg, 0.495 mmol) was added. The mixture was stirred at room temperature for another hour. The reaction was stopped after being judged complete. The mixture was filtered. The filter cake was rinsed with ethyl acetate (15 mL). The filtrate was concentrated under reduced pressure to remove ethyl acetate. The remaining mother liquor was purified by preparative HPLC to give compound **1-Z01** (86 mg, yield: 19%).

MS-ESI: m/z 937.4 $[M+H]^+$.

Step 2

**1-Z01** (1.59 g, 1.70 mmol) and PyBOP (1.70 g, 3.27 mmol) were dissolved in anhydrous DMF (28.0 mL) under nitrogen atmosphere. The solution was well stirred. Dendrimer 1 (2.00 g, $4.25 \times 10^{-2}$ mmol) and a solution of DIPEA (0.42 g, 3.27 mmol) in anhydrous DMF (28.0 mL) were then added dropwise to the above reaction solution. The mixture was reacted for 2 h. The reaction mixture was diluted with acetonitrile (56.0 mL) and then purified by ultrafiltration with acetonitrile in an ultrafiltration (10 KD, Hydrosart®) device to give a crude product (2.70 g). The product was dissolved in pure water (150 mL) by vortexing. The solution was filtered through a filter membrane (0.22 μm) and lyophilized to give compound **1-Z00** (2.40 g, yield: 85%).

$^1$H NMR indicated 25 DTX/dendrimer. The actual molecular weight was approximately 66.4 kDa (30.39% wt.% DTX).

$^1$H NMR (400 MHz, $CD_3OD$) δ (ppm) 8.05-8.24 (m, 64 H), 7.11-7.78 (m, 272 H), 5.98-6.25 (m, 25 H), 5.55-5.71 (m, 29 H), 5.19-5.49 (m, 84 H), 4.93-5.07 (s, 47 H), 4.10-4.64 (m, 162 H), 3.40-4.08 (m, 3200 H), 3.33-3.37 (S, 96 H), 3.02-3.23 (m, 126 H), 2.14-2.63 (m, 271 H), 0.99-2.10 (m, 1109 H).

Example 4: Preparation of Compound **1-Z00-J**

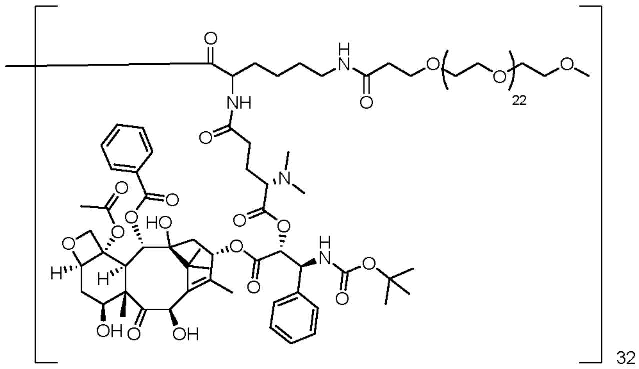

**1-Z00-J**

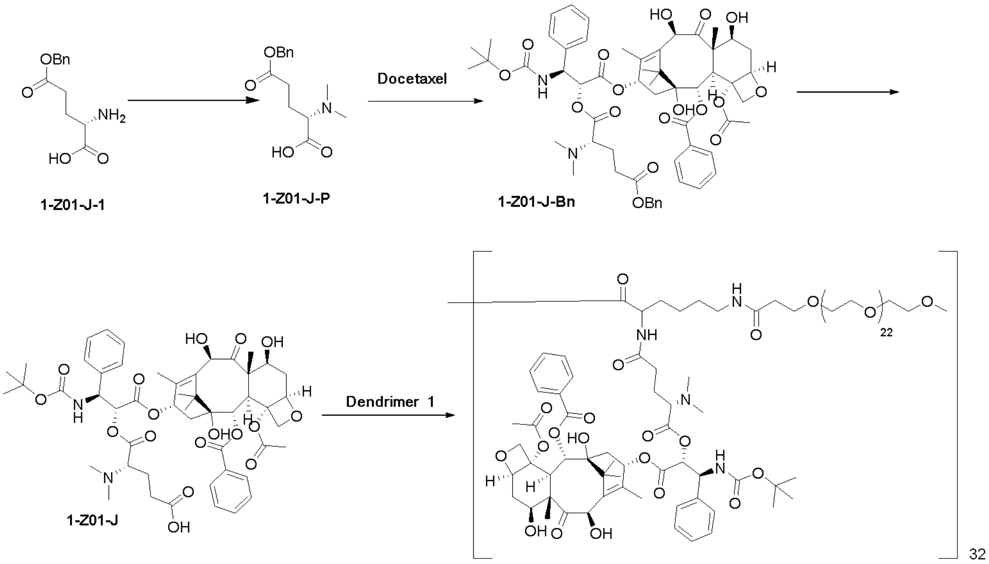


1-Z01-J-1

1-Z01-J-P

1-Z01-J-Bn

1-Z01-J

1-Z00-J

Step 1

Compound **1-Z01-J-1** (7 g, 29.5 mmol) was dissolved in methanol (150 mL). A formaldehyde solution (15 mL) and $NaBH(AcO)_3$ (63 g, 295 mmol) were added. The mixture was reacted at room temperature for 1 h. After concentration, the residue was purified by column chromatography to give compound **1-Z01-J-P** (5.9 g, yield: 75%).

$^1$H-NMR (400 MHz, DMSO-$d_6$) δ 7.36-7.32 (m, 5H), 5.09 (s, 2H), 3.12 (t, *J* = 7.2 Hz, 1H), 2.5-2.44 (m, 2H), 2.38 (s, 6H), 1.87-1.84 (m, 2H). MS-ESI: m/z 266.2 $[M+H]^+$.

Step 2

Compound **1-Z01-J-P** (1.44 g, 5.45 mmol) and docetaxel (4 g, 4.96 mmol) were dissolved in dichloromethane (50 mL). EDCI (1.04 g, 5.45 mmol) and DMAP (665 mg, 5.45 mmol) were added. The mixture was stirred at room temperature overnight. The mixture was quenched with water and extracted with dichloromethane. After concentration, the residue was purified by column chromatography to give compound **1-Z01-J-Bn** (5 g, yield: 95%).

MS-ESI: m/z 1055.5 $[M+H]^+$.

Step 3

Compound **1-Z01-J-Bn** (5.75 g, 5.46 mmol) was dissolved in tetrahydrofuran (60 mL). Pd/C (600 mg, 10 wt.%) was added. The system was purged three times with hydrogen. The mixture was reacted at room temperature overnight. After filtration and concentration, the residue was purified by column chromatography to give compound **1-Z01-J** (3.2 g, yield: 57%).

MS-ESI: m/z 965.3 $[M+H]^+$.

Step 4

Compound **1-Z01-J** (819 mg, 849.5 μmol) and PyBOP (853 mg, 1640 μmol) were dissolved in anhydrous *N*,*N*-dimethylformamide (14 mL) under nitrogen atmosphere. A solution of the compound dendrimer **1** (1000 mg, 21.24 μmol) and DIPEA (212 mg, 1640 μmol) in anhydrous *N*,*N*-dimethylformamide (14 mL) was added dropwise. The mixture was reacted for 2 h. The reaction mixture was diluted with acetonitrile (28 mL) and then purified by ultrafiltration with acetonitrile in an ultrafiltration (10 KD, Hydrosart®) device to give a crude product (1.42 g). The product was dissolved in pure water (100 mL). The solution was filtered through a filter membrane (0.22 μm) and lyophilized to give compound **1-Z00-J** (1.42 g, yield: 95%).

$^1$H NMR indicated 25 DTX/dendrimer. The actual molecular weight was approximately 67.1 kDa (30.0% wt.% DTX).

$^1$H NMR (400 MHz, $CD_3OD$) δ 7.15-8.31 (m, 405H), 6.00-6.31 (m, 25H), 5.57-5.73 (m, 25H), 5.20-5.54 (m, 83H), 4.96-5.08 (m, 35H), 4.10-4.68 (m, 141H), 3.40-4.08 (m, 3200H), 3.33-3.37 (s, 103H), 3.07-3.24 (m, 92H), 2.14-2.69 (m, 260H), 1.00-2.10 (m, 1183H).

Example 5: Preparation of Compound **1-Z00-K**

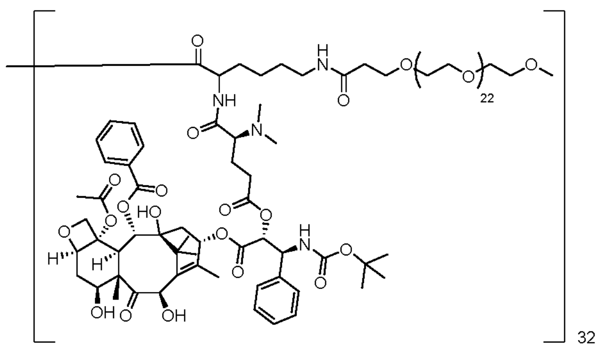

**1-Z00-K**

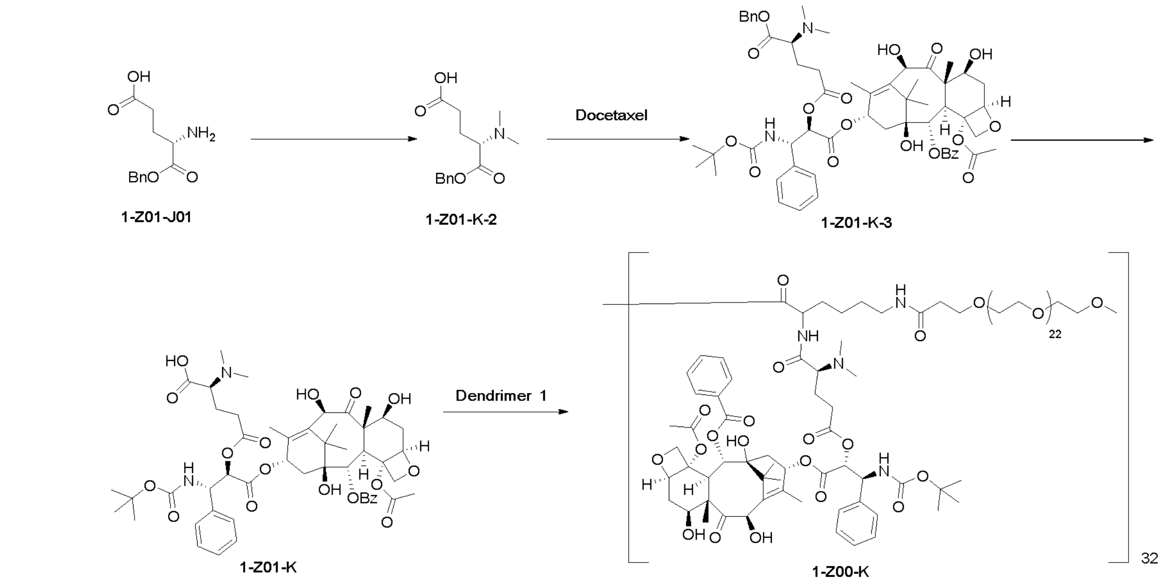


1-Z01-J01 1-Z01-K-2 1-Z01-K-3

1-Z01-K 1-Z00-K

Step 1

Compound **1-Z01-J01** (5.0 g, 21.09 mmol, GL Biochem (Shanghai) Ltd.) was dissolved in MeOH (100 mL) with stirring, and then an aqueous solution of formaldehyde (37 wt.%, 10 mL). was added. $NaBH(AcO)_3$ (22.3 g, 105.4 mmol) was added in batches under ice bath conditions. After the addition, the mixture was reacted at room temperature for 18 h. After the reaction was judged complete, the reaction mixture was concentrated under reduced pressure. The resulting residue was purified by column chromatography to give compound **1-Z01-K-2** (3.2 g, yield: 57%).

MS-ESI: m/z 266.1 $[M+H]^+$.

$^1$H-NMR: (CDCl3, 400 MHz) $\delta$ 7.36-7.27 (m, 5 H), 5.18 (dd, $J_1$=20.0 Hz, $J_2$= 12.0 Hz, 2 H), 3.42-3.36 (m, 2 H), 2.48-2.46 (m, 2 H), 2.45 (s, 6 H), 2.05-2.00 (m, 2 H).

Step 2

To a reaction flask were added under nitrogen atmosphere compound **1-Z01-K-2** (1.0 g, 1.23 mmol), docetaxel (1.0 g, 1.23 mmol), EDCI (261 mg, 1.36 mmol) and DMAP (166 mg, 1.36 mmol), followed by anhydrous DMF (10 mL). The mixture was stirred at room temperature overnight. Saturated sodium bicarbonate solution and ethyl acetate were added. The organic phase was washed with saturated brine, dried over anhydrous sodium sulfate, filtered and then concentrated under reduced pressure. The resulting residue was purified by column chromatography to give compound **1-Z01-K-3** (1.021 g, yield: 71%).

MS-ESI: m/z 1055.4 $[M+H]^+$.

Step 3

To a reaction flask were added the starting material **1-Z01-K-3** (1.0 g, 0.947 mmol) and wet palladium on carbon (10 wt.%, 200 mg), followed by tetrahydrofuran (20 mL). The system was purged 3 times with hydrogen. The mixture was reacted at room temperature for 18 h. The reaction mixture was filtered through celite. The filter cake was rinsed with ethyl acetate. The filtrate was concentrated and dried *in vacuo* to give compound **1-Z01-K** (736 mg, yield: 80%).

MS-ESI: m/z 965.3 $[M+H]^+$.

Step 4

Compound **1-Z01-K** (162 mg, 0.168 mmol) and PyBOP (168 mg, 0.323 mmol) were dissolved in anhydrous DMF (6.0 mL) under nitrogen atmosphere, and the solution was well stirred. Then a solution of dendrimer **1** (200 mg, $4.2 \times 10^{-3}$ mmol) and DIPEA (42 mg, 0.323 mmol) in anhydrous DMF (6.0 mL) was added dropwise to the above reaction solution. The mixture was reacted for 2 h. The reaction mixture was diluted with acetonitrile and then purified by ultrafiltration with acetonitrile in an ultrafiltration (10 KD, Hydrosart®) device to give a crude product (0.215 g). The product was dissolved in pure water (50 mL). The solution was filtered through a filter membrane (0.22 $\mu$m) and lyophilized to give compound **1-Z00-K** (0.21 g, yield: 76%).

$^1$H NMR indicated 24 DTX/dendrimer. The actual molecular weight was approximately 66.1 kDa (29.30% wt.% DTX).

$^1$H NMR (400 MHz, $CD_3OD$) δ (ppm) 8.00-8.23 (m, 55 H), 7.11-7.78 (m, 239 H), 5.95-6.30 (m, 24 H), 5.51-5.75 (m, 24 H), 5.15-5.45 (m, 72 H), 4.93-5.07 (s, 19 H), 4.10-4.64 (m, 157 H), 3.40-4.08 (m, 3200 H), 3.33-3.37 (s, 102 H), 2.93-3.23 (m, 167 H), 2.12-2.63 (m, 212 H), 0.99-2.10 (m, 1145 H).

Example 6: Preparation of Compound **1-Z00-L**

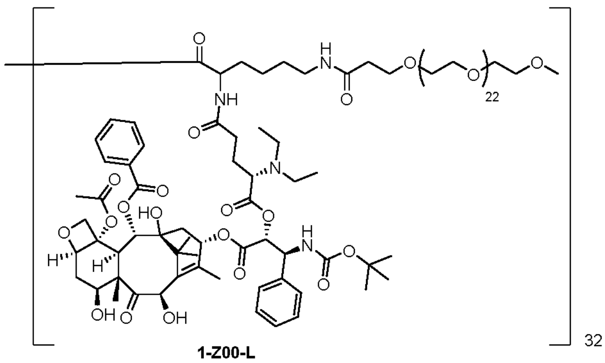

1-Z00-L

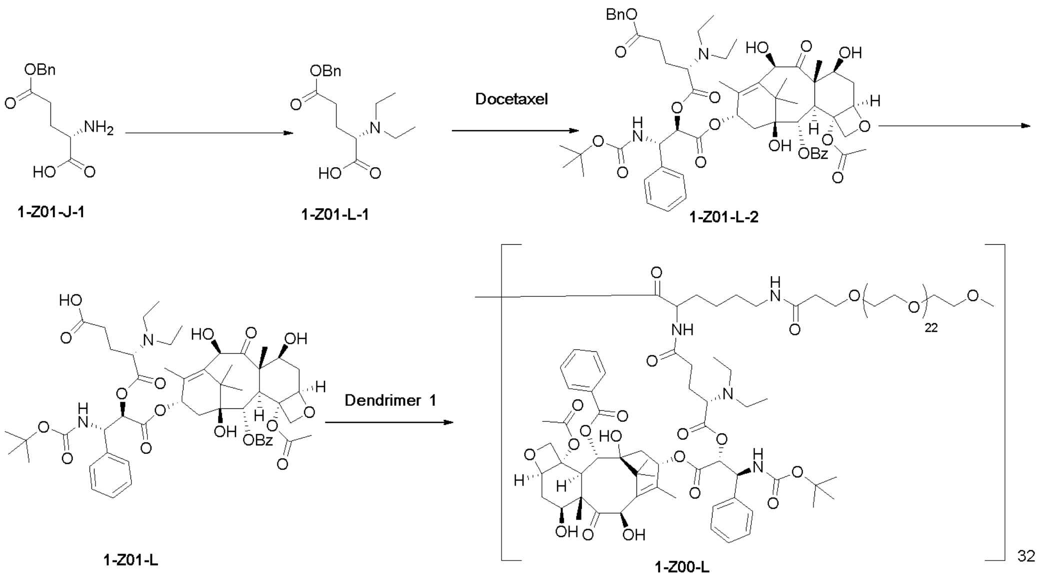


1-Z01-J-1 1-Z01-L-1 1-Z01-L-2 1-Z01-L 1-Z00-L

Step 1

Compound **1-Z01-J-1** (1.0 g, 4.2 mmol, Shanghai Bide Pharmatech) was dissolved in MeOH (20 mL) with stirring, and then an aqueous solution of acetaldehyde (40 wt.%, 2 mL) was added. $NaBH(AcO)_3$ (4.472 g, 21.0 mmol) was added under ice bath conditions. The mixture was reacted at room temperature for 20 h. After concentration under reduced pressure, the residue was purified by column chromatography to give compound **1-Z01-L-1** (733 mg, yield: 59.6%).

MS-ESI: m/z 294.1 $[M+H]^+$.

$^1$H-NMR: (CDCl3, 400 MHz) δ 7.35-7.28 (m, 5H), 5.10 (dd, $J_1$=18 Hz, $J_2$= 12.4 Hz, 2H), 4.15 (brs 1 H), 3.56 (t, $J$=6.8 Hz 1H), 3.27-3.22 (m, 2H), 3.07-3.02 (m, 2H), 2.86-2.69 (m, 2H), 2.05-2.00 (m, 2H), 1.30 (t, $J$=7.6 Hz, 6H).

Step 2

To a reaction flask were added compound 1-Z01-L-1 (399 mg, 1.36 mmol), docetaxel (1.0 g, 1.23 mmol), EDCI (261 mg, 1.36 mmol) and DMAP (166 mg, 1.36 mmol), followed by anhydrous DMF (10 mL). The mixture was stirred at

room temperature overnight. Saturated sodium bicarbonate solution and ethyl acetate were added. The aqueous phase was extracted with ethyl acetate. The organic phases were combined, washed with saturated brine, dried over anhydrous sodium sulfate, filtered and concentrated under reduced pressure. The residue was purified by column chromatography to give compound **1-Z01-L-2** (798 mg, yield: 54%).

MS-ESI: m/z 1083.4 $[M+H]^+$.

Step 3

To a reaction flask were added compound **1-Z01-L-2** (790 mg, 0.729 mmol) and wet palladium on carbon (10 wt.%, 158 mg), followed by tetrahydrofuran (16 mL). The system was purged 3 times with hydrogen. The mixture was stirred at room temperature for 18 h. The reaction mixture was filtered through celite. The filter cake was rinsed with ethyl acetate. The filtrate was concentrated and dried to give compound **1-Z01-L** (616 mg, yield: 85%).

MS-ESI: m/z 993.4 $[M+H]^+$.

Step 4

Compound **1-Z01-L** (530 mg, 0.53 mmol) and PyBOP (537 mg, 1.03 mmol) were dissolved in anhydrous DMF (9.0 mL) under nitrogen atmosphere, and the solution was well stirred. Then a solution of dendrimer **1** (629 mg, $13.4 \times 10^{-3}$ mmol) and DIPEA (133 mg, 1.03 mmol) in anhydrous DMF (9.0 mL) was added dropwise to the above reaction solution. The mixture was reacted for 2 h. The reaction mixture was diluted with acetonitrile and then purified by ultrafiltration with acetonitrile in an ultrafiltration (10 KD, Hydrosart®) device to give a crude product (0.66 g). The product was dissolved in pure water (100 mL). The solution was filtered through a filter membrane (0.22 μm) and lyophilized to give compound **1-Z00-L** (0.65 g, yield: 71%).

$^1H$ NMR indicated 26 DTX/dendrimer. The actual molecular weight was approximately 68.8 kDa (30.51% wt.% DTX).

$^1H$ NMR (400 MHz, $CD_3OD$) δ (ppm) 7.20-8.25 (m, 288 H), 5.98-6.30 (m, 26 H), 5.52-5.78 (m, 28 H), 5.15-5.45 (m, 84 H), 4.92-5.08 (s, 31 H), 4.10-4.64 (m, 212 H), 3.40-4.08 (m, 3200 H), 3.33-3.37 (S, 102 H), 2.93-3.23 (m, 172 H), 2.12-2.63 (m, 262 H), 0.99-2.10 (m, 1186 H).

Example 7: Preparation of Compound **1-Z00-M**

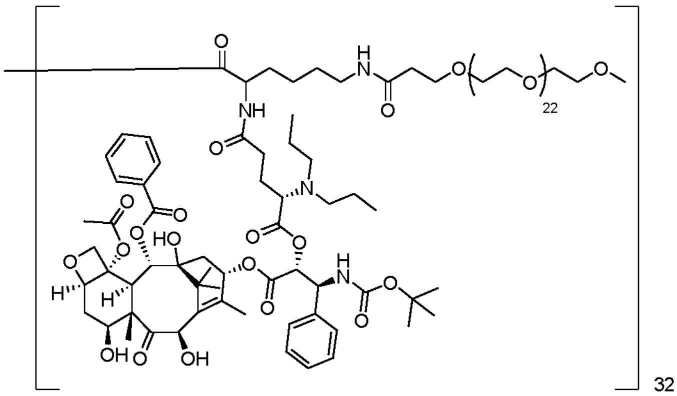

1-Z00-M

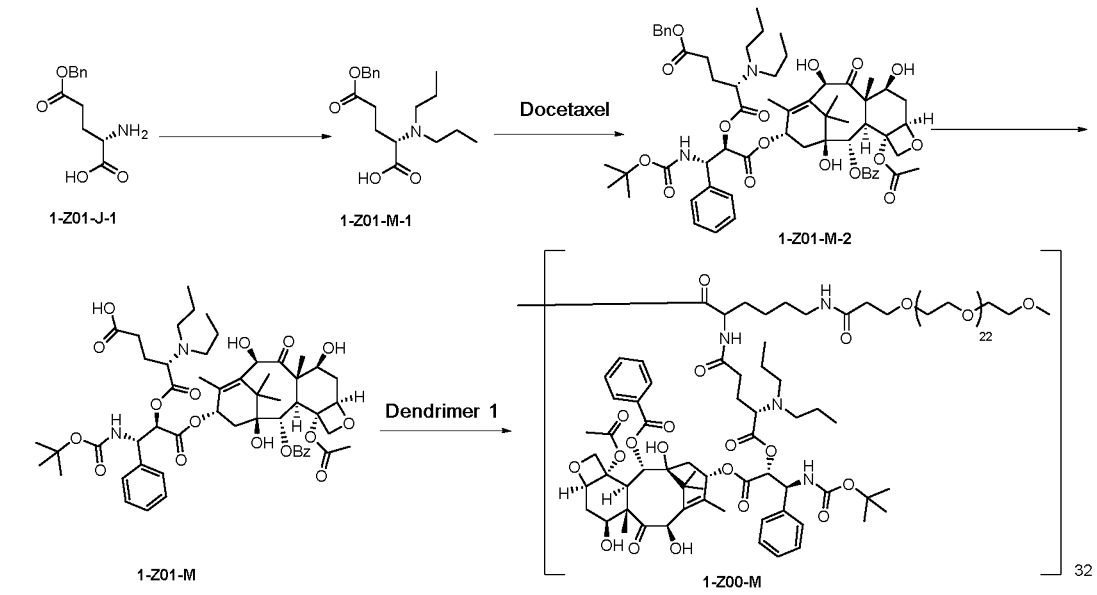


Step 1

Compound **1-Z01-J-1** (5.1 g, 21.496 mmol, Shanghai Bide Pharmatech) was dissolved in MeOH (100 mL) with stirring, and then propionaldehyde (6.3 g, 108.47 mmol) was added. $NaBH(AcO)_3$ (22.9 g, 108.02 mmol) was added with cooling in an ice bath. After the addition, the mixture was reacted at room temperature for 20 h. The reaction mixture was concentrated under reduced pressure to remove methanol. The residue was stirred with water and insoluble matter was filtered out. The filtrate was purified by column chromatography to give compound **1-Z01-M-1** (4.85 g, yield: 68.7%).

MS-ESI: m/z 322.2[M+H]$^+$.

$^1$H-NMR ($CDCl_3$, 400 MHz) $\delta$ 7.37-7.26 (m, 5 H), 5.12 (s, 2 H), 3.53 (brs 1 H), 3.04-2.72 (m, 7 H), 2.09-1.98 (m, 2 H), 1.79-1.67 (m, 4 H), 0.97-0.93 (m, 6 H).

Step 2

To a reaction flask were added under nitrogen atmosphere compound **1-Z01-M-1** (367 mg, 1.1 mmol), docetaxel (801 g, 0.991 mmol), EDCI (212 mg, 1.1 mmol) and DMAP (136 mg, 1.1 mmol), followed by anhydrous DMF (10 mL). The mixture was stirred at room temperature overnight. Saturated sodium bicarbonate solution and ethyl acetate were added. The aqueous phase was extracted with ethyl acetate. The organic phases were combined, washed with saturated brine, dried over anhydrous sodium sulfate and filtered. The filtrate was concentrated under reduced pressure. The residue was purified by column chromatography to give compound **1-Z01-M-2** (732 mg, yield: 66%).

MS-ESI: m/z 1111.4 [M+H]$^+$.

Step 3

To a reaction flask were added compound **1-Z01-M-2** (732 mg, 0.658 mmol) and wet palladium on carbon (10 wt.%, 133 mg), followed by tetrahydrofuran (18 mL). The system was purged 3 times with hydrogen. The mixture was stirred at room temperature for 18 h. The reaction mixture was filtered through celite. The filter cake was rinsed with ethyl acetate. The filtrate was concentrated under reduced pressure to give compound **1-Z01-M** (669 mg, yield: 99%).

MS-ESI: m/z 1021.4 [M+H]$^+$.

Step 4

Compound **1-Z01-M** (500 mg, 0.49 mmol) and PyBOP (490 mg, 0.943 mmol) were dissolved in anhydrous DMF (7.5 mL) under nitrogen atmosphere, and the solution was well stirred. Then a solution of dendrimer **1** (543 mg, 12.25 $\times$ $10^{-3}$ mmol) and DIPEA (122 mg, 0.943 mmol) in anhydrous DMF (7.5 mL) was added dropwise to the above reaction solution. The mixture was reacted for 2 h. The reaction mixture was diluted with acetonitrile and then purified by ultrafiltration with acetonitrile in an ultrafiltration (10 KD, Hydrosart®) device to give a crude product (0.64 g). The product was dissolved in pure water (100 mL). The solution was filtered through a filter membrane (0.22 μm) and lyophilized to give compound **1-Z00-M** (0.63 g, yield: 83%).

$^1$H NMR indicated 21 DTX/dendrimer. The actual molecular weight was approximately 61.7 kDa (27.4% wt.% DTX).

$^1$H NMR (400 MHz, $CD_3OD$) δ (ppm) 8.00-8.22 (m, 45 H), 7.09-7.81 (m, 198 H), 5.95-6.30 (m, 21 H), 5.51-5.75 (m, 20 H), 5.13-5.45 (m, 62 H), 4.93-5.07 (s, 17 H), 4.10-4.64 (m, 175 H), 3.40-4.08 (m, 3000 H), 3.33-3.37 (S, 91 H), 2.93-3.23 (m, 127 H), 2.12-2.63 (m, 217H), 0.79-2.10 (m, 1396 H).

Example 8: Preparation of Compound **1-Z00-Q**

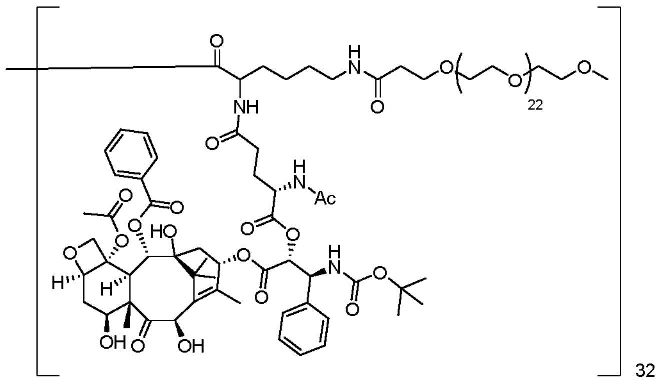

**1-Z00-Q**

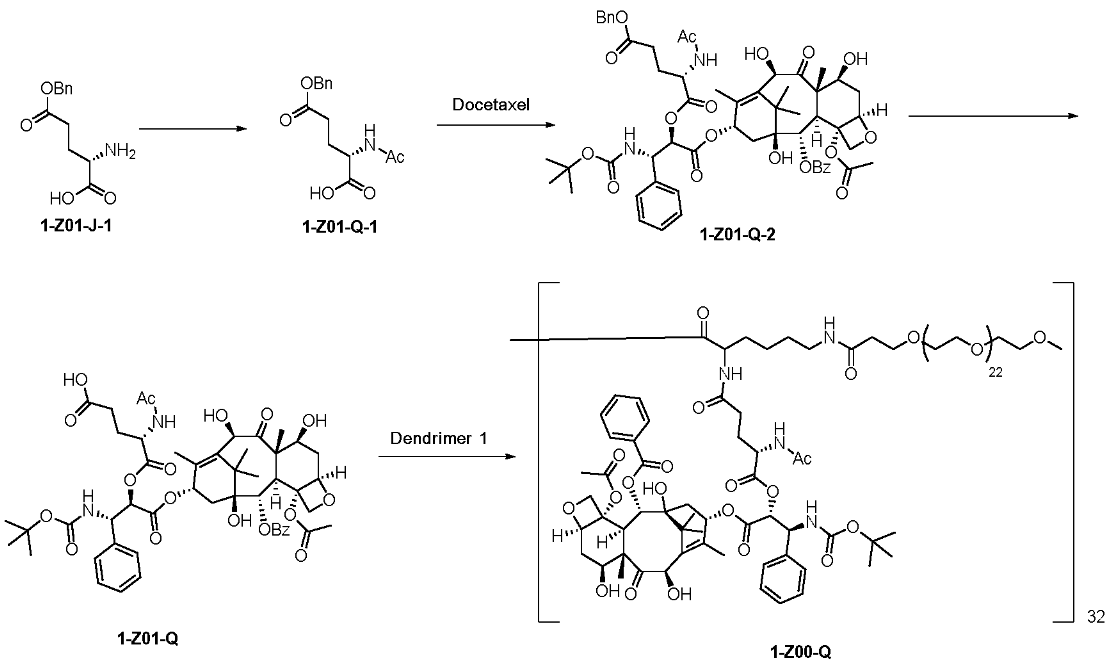


**1-Z01-J-1** **1-Z01-Q-1** **1-Z01-Q-2** **1-Z01-Q** **1-Z00-Q**

Step 1

Compound **1-Z01-J-1** (5 g, 21.1 mmol, Shanghai Bide Pharmatech) was dissolved in DCM (50 mL). Triethylamine (6.41 g, 63.3 mmol) was added under nitrogen atmosphere. The mixture was cooled in an ice-water bath. Acetic anhydride (2.37 g, 23.2 mmol) was added dropwise. The mixture was reacted at room temperature overnight. The reaction mixture was washed with water. The aqueous phases were combined, adjusted to pH 1 with concentrated hydrochloric acid under ice-water bath conditions and extracted three times with ethyl acetate. The organic phases were combined, dried over anhydrous sodium sulfate, filtered and concentrated to give a white solid (5.8 g). The white solid was triturated with a mixed solution of ethyl acetate and petroleum ether overnight. The mixture was filtered to give compound **1-Z01-Q-1** (4.9 g, yield: 83%). The product was directly used in the next step.

MS-ESI: m/z 280.2 $[M+H]^+$.

$^1H$ NMR (400 MHz, DMSO-$d_6$) δ 12.62 (br, 1 H), 8.14 (d, *J* = 8 Hz, 1 H), 7.40-7.31 (m, 5 H), 5.09 (s, 2 H), 4.24-4.18 (m, 1 H), 2.48-2.38 (m, 2 H), 2.05-2.00 (m, 1 H), 1.98-1.77 (m, 1 H), 1.97 (s, 3 H).

Step 2

Compound **1-Z01-Q-1** (377 mg, 1.350 mmol), docetaxel (1 g, 1.239 mmol) and DMAP (166 mg, 1.363 mmol) were weighed into a reaction flask. Dry DMF (10 mL) and EDCI (261 mg, 1.363 mmol) were added under nitrogen atmosphere and ice-water bath conditions. After the addition, the mixture was reacted at room temperature overnight. Ethyl acetate was added to the reaction mixture. The organic phase was washed sequentially with saturated sodium bicarbonate solution and saturated NaCl solution, dried over anhydrous sodium sulfate, filtered and concentrated. The residue was purified by column chromatography to give compound **1-Z01-Q-2** (805 mg, yield: 60.8%).

MS-ESI: m/z 1069.5 $[M+H]^+$.

Step 3

Compound **1-Z01-Q-2** (1.1 g, 1.029 mmol) was dissolved in THF (20 mL). Wet palladium on carbon (10 wt.%, 220 mg) was added. The system was purged three times with hydrogen, and then the mixture was stirred overnight at room temperature under hydrogen atmosphere. The reaction mixture was filtered through celite. The filtrate was concentrated under reduced pressure and dried *in vacuo* to give compound **1-Z01-Q** (962 mg, yield: 95.5%).

MS-ESI: m/z 979.4 $[M+H]^+$.

Step 4

Compound **1-Z01-Q** (500 mg, 0.511 mmol) and PyBOP (513 mg, 0.986 mmol) were dissolved in anhydrous DMF (7.9 mL) under nitrogen atmosphere, and the solution was well stirred. Then a solution of dendrimer 1 (567 mg, $12.8 \times 10^{-3}$ mmol) and DIPEA (127 mg, 0.986 mol) in anhydrous DMF (7.9 mL) was added dropwise to the above reaction solution. The mixture was reacted for 2 h. The reaction mixture was diluted with acetonitrile and then purified by ultrafiltration with acetonitrile in an ultrafiltration (10 KD, Hydrosart®) device to give a crude product (0.625 g). The product was dissolved in pure water (100 mL). The solution was filtered through a filter membrane (0.22 μm) and lyophilized to give compound **1-Z00-Q** (0.62 g, yield: 74%).

$^1H$ NMR indicated 26 DTX/dendrimer. The actual molecular weight was approximately 65.7 kDa (31.96% wt.% DTX).

$^1H$ NMR (400 MHz, $CD_3OD$) δ 8.00-8.22 (m, 55 H), 7.09-7.81 (m, 253 H), 5.95-6.31 (m, 26 H), 5.51-5.77 (m, 28 H), 5.13-5.45 (m, 91H), 4.92-5.08 (s, 41 H), 4.10-4.64 (m, 225 H), 3.40-4.08 (m, 3000 H), 3.33-3.37 (s, 91 H), 2.93-3.23 (m, 102 H), 2.12-2.63 (m, 310 H), 0.96-2.10 (m, 1119 H).

Example 9: Preparation of Compound **3-L00**

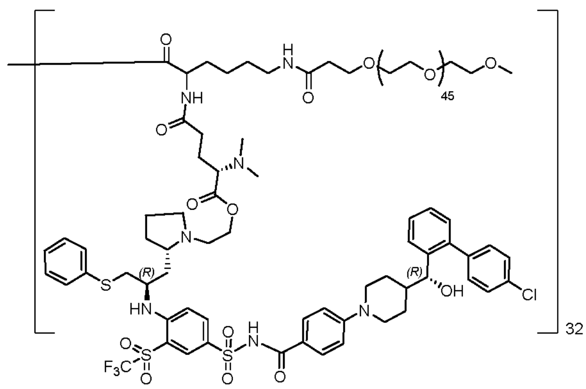

**3-L00**

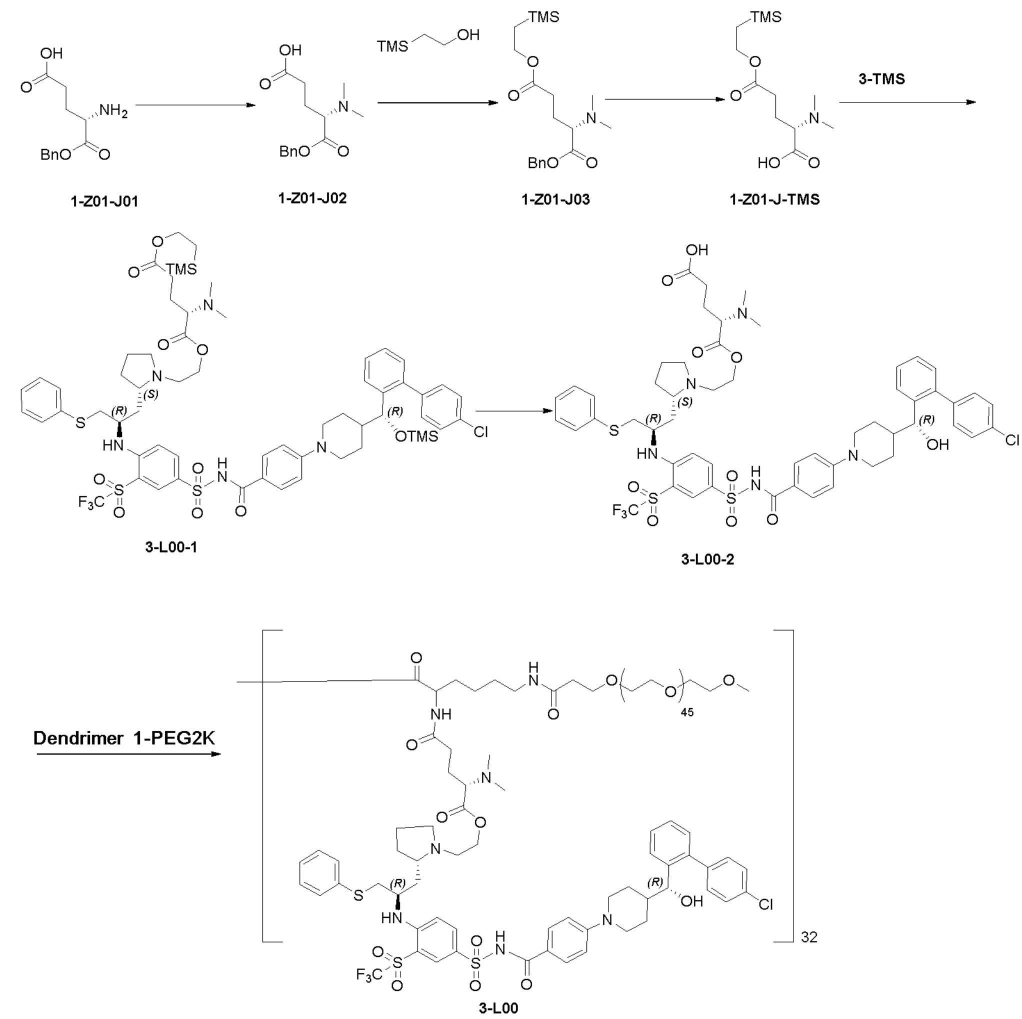


1-Z01-J01 1-Z01-J02 1-Z01-J03 1-Z01-J-TMS

3-L00-1 3-L00-2

3-L00

Step 1

Compound **1-Z01-J01** (5.0 g, 18.3 mmol) was dissolved in methanol (100 mL). The solution was cooled to 0 °C. An aqueous solution of formaldehyde (10 mL, 40 wt.%) and $NaBH(OAc)_3$ (20.0 g, 91.6 mmol) were added. The mixture was reacted at room temperature overnight. After concentration, the residue was purified by column chromatography to give compound **1-Z01-J02** (3.4 g, yield: 61%).

MS-ESI: m/z 266.1 $[M+H]^+$.

Step 2

Compound **1-Z01-J02** (3.4 g, 12.8 mmol), trimethylsilyl ethanol (3.0 g, 26.0 mmol), DMAP (780 mg, 6.4 mmol) and *N,N*-diisopropylethylamine (6.6 g, 51.2 mmol) were dissolved in anhydrous tetrahydrofuran (30 mL). The solution was cooled to 0 °C. HATU (6.3 g, 16.6 mmol) was added. The mixture was reacted at room temperature for 16 h. The mixture was quenched with water (50 mL) and extracted with ethyl acetate (50 mL × 3). After concentration, the residue was purified by column chromatography to give compound **1-Z01-J03** (4.0 g, yield: 85%).

MS-ESI: m/z 366.2 $[M+H]^+$.

Step 3

Compound **1-Z01-J03** (4.0 g, 10.9 mmol) was dissolved in anhydrous tetrahydrofuran (40 mL). Pd/C (200 mg, 10 wt.%) was added. The system was purged three times with hydrogen. The mixture was reacted at room temperature for 16 h. After filtration and concentration, a crude product (3.6 g) was obtained. The product was triturated with anhydrous tetrahydrofuran (30 mL) for 30 min. The mixture was filtered to give compound **1-Z01-J-TMS** (1.6 g, yield: 45%).

MS-ESI: m/z 276.1 $[M+H]^+$.

$^1H$ NMR (400MHz, $CDCl_3$) δ 4.14-4.18 (m, 2H), 3.44-3.47 (m, 1H), 2.87(s, 6H), 2.66-2.70 (m,2H), 2.03-2.12 (m, 2H), 0.96-0.99 (m,2H), 0.04 (s, 9H).

Step 4

Compound **1-Z01-J-TMS** (118 mg, 0.431 mmol), 3-TMS (300 mg, 0.287 mmol) and DMAP (17.5 mg, 0.216 mmol) were dissolved in anhydrous dichloromethane (10 mL). DCC (88.8 mg, 0.431 mmol) was added. The mixture was reacted at room temperature for 16 h. After filtration and concentration, the residue was purified by column chromatography to give compound **3-L00-1** (373 mg, yield: 98%).

MS-ESI: m/z 650.8 $[1/2M+H]^+$.

Step 5

Compound **3-L00-1** (373 mg, 0.288 mmol) was dissolved in anhydrous tetrahydrofuran (3 mL). TBAF (2.9 mL, 2.88 mmol, 1.0 mol/L in THF) was added. The mixture was reacted at room temperature for 2 h. The mixture was quenched with water (10 mL) and extracted with ethyl acetate (10 mL × 3). After concentration, the residue was purified by column chromatography to give compound **3-L00-2** (238 mg, yield: 73%).

MS-ESI: m/z 564.5 $[1/2M+H]^+$.

Step 6

Compound **3-L00-2** (0.390 g, 0.346 mmol) and PyBOP (0.216 g, 0.415 mmol) were dissolved in anhydrous *N*,*N*-dimethylformamide (5.4 mL) under nitrogen atmosphere. A solution of dendrimer 1-PEG2K (0.535 g, 6.92 μmol, synthesized according to Journal of Controlled Release, 2011, 152, 241-248) and NMM (0.139 g, 1.38 mmol) in anhydrous *N,N*-dimethylformamide (5.4 mL) was added dropwise. The mixture was reacted at room temperature for 2 h. The reaction mixture was diluted with acetonitrile (11 mL) and then purified by ultrafiltration with acetonitrile (0.55 L, 25 V) in an ultrafiltration (10 KD, Hydrosart®) device to give a crude product (0.625 g). The product was dissolved in pure water. The solution was filtered through a filter membrane (0.22 μm) and lyophilized to give compound **3-L00** (0.62 g, yield: 89%).

$^1H$ NMR indicated 24 drug molecules/dendrimer. The actual molecular weight was approximately 100.2 kDa (23.22% wt.% compound 3).

$^1H$ NMR (400 MHz, $CD_3OD$) δ6.40-8.51 (m, 498H),4.19-4.68 (m, 111H), 3.40-4.10 (m, 5900H), 3.33-3.36 (s, 106H), 2.79-3.20 (m, 112H), 2.22-2.74 (m, 246H), 0.61-1.92 (m, 643H).

Example 10: Preparation of Compound **3-M00**

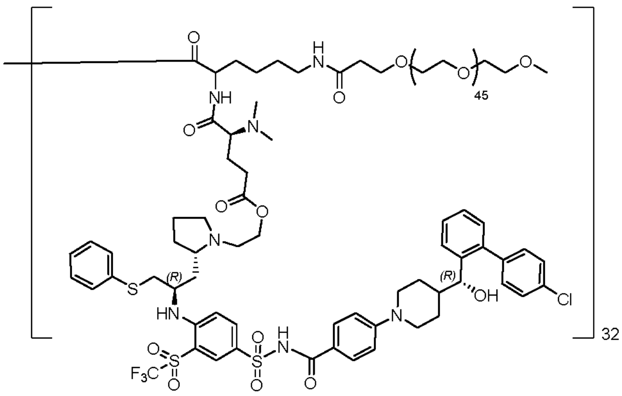

**3-M00**

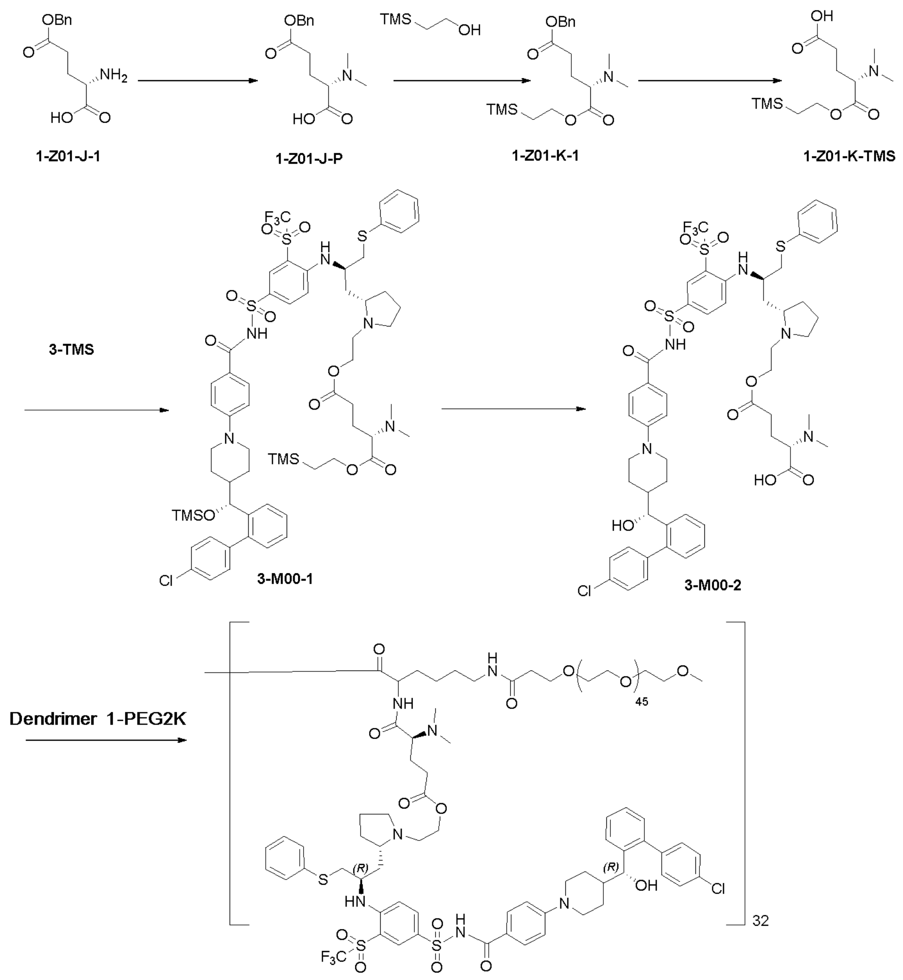


1-Z01-J-1 1-Z01-J-P 1-Z01-K-1 1-Z01-K-TMS

3-M00-1 3-M00-2

3-M00

Step 1

Compound **1-Z01-J-1** (7 g, 29.5 mmol) was dissolved in methanol (150 mL). An aqueous solution of formaldehyde (15 mL, 40 wt.%) and $NaBH(OAc)_3$ (63 g, 295 mmol) were added. The mixture was reacted at room temperature for 1 h. After concentration, the residue was purified by column chromatography to give compound **1-Z01-J-P** (5.9 g, yield: 75%).

MS-ESI: m/z 266.2 $[M+H]^+$.

$^1H$ NMR (400 MHz, DMSO-$d_6$) $\delta$ 7.36-7.32 (m, 5H), 5.09 (s, 2H), 3.12 (t, $J$ = 7.2 Hz, 1H), 2.5-2.44 (m, 2H), 2.38 (s, 6H), 1.87-1.84 (m, 2H).

Step 2

Compound **1-Z01-J-P** (4.2 g, 15.8 mmol), trimethylsilyl ethanol (2.24 g, 19 mmol), DMAP (0.193 g, 1.58 mmol) and N,N-diisopropylethylamine (6.1 g, 47.5 mmol) were dissolved in dichloromethane (42 mL). The solution was cooled to 0 °C. HATU (7.2 g, 19 mmol) was added. The mixture was reacted at room temperature for 16 h. The mixture was quenched with saturated sodium bicarbonate solution (150 mL) and extracted with dichloromethane (50 mL × 3). After concentration, the residue was purified by column chromatography to give compound **1-Z01-K-1** (4.9 g, yield: 86%).

MS-ESI: m/z 366.2 $[M+H]^+$.

Step 3

Compound **1-Z01-K-1** (900 mg, 2.46 mmol) was dissolved in tetrahydrofuran (18 mL) under nitrogen atmosphere. Pd/C (180 mg, 10 wt.%) was added. The system was purged three times with hydrogen. The mixture was reacted at room temperature for 16 h. The mixture was filtered and concentrated to give compound **1-Z01-K-TMS** (670 mg, yield: 99%).

MS-ESI: m/z 276.1 $[M+H]^+$.

$^1H$ NMR (400 MHz, $CDCl_3$) δ 4.22 (dd, *J*=10.0 Hz, J=8.4 Hz, 2H), 3.39 (t, *J* = 7.2 Hz, 1H), 2.65-2.58 (m, 1H), 2.49 (s, 6H), 2.47-2.39 (m, 1H), 2.02 (dd, *J*=12.8 Hz, *J*=6.8 Hz, 2H), 1.04-1.00 (m,2H), 0.04 (s, 9H).

Step 4

Compound 3-TMS (800 mg, 0.767 mmol), **1-Z01-K-TMS** (316 mg, 0.767 mmol) and DMAP (46 mg, 0.383 mmol) were dissolved in dichloromethane (16 mL) under nitrogen atmosphere. The solution was cooled to 0 °C. DCC (237 mg, 1.15 mmol) was added. The mixture was reacted at room temperature for 18 h. After filtration and concentration, the residue was purified by column chromatography to give compound **3-M00-1** (1.036 g, yield: 100%).

MS-ESI: m/z 650.8 $[1/2M+H]^+$.

Step 5

Compound **3-M00-1** (1.0 g, 0.768 mmol) was dissolved in tetrahydrofuran (10 mL) under nitrogen atmosphere. The solution was cooled to 0 °C. TBAF (7.7 mL, 7.68 mmol, 1.0 mol/L in THF) was added. The mixture was reacted at room temperature for 1 h. The mixture was quenched with water (30 mL) and extracted with ethyl acetate (50 mL × 3). After concentration, the residue was purified by column chromatography to give compound **3-M00-2** (670 mg, yield: 77%).

MS-ESI: m/z 1128.3 $[M+H]^+$.

Step 6

Compound **3-M00-2** (0.400 g, 0.355 mmol) and PyBOP (0.222 g, 0.426 mmol) were dissolved in anhydrous *N*,*N*-dimethylformamide (5.6 mL) under nitrogen atmosphere. A solution of dendrimer 1-PEG2K (0.548 g, 7.10 μmol) and NMM (0.144 g, 1.42 mmol) in anhydrous *N,N*-dimethylformamide (5.6 mL) was added dropwise. The mixture was reacted at room temperature for 2 h. The reaction mixture was diluted with acetonitrile (11 mL) and then purified by ultrafiltration with acetonitrile (0.55 L, 25 V) in an ultrafiltration (10 KD, Hydrosart®) device to give a crude product (0.614 g). The product was dissolved in pure water. The solution was filtered through a filter membrane (0.22 μm) and lyophilized to give compound **3-M00** (0.61 g, yield: 88%). $^1H$ NMR indicated 22 drug molecules/dendrimer. The actual molecular weight was approximately 98.0 kDa (21.77% wt.% compound 3).

$^1H$ NMR (400 MHz, $CD_3OD$) δ6.40-8.52 (m, 440H), 4.13-4.70 (m, 137H), 3.40-4.10 (m, 5900H), 3.33-3.36 (s, 109H), 2.79-3.25 (m, 99H), 2.22-2.74 (m, 246H), 0.61-1.92 (m, 622H).

Example 11: Preparation of Compound **3-N00**

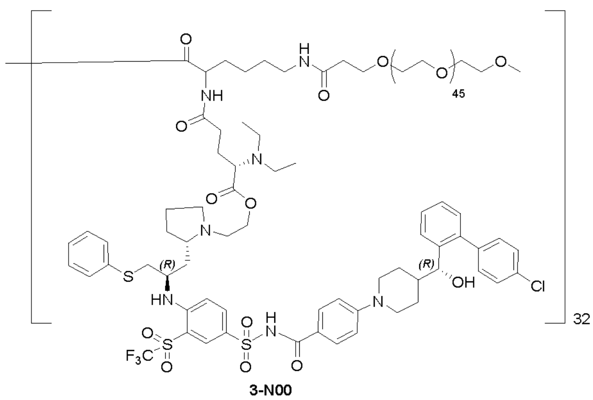

3-N00

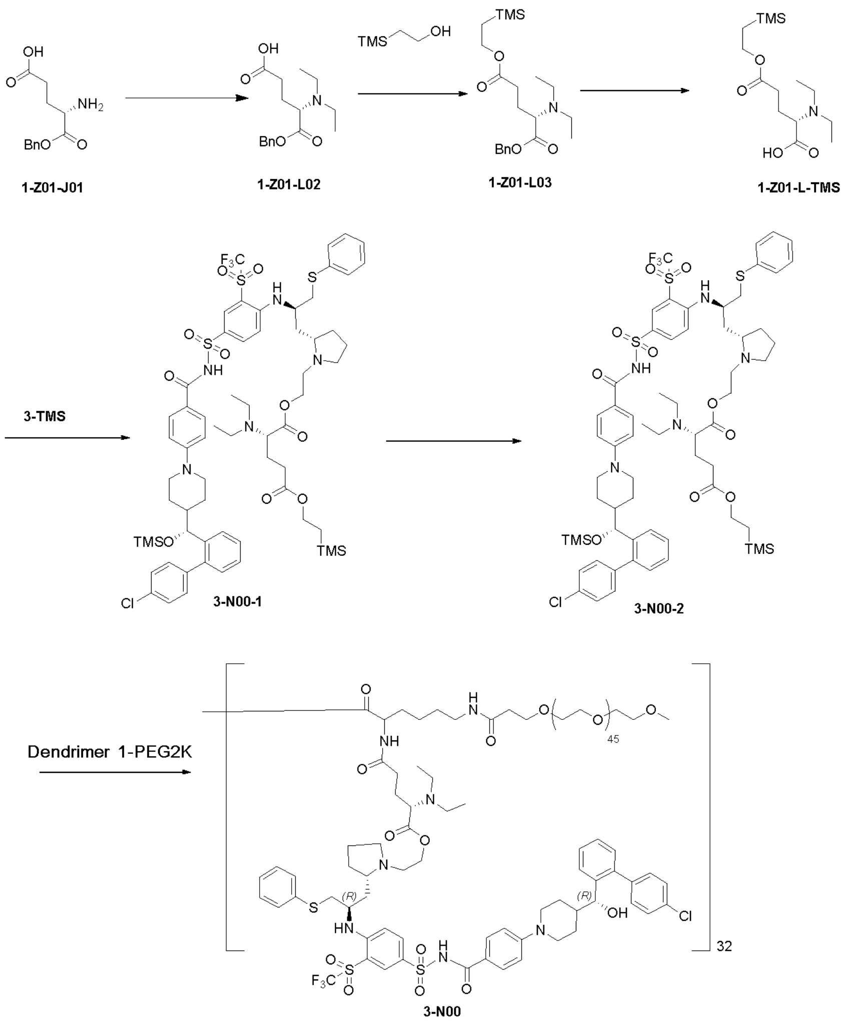


1-Z01-J01 1-Z01-L02 1-Z01-L03 1-Z01-L-TMS

3-N00-1 3-N00-2

3-N00

Step 1

Compound **1-Z01-J01** (10.0 g, 36.6 mmol) was dissolved in methanol (200 mL). The solution was cooled to 0 °C. An aqueous solution of acetaldehyde (20 mL, 40 wt.%) and then $NaBH(OAc)_3$ (40.0 g, 183.2 mmol) were added. The mixture was reacted at room temperature overnight. After concentration, the residue was purified by column chromatography to give compound **1-Z01-L02** (5.4 g, yield: 50.4%).

MS-ESI: m/z 294.1 $[M+H]^+$.

Step 2

Compound **1-Z01-L02** (500 mg, 1.7 mmol), trimethylsilyl ethanol (404 mg, 3.4 mmol), DMAP (104 mg, 0.85 mmol) and *N*,*N*-diisopropylethylamine (658 mg, 5.1 mmol) were dissolved in anhydrous tetrahydrofuran (10 mL). The solution was cooled to 0 °C. HATU (840 mg, 2.2 mmol) was added. The mixture was reacted at room temperature for 16 h. The mixture was quenched with water (20 mL) and extracted with ethyl acetate (20 mL × 3). After concentration, the residue was purified by column chromatography to give compound **1-Z01-L03** (476 mg, yield: 71%).

MS-ESI: m/z 394.2 $[M+H]^+$.

Step 3

Compound **1-Z01-L03** (476 mg, 1.2 mmol) was dissolved in anhydrous tetrahydrofuran (10 mL). Pd/C (50 mg, 10 wt.%) was added. The system was purged three times with hydrogen. The mixture was reacted at room temperature for 16 h. The mixture was filtered and concentrated to give compound **1-Z01-L-TMS** (366 mg, yield: 100%).

MS-ESI: m/z 304.2 $[M+H]^+$.

$^1H$ NMR (400 MHz, $CD_3OD$) δ 4.14-4.18 (m, 2H), 3.58-3.60 (m, 1H), 3.26-3.30 (m, 2H), 3.06-3.13 (m, 2H),

2.66-2.77 (m,2H), 2.00-2.06 (m, 2H), 1.33-1.38 (m,6H),0.94-0.99 (m,2H),0.04 (s, 9H).

Step 4

Compound **1-Z01-L-TMS** (366 mg, 1.21 mmol), 3-TMS (800 mg, 0.767 mmol) and DMAP (47.0 mg, 0.38 mmol) were dissolved in anhydrous dichloromethane (25 mL). DCC (246.0 mg, 1.2 mmol) was added. The mixture was reacted at room temperature for 16 h. After filtration and concentration, the residue was purified by column chromatography to give compound **3-N00-1** (1.0 g, yield: 98%).

MS-ESI: m/z 664.8 [1/2M+H]$^+$.

Step 5

Compound **3-N00-1** (1.0 g, 0.75 mmol) was dissolved in anhydrous tetrahydrofuran (10 mL). TBAF (7.5 mL, 7.5 mmol, 1.0 mol/L in THF) was added. The mixture was reacted at room temperature for 2 h. The mixture was quenched with water (20 mL) and extracted with ethyl acetate (50 mL × 3). After concentration, the residue was purified by column chromatography to give compound **3-N00-2** (740 mg, yield: 85%).

MS-ESI: m/z 578.7 [1/2M+H]$^+$.

Step 6

Compound **3-N00-2** (0.400 g, 0.346 mmol) and PyBOP (0.215 g, 0.414 mmol) were dissolved in anhydrous *N*,*N*-dimethylformamide (5.6 mL) under nitrogen atmosphere. A solution of the compound dendrimer 1-PEG2K (0.535 g, 6.90 μmol) and NMM (0.139 g, 1.38 mmol) in anhydrous *N,N*-dimethylformamide (5.6 mL) was added dropwise. The mixture was reacted at room temperature for 2 h. The reaction mixture was diluted with acetonitrile (11 mL) and then purified by ultrafiltration with acetonitrile (0.56 L, 25 V) in an ultrafiltration (10 KD, Hydrosart®) device to give a crude product (0.631 g). The product was dissolved in pure water. The solution was filtered through a filter membrane (0.22 μm) and lyophilized to give compound **3-N00** (0.625 g, yield: 91%).

$^1$H NMR indicated 23 drug molecules/dendrimer. The actual molecular weight was approximately 99.8 kDa (22.36% wt.% compound 3).

$^1$H NMR (400 MHz, $CD_3OD$) δ6.40-8.52 (m, 477H), 4.19-4.68 (m, 184H), 3.40-4.10 (m, 5900H), 3.33-3.36 (s, 108H), 2.79-3.20 (m, 101H), 2.22-2.74 (m, 158H), 0.61-1.92 (m, 726H).

Example 12: Preparation of Compound **3-O00**

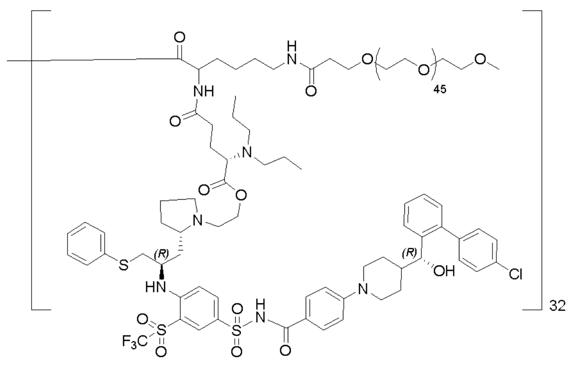

**3-O00**

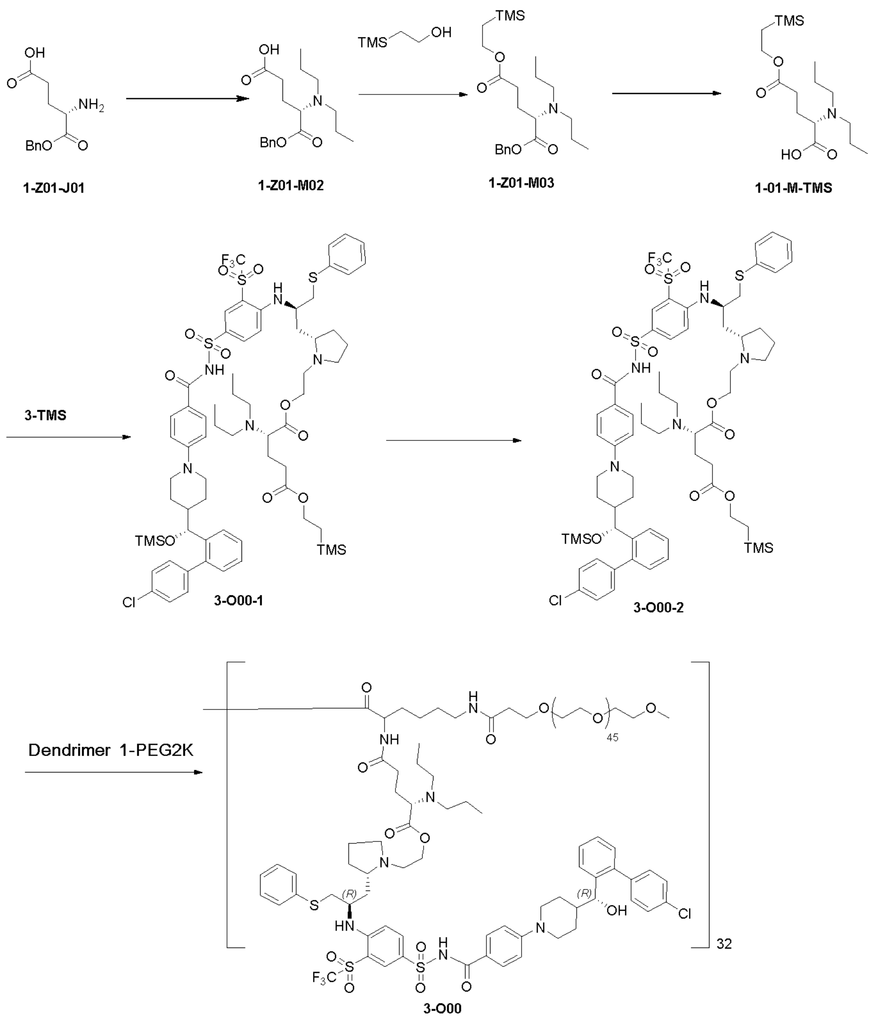


1-Z01-J01 1-Z01-M02 1-Z01-M03 1-01-M-TMS 3-O00-1 3-O00-2 3-O00

Step 1

Compound **1-Z01-J01** (5.0 g, 21.1 mmol) was dissolved in methanol (100 mL). The solution was cooled to 0 °C. Propionaldehyde (6.1 g, 105.0 mmol) and $NaBH(OAc)_3$ (31.1 g, 105.0 mmol) were added. The mixture was reacted at room temperature overnight. After concentration, the residue was purified by column chromatography to give compound **1-Z01-M02** (5.0 g, yield: 74%).

MS-ESI: m/z 322.1 $[M+H]^+$.

Step 2

Compound **1-Z01-M02** (890 mg, 2.8 mmol), trimethylsilyl ethanol (393.3 mg, 3.3 mmol), DMAP (33.8 mg, 0.3 mmol) and *N,N*-diisopropylethylamine (1.1 g, 9.3 mmol) were dissolved in anhydrous dichloromethane (20 mL). The solution was cooled to 0 °C. HATU (1.3 g, 3.3 mmol) was added. The mixture was reacted at room temperature for 16 h. The mixture was quenched with water (40 mL) and extracted with ethyl acetate (20 mL $\times$ 3). After concentration, the residue was purified by column chromatography to give compound **1-Z01-M03** (1 g, yield: 86%).

MS-ESI: m/z 422.2 $[M+H]^+$.

Step 3

Compound **1-Z01-M03** (1 g, 2.4 mmol) was dissolved in anhydrous tetrahydrofuran (30 mL). Pd/C (200 mg, 10 wt.%) was added. The system was purged three times with hydrogen. The mixture was reacted at room temperature for 16 h. The mixture was filtered and concentrated to give compound **1-Z01-M-TMS** (766 mg, yield: 100%). MS-ESI: m/z 332.2 $[M+H]^+$.

$^1H$ NMR (400 MHz, $CDCl_3$) $\delta$ 4.10-4.14 (m, 2H), 3.50-3.53 (m, 1H), 2.84-3.04 (m, 4H), 2.63-2.69 (m,2H),

1.95-2.01 (m, 2H), 1.69-1.77 (m,4H), 0.90-0.96 (m,8H), 0.04 (s, 9H).

Step 4

Compound **1-Z01-M-TMS** (381.7 mg, 1.2 mmol), 3-TMS (800 mg, 0.767 mmol) and DMAP (46.08 mg, 0.38 mmol) were dissolved in anhydrous dichloromethane (20 mL). DCC (237.0 mg, 1.2 mmol) was added. The mixture was reacted at room temperature for 16 h. After filtration and concentration, the residue was purified by column chromatography to give compound **3-000-1** (890 mg, yield: 86%).

MS-ESI: m/z 678.8 [1/2M+H]$^+$.

Step 5

Compound **3-000-1** (890 mg, 0.66 mmol) was dissolved in anhydrous tetrahydrofuran (10 mL). TBAF (6.6 mL, 6.6 mmol, 1.0 mol/L in THF) was added. The mixture was reacted at room temperature for 3 h. The mixture was quenched with water (20 mL) and extracted with ethyl acetate (50 mL × 3). After concentration, the residue was purified by column chromatography to give compound **3-O00-2** (810 mg, yield: 100%).

MS-ESI: m/z 592.8 [1/2M+H]$^+$.

Step 6

Compound **3-O00-2** (0.409 g, 0.346 mmol) and PyBOP (0.216 g, 0.415 mmol) were dissolved in anhydrous *N*,*N*-dimethylformamide (5 mL) under nitrogen atmosphere. A solution of dendrimer 1-PEG2K (0.52 g, 6.8 μmol) and NMM (0.137 g, 1.36 mmol) in anhydrous *N,N*-dimethylformamide (5 mL) was added dropwise. The mixture was reacted at room temperature for 2 h. The reaction mixture was diluted with acetonitrile (10 mL) and then purified by ultrafiltration with acetonitrile (0.5 L, 25 V) in an ultrafiltration (10 KD, Hydrosart®) device to give a crude product (0.605 g). The product was dissolved in pure water. The solution was filtered through a filter membrane (0.22 μm) and lyophilized to give compound **3-O00** (0.60 g, yield: 87%).

$^1$H NMR indicated 24 drug molecules/dendrimer. The actual molecular weight was approximately 101.5 kDa (22.92% wt.% compound 3).

$^1$H NMR (400 MHz, $CD_3OD$) δ 6.40-8.51 (m, 496H), 4.19-4.68 (m, 126H), 3.40-4.10 (m, 5900H), 3.33-3.36 (s, 100H), 2.79-3.20 (m, 136H), 2.22-2.74 (m, 199H), 0.61-1.92 (m, 683H).

Example 13: Preparation of Compound **3-R00**

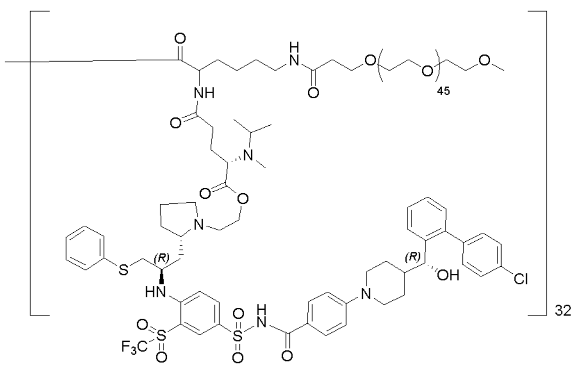

3-R00

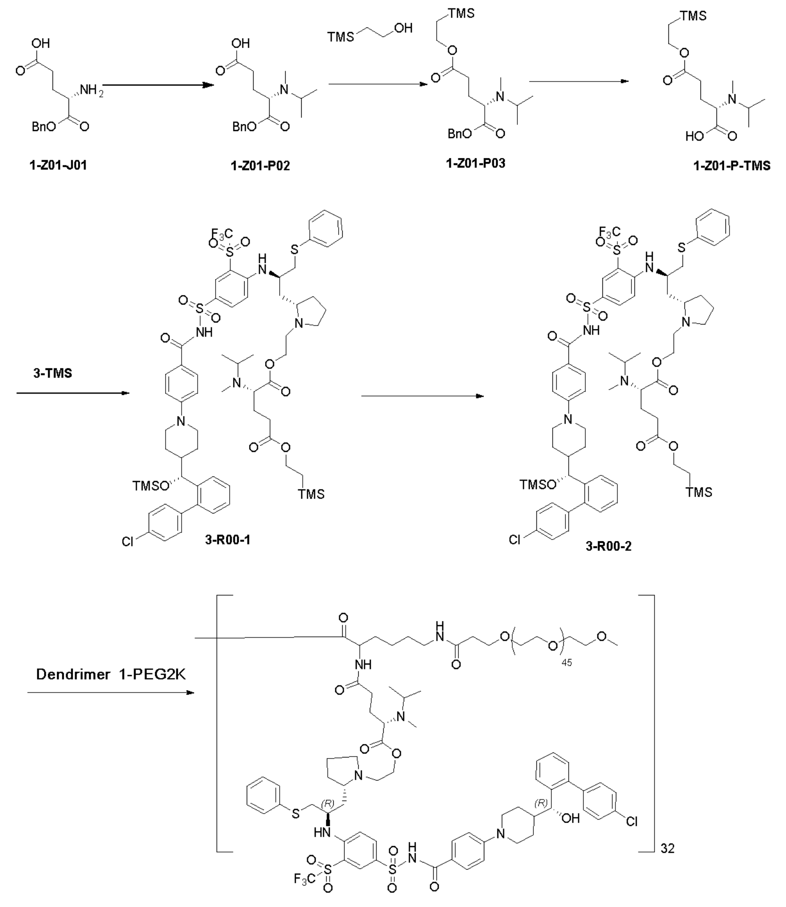


1-Z01-J01 1-Z01-P02 1-Z01-P03 1-Z01-P-TMS

3-R00-1 3-R00-2

3-R00

Step 1

Compound **1-Z01-J01** (5.01 g, 18.3 mmol) was dissolved in methanol (100 mL). The solution was cooled to 0 °C. Acetone (5.5 g, 94.7 mmol) and $NaBH(OAc)_3$ (11.9 g, 56.13 mmol) were added. The mixture was reacted at room temperature for 2 h. An aqueous solution of formaldehyde (14 mL, 40 wt.%) and $NaBH(OAc)_3$ (8.26 g, 38.96 mmol) were added. The mixture was reacted overnight at room temperature. After concentration, the residue was purified by column chromatography to give compound **1-Z01-P02** (5 g, yield: 93%).

MS-ESI: m/z 294.1 $[M+H]^+$.

Step 2

Compound **1-Z01-P02** (2.58 g, 8.8 mmol), trimethylsilyl ethanol (2 g, 16.9 mmol), DMAP (540 mg, 4.43 mmol) and N,N-diisopropylethylamine (2.98 g, 23.1 mmol) were dissolved in anhydrous tetrahydrofuran (20 mL). HATU (3.38 g, 8.89 mmol) was added. The mixture was reacted at room temperature for 17 h. The mixture was quenched with water (20 mL) and extracted with ethyl acetate (20 mL $\times$ 3). After concentration, the residue was purified by column chromatography to give compound **1-Z01-P03** (3.09 g, yield: 89%).

MS-ESI: m/z 394.2 $[M+H]^+$.

Step 3

Compound **1-Z01-P03** (3 g, 7.62 mmol) was dissolved in anhydrous tetrahydrofuran (30 mL). Pd/C (152 mg, 10 wt.%) was added. The system was purged three times with hydrogen. The mixture was reacted at room temperature for 15 h. After filtration and concentration, crude **1-Z01-P-TMS** (2.32 g) was obtained. The crude product was triturated with methyl tert-butyl ether (20 mL) for 30 min. The mixture was filtered to give compound **1-Z01-P-TMS** (0.92 g, yield: 39%).

MS-ESI: m/z 304.2 $[M+H]^+$.

$^1$HNMR (400 MHz, DMSO-$d_6$) $\delta$ 4.05-4.10 (m, 2H), 3.26-3.30 (m, 1H), 3.12-3.15 (m, 1H), 3.35-3.43 (m, 2H),

2.31 (s,3H), 1.75-1.85 (m, 2H), 1.03-1.07 (m,6H), 0.89-0.94 (m,2H),0.01 (s, 9H).

Step 4

Compound **1-Z01-P-TMS** (379 mg, 1.25 mmol), 3-TMS (805 mg, 0.772 mmol) and DMAP (54.0 mg, 0.44 mmol) were dissolved in anhydrous dichloromethane (16 mL). DCC (239.0 mg, 1.16 mmol) was added. The mixture was reacted at room temperature for 16 h. After filtration and concentration, the residue was purified by column chromatography to give compound **3-R00-1** (863 mg, yield: 84%).

MS-ESI: m/z 664.8 $[1/2M+H]^+$.

Step 5

Compound **3-R00-1** (847 mg, 0.64 mmol) was dissolved in anhydrous tetrahydrofuran (6 mL). TBAF (6 mL, 6 mmol, 1.0 mol/L in THF) was added. The mixture was reacted at room temperature for 2 h. The mixture was quenched with water (20 mL) and extracted with ethyl acetate (50 mL $\times$ 3). After concentration, the residue was purified by column chromatography to give compound **3-R00-2** (702 mg, yield: 95%).

MS-ESI: m/z 578.8 $[1/2M+H]^+$.

Step 6

Compound **3-R00-2** (0.299 g, 0.259 mmol) and PyBOP (0.162 g, 0.311 mmol) were dissolved in anhydrous *N,N*-dimethylformamide (4 mL) under nitrogen atmosphere. A solution of the compound dendrimer 1-PEG2K (0.400 g, 5.177 $\mu$mol) and NMM (0.209 g, 2.071 mmol) in anhydrous *N,N*-dimethylformamide (4 mL) was added dropwise. The mixture was heated to 38 °C and reacted for 3 h. The reaction mixture was diluted with acetonitrile and then purified by ultrafiltration with acetonitrile (0.4 L, 13 V) in an ultrafiltration (10 KD, Hydrosart®) device to give a crude product (0.470 g). The product was dissolved in pure water. The solution was filtered through a filter membrane (0.22 $\mu$m) and lyophilized to give compound **3-R00** (0.465 g, yield: 88%). $^1$H NMR indicated 25 drug molecules/dendrimer. The actual molecular weight was approximately 102.0 kDa (23.76% wt.% compound 3).

$^1$H NMR (400 MHz, $CD_3OD$) $\delta$ 6.40-8.51 (m, 514H), 4.13-4.72 (m, 123H), 3.40-4.10 (m, 5900H), 3.33-3.36 (s, 93H), 2.79-3.25 (m, 73H), 2.22-2.74 (m, 206H), 0.61-1.92 (m, 810H).

Example 14: Preparation of Compound **3-S00**

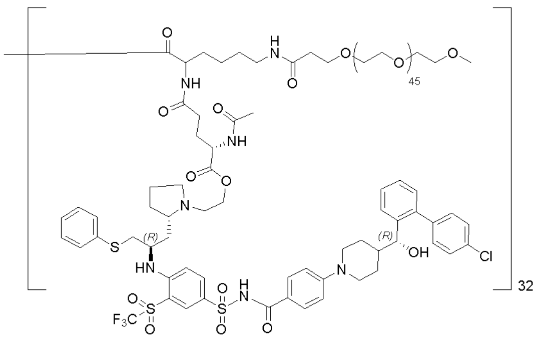

**3-S00**

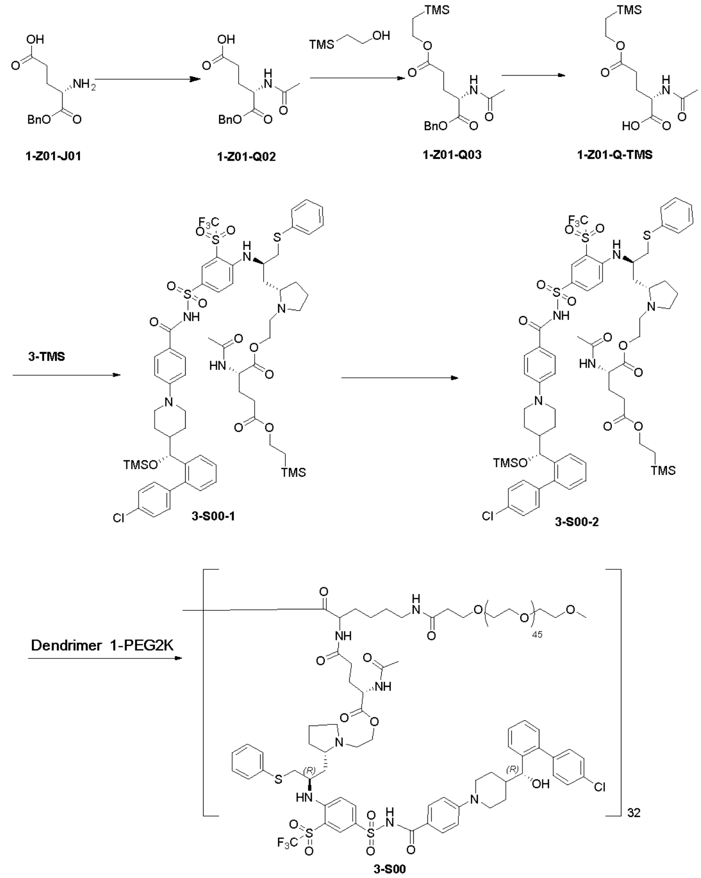


1-Z01-J01 1-Z01-Q02 1-Z01-Q03 1-Z01-Q-TMS

3-S00-1 3-S00-2

3-S00

Step 1

Compound **1-Z01-J01** (5 g, 18.3 mmol) was dissolved in dichloromethane (50 mL) under nitrogen atmosphere. Triethylamine (5.5 g, 54.9 mmol) was added. The mixture was cooled to 0 °C. Acetic anhydride (2 g, 18.7 mmol) was added dropwise. The mixture was reacted overnight at room temperature. The mixture was with water (20 mL × 2). The aqueous phases were combined, adjusted to pH 1 with concentrated hydrochloric acid and extracted with ethyl acetate (50 mL × 3). After concentration, compound **1-Z01-Q-02** (4.7 g, yield: 91%) was obtained.

MS-ESI: m/z 280.2 $[M+H]^+$.

Step 2

Compound **1-Z01-Q-02** (4.5 g, 16.1 mmol) and trimethylsilyl ethanol (5.7 g, 48.3 mmol) were dissolved in dichloromethane (5 mL) under nitrogen atmosphere. The solution was cooled to 0 °C. Thionyl chloride (1.9 g, 16.1 mmol) was added. The mixture was reacted overnight at room temperature. After concentration, the residue was purified by column chromatography to give compound **1-Z01-Q-03** (1.6 g, yield: 26%).

MS-ESI: m/z 380.3 $[M+H]^+$.

$^1$H NMR (400 MHz, $CDCl_3$) $\delta$ 7.39-7.28 (m, 5H), 6.20 (d, $J$ = 7.6 Hz, 1H), 5.14 (s, 2H), 4.61-4.63 (m, 1H), 4.19-4.04 (m, 2H), 2.21-2.26 (m, 3H), 2.02-1.94 (m, 4H), 1.02-0.90 (m, 2H), 0.05-0.02 (m, 9H).

Step 3

Compound **1-Z01-Q-03** (0.9 g, 2.3 mmol) was dissolved in tetrahydrofuran (10 mL). Wet Pd/C (180 mg, 10 wt.%) was added. The system was purged three times with hydrogen. The mixture was reacted overnight at room temperature. The mixture was filtered and concentrated to give compound **1-Z01-Q-TMS** (621 mg, yield: 93%).

MS-ESI: m/z 290.3 $[M+H]^+$.

$^1$H NMR (400 MHz, $CDCl_3$) $\delta$ 6.75 (d, $J$ = 6.9 Hz, 1H), 4.54-4.42 (m, 1H), 4.22-4.07 (m, 2H), 2.56-2.32 (m, 2H), 2.24-2.12 (m, 1H), 2.09-1.90 (m, 4H), 1.03-0.90 (m, 2H), 0.00 (s, 9H).

Step 4

Compound **1-Z01-Q-TMS** (400 mg, 1.3 mmol) was dissolved in dichloromethane (20 mL) under nitrogen atmosphere. DMAP (56 mg, 0.46 mmol), 3-TMS (962 mg, 0.92 mmol) and DCC (285 mg, 1.3 mmol) were added. The mixture was reacted overnight at room temperature. Additional **1-Z01-Q-TMS** (150 mg, 0.5 mmol) and DCC (143 mg, 0.6 mmol) were added. The mixture was reacted overnight at room temperature. After filtration and concentration, the residue was purified by column chromatography to give compound **3-S00-1** (560 mg, yield: 33%).

MS-ESI: m/z 1314.3 $[M+H]^+$.

Step 5

Compound **3-S00-1** (560 mg, 0.426 mmol) was dissolved in tetrahydrofuran (8 mL) under nitrogen atmosphere. TBAF (4.3 mL, 4.3 mmol, 1.0 mol/L in THF) was added. The mixture was reacted for 2 h. The mixture was quenched with water (10 mL) and extracted with ethyl acetate (50 mL × 3). After concentration, the residue was purified by column chromatography to give compound **3-S00-2** (420 mg, yield: 86%).

MS-ESI: m/z 1142.3 $[M+H]^+$.

Step 6

Compound **3-S00-2** (0.074 g, 0.065 mmol) and PyBOP (0.041 g, 0.078 mmol) were dissolved in anhydrous *N,N*-dimethylformamide (1 mL) under nitrogen atmosphere. A solution of dendrimer 1-PEG2K (0.100 g, 1.30 μmol) and NMM (0.026 g, 0.258 mmol) in anhydrous *N,N*-dimethylformamide (1 mL) was added dropwise. The mixture was reacted at room temperature for 2 h. The reaction mixture was diluted with acetonitrile, then purified by ultrafiltration with acetonitrile (0.3 L, 25 V) in an ultrafiltration (10 KD, Hydrosart®) device and concentrated to give a crude product (0.124 g). The product was dissolved in pure water. The solution was filtered through a filter membrane (0.22 μm) and lyophilized to give compound **3-S00** (0.12 g, yield: 87%).

$^1H$ NMR indicated 30 drug molecules/dendrimer. The actual molecular weight was approximately 107.3 kDa (27.12% wt.% compound 3).

$^1H$ NMR (400 MHz, $CD_3OD$) δ 6.40-8.52 (m, 609H), 4.19-4.68 (m, 184H), 3.40-4.10 (m, 5900H), 3.33-3.36 (s, 102H), 2.79-3.20 (m, 102H), 2.22-2.74 (m, 146H), 0.61-1.92 (m, 767H).

Example 15: Preparation of Compound **3-T00**

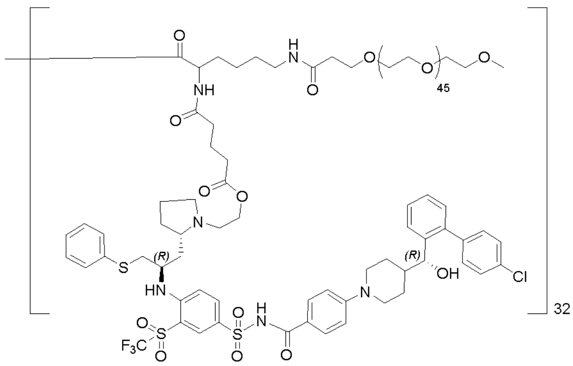

**3-T00**

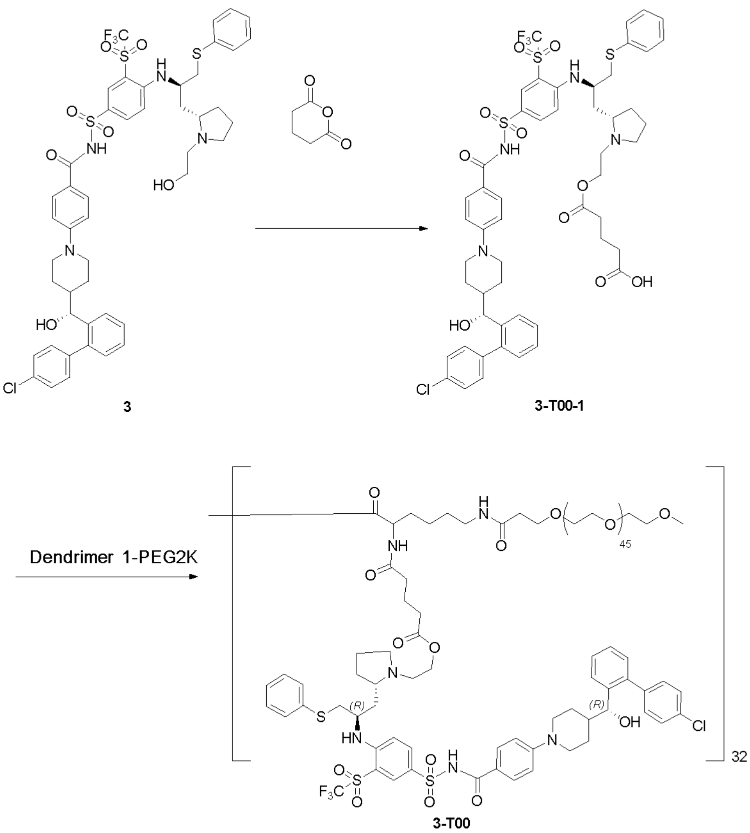


3 3-T00-1

3-T00

Step 1

Compound **3** (1.0 g, 1.02 mmol) was dissolved in dichloromethane (20 mL) under nitrogen atmosphere. A solution of NMM (322 mg, 3.18 mmol) and *N,N*-diisopropylethylamine (89 mg, 0.69 mmol) in dichloromethane (5 mL) was added dropwise. Glutaric anhydride (141 mg, 1.23 mmol) was added. The mixture was reacted at room temperature for 20 h. Glutaric anhydride (94 mg, 0.82 mmol) was added. The mixture was reacted at room temperature for 3 h. After concentration, the residue was purified by column chromatography to give compound **3-T00-1** (1.030 g, yield: 92%).

MS-ESI: m/z 1085.2 $[M+H]^+$.

Step 2

Compound **3-T00-1** (0.352 g, 0.325 mmol) and PyBOP (0.203 g, 0.390 mmol) were dissolved in anhydrous *N,N*-dimethylformamide (5 mL) under nitrogen atmosphere. A solution of dendrimer 1-PEG2K (0.500 g, 6.50 μmol) and NMM (0.131 g, 1.300 mmol) in anhydrous *N,N*-dimethylformamide (5 mL) was added dropwise. The mixture was reacted at room temperature for 2 h. The reaction mixture was diluted with acetonitrile, then purified by ultrafiltration with acetonitrile (0.5 L, 25 V) in an ultrafiltration (10 KD, Hydrosart®) device and concentrated to give a crude product (0.585 g). The product was dissolved in pure water. The solution was filtered through a filter membrane (0.22 μm) and lyophilized to give compound **3-T00** (0.580 g, yield: 84%).

$^1H$ NMR indicated 31 drug molecules/dendrimer. The actual molecular weight was approximately 106.6 kDa (28.19% wt.% compound 3).

$^1H$ NMR (400 MHz, $CD_3OD$) δ 6.40-8.52 (m, 623H), 4.19-4.67 (m, 173H), 3.40-4.10 (m, 5900H), 3.33-3.36 (s, 95H), 2.75-3.10 (m, 73H), 2.22-2.74 (m, 217H), 0.61-1.92 (m, 613H).

Biological Evaluation

Test Example 1: Pharmacokinetic Study of Administering Different Compounds to Rats by Single Intravenous Injections

1. Test samples

Docetaxel (G1), and the compounds of the present disclosure 1-Z00 (G2), 1-Z00-J (G3), 1-Z00-K (G4), 1-Z00-M (G5) and 1-C00 (G6).

Sample preparation:

(1) Preparation of docetaxel: commercially available docetaxel injection (0.5 mL:20 mg) was diluted to 4 mg/mL with normal saline;
(2) Preparation of other test compounds: the compounds were dissolved in DMSO with a final concentration of 5%, and the solutions were diluted to 4 mg/mL with normal saline.

2. Test animals

Female SD rats, weighing 160-180 g, 6 weeks old, SPF

3. Method

Blood was collected at different time points after intravenous administration (dose: 10 mg/kg) (G1 group: 0 h, 0.0833 h, 0.5 h, 1 h, 4 h, 8 h and 24 h; G2-6 groups: 0 h, 0.0833 h, 1 h, 4 h, 8 h, 24 h, 48 h, 96 h and 168 h) and anticoagulated with EDTAK2. Plasma samples were separated, and the esterase inhibitor DDVP was added. The samples were stored at -80 °C and tested for docetaxel level (N = 3).

Table 1. Grouping and administration regimen

| Group | Test compound | Number Female | Dose | Concentration | Dosing volume | Route of administration | Sample type |
|---|---|---|---|---|---|---|---|
| | | | (mg/kg) | (mg/mL) | (mL/kg) | | |
| G1 | Docetaxel | 3 | 10 | 2 | 5 | IV | Plasma |
| G2 | 1-Z00 | 3 | 10 | 2 | 5 | IV | Plasma |
| G3 | 1-Z00-J | 3 | 10 | 2 | 5 | IV | Plasma |
| G4 | 1-Z00-K | 3 | 10 | 2 | 5 | IV | Plasma |
| G5 | 1-Z00-M | 3 | 10 | 2 | 5 | IV | Plasma |
| G6 | 1-C00 | 3 | 10 | 2 | 5 | IV | Plasma |

4. Results

As shown in Table 2 below and FIG. 1, after intravenous administration, the 10 mg/kg 1-Z00-J group, 1-Z00-K group and 1-Z00-M group all had significantly better $AUC_{0-t}$ than the other groups, and the 1-Z00-J group and 1-Z00-M group had lower plasma clearance, which indicates a better sustained-release effect.

Table 2. PK parameters of each group

| PK parameters | | Docetaxel G1 | 1-Z00 G2 | 1-Z00-JG 3 | 1-Z00-K G4 | 1-Z00-M G5 | 1-C00 G6 |
|---|---|---|---|---|---|---|---|
| Dose | $mg·kg^{-1}$ | 10 | | | | | |
| $K_{el}$ | $h^{-1}$ | 0.3 | 0.0 | 0.0 | 0.0 | 0.0 | 0.0 |
| $t_{1/2}$ | h | 2.1 | 44.1 | 22.1 | 42.3 | 29.7 | 39.0 |
| $C_{max}$ | $ng·mL^{-1}$ | 1985.3 | 1637.7 | 9081.0 | 5064.0 | 16178.0 | 620.7 |
| $C_0$ | $ng·mL^{-1}$ | 2838.9 | 1526.5 | 8109.7 | 4083.4 | 16061.6 | 710.0 |
| $AUC_{0-t}$ | $h·ng·mL^{-1}$ | 1395.2 | 27892.4 | 180709.7 | 142123.3 | 513567.1 | 5750.3 |
| $AUC_{0-inf}$ | $h·ng·mL^{-1}$ | 1484.4 | 29544.1 | 181546.3 | 153479.4 | 522896.4 | 6093.5 |
| $AUMC_{0-t}$ | $h·h·ng·mL^{-1}$ | 1669.3 | 1104019.6 | 3946180.0 | 5110841.0 | 17722784.5 | 254075.5 |
| $AUMC_{0-inf}$ | $h·h·ng·mL^{-1}$ | 2663.5 | 1490217.7 | 4115545.4 | 7750227.2 | 19714299.7 | 327079.5 |

(continued)

| PK parameters | | Docetaxel G1 | 1-Z00 G2 | 1-Z00-JG 3 | 1-Z00-K G4 | 1-Z00-M G5 | 1-C00 G6 |
|---|---|---|---|---|---|---|---|
| CL | $mL \cdot kg^{-1} \cdot min^{-1}$ | 115.5 | 5.7 | 0.9 | 1.1 | 0.3 | 28.6 |
| $MRT_{IV}$ | h | 1.8 | 50.6 | 23.3 | 51.1 | 37.9 | 56.8 |
| Vdss | $L \cdot kg^{-1}$ | 12.1 | 17.5 | 1.4 | 3.4 | 0.7 | 102.4 |

5. Conclusion

Compared to the common docetaxel injection which demonstrated rapid plasma clearance and low exposure, 1-Z00-J, 1-Z00-K and 1-Z00-M all demonstrated significant sustained-release effect and higher exposure. Compared to 1-C00 and 1-Z00, the compounds of the present disclosure have significantly improved drug exposure and lower drug clearance.

Test Example 2: Pharmacokinetic Study of Administering Different Compounds to Beagle Dogs by Single Intravenous Injections

1. Test samples

The compounds of the present disclosure 1-B00, 1-Z00 and 1-Z00-J, and docetaxel Sample preparation:
1-B00, 1-Z00 and 1-Z00-J: to the test compounds (84 mg/vial) was added an appropriate volume of normal saline to obtain 0.6 mg/mL solutions for intravenous injection.

Docetaxel (84 mg/vial, commercially available) was dissolved in 5% (final volume) DMSO + 30% PEG300 + 5% Tween 80 + 60% deionized water to obtain 0.6 mg/mL formulations for intravenous injection.

2. Test animals

Beagle dogs, non-naïve, sourced from Medicilon: 999M-004.

3. Method

Method of administration: the weight was measured and the dosing amount was calculated based on the weight. The drugs were slowly administered by intravenous injection. Blood was collected from the jugular vein or by other suitable means, about 1 mL per sample. Plasma samples were collected before administration and at different time points after administration and tested for free docetaxel level in plasma. For the docetaxel group, collection was performed before administration and at 0.083 h, 0.5 h, 1 h, 2 h, 4 h, 6 h, 8 h and 24 h after administration. For the other test compound groups, collection was performed before administration and at 0.083 h, 1 h, 4 h, 8 h, 24 h, 48 h, 72 h, 96 h, 120 h, 144 h and 168 h after administration.

Table 3. Administration regimen

| Group | Test compound | Number Male + female | Dose (mg/kg) | Concentration (mg/mL) | Dosing volume (mL/kg) | Route of administration | Sample type |
|---|---|---|---|---|---|---|---|
| 1 | 1-B00 | 1+1 | 3 | 0.6 | 5 | IV | Plasma |
| 2 | 1-Z00 | 1+1 | 3 | 0.6 | 5 | IV | Plasma |
| 3 | 1-Z00-J | 1+1 | 3 | 0.6 | 5 | IV | Plasma |
| 4 | Docetaxel | 1+1 | 3 | 0.6 | 5 | IV | Plasma |

4. Results

The pharmacokinetics of each group are shown in Table 4 below and in FIGs. 2 and 3.

Table 4. Pharmacokinetics

| PK parameters | | Mean | | | |
|---|---|---|---|---|---|
| | | 1-B00 | 1-Z00 | 1-Z00-J | Docetaxel |
| Dose | $mg \cdot kg^{-1}$ | 3 | 3 | 3 | 3 |
| $K_{el}$ | $h^{-1}$ | 0.0225 | 0.0140 | 0.0460 | 0.0515 |
| $t_{1/2}$ | h | 31.0 | 50.1 | 15.9 | 13.5 |
| $AUC_{0-168}$ | $h \cdot ng \cdot mL^{-1}$ | 4078 | 10324 | 25819 | 1892 |
| $AUC_{0-inf}$ | $h \cdot ng \cdot mL^{-1}$ | 4228 | 11295 | 26633 | 2039 |
| $AUMC_{0-t}$ | $h \cdot h \cdot ng \cdot mL^{-1}$ | 200865 | 590202 | 846908 | 3854 |
| $AUMC_{0-inf}$ | $h \cdot h \cdot ng \cdot mL^{-1}$ | 232853 | 822734 | 948715 | 10234 |
| CL | mL/min/kg | 11.9 | 4.40 | 1.90 | 24.6 |
| $MRT_{PO}$ | h | 54.6 | 72.8 | 35.5 | 5.02 |
| Vdss | L/kg | 38.7 | 19.4 | 4.00 | 7.39 |

As can be seen from the results, 1-Z00-J has higher *in vivo* exposure and lower drug clearance than docetaxel and 1-Z00.

Test Example 3: Pharmacodynamics and Drug Efficacy Accompanied by Hematological Evaluations of Subcutaneous Xenograft Tumor Model of Human Lung Cancer Cell A549 in BALB/C Nude Mice

1. Test samples

Docetaxel, 1-Z00 and 1-Z00-J

Samples are prepared according to Table 5 below.

Table 5. Preparation of solutions of test samples and control

| Test samples /control | Stock solution (mg/kg) | Working solution (mg/mL) | Preparation | State of prepared sample | Frequency of preparation | Storage of prepared sample |
|---|---|---|---|---|---|---|
| Control group (6% DMSO + 94% normal saline) | - | - | 0.24 mL of DMSO was added to 3.76 mL of normal saline, and the mixture was well shaken and mixed. | Solution | Freshly prepared before use | 4°C |
| Docetaxel (DTX) (formulation for 1st administration) | 20 | 2 | To the test compound (10 mg/vial) was added 0.3 mL of DMSO, and the compound was dissolved by vortexing to obtain 33.33 mg/mL working stock solutions, which were then diluted with 4.7 mL of normal saline to obtain 2 mg/mL formulations for intravenous injection. | Solution | Freshly prepared before use | 4°C |
| Docetaxel | 20 | 2 | 0.5 mL of DMSO was added to | Solution | Freshly | 4°C |

(continued)

| Test samples /control | Stock solution (mg/kg) | Working solution (mg/mL) | Preparation | State of prepared sample | Frequency of preparation | Storage of prepared sample |
|---|---|---|---|---|---|---|
| (DTX) (formulation for 2nd, 3rd and 4th administration) | | | 10 mg of DTX; 0.3 mL of 20.0 mg/mL clear DMSO stock solution was added to 1200 μL of PEG300, and the solution was well mixed; 150 μL of Tween-80 was added, and the solution was well mixed; 1350 μL of normal saline was then added to bring the solution to a volume of 3 mL. | | prepared before use | |
| 1-Z00 (99.3%) | 20 | 2 | To the test compound (10 mg/vial) was added 0.3 mL of DMSO, and the compound was dissolved by vortexing to obtain 33.33 mg/mL working stock solutions, which were then diluted with 4.667 mL of normal saline to obtain 2 mg/mL formulations for intravenous injection. | Solution | Freshly prepared before use | 4°C |
| 1-Z00-J (99.8%) | 10 | 1 | 2 mL of 2 mg/mL 1-Z00-J was diluted with 2 mL of carrier, and the dilution was well mixed by vortexing to obtain a 1 mg/mL formulation for intravenous injection. | Solution | Freshly prepared before use | 4°C |

2. Experimental animals

BALB/C nude mice, female, 6-7 week old (the age when tumor cell inoculation was performed), weighing 16.5-22.6 g, purchased from GemPharmatech Co., Ltd., certificate No. 320727210100243463. Housing environment: SPF.

3. Method

3. 1. Cell culture

A549 cells (National Collection of Authenticated Cell Cultures/Cell Bank of Shanghai Institutes for Biological Sciences (cellbank.org.cn, SIBS)) were cultured in an F12K medium (Gibco) containing 10% fetal bovine serum. Cells were collected when growing at the exponential phase and resuspended in PBS to a suitable concentration for use in subcutaneous tumor inoculation in mice.

3.2. Animal modeling and random grouping

$5 \times 10^6$ A549 cells were subcutaneously inoculated into the right side of female mice (inoculation volume: 0.1 mL/mouse). The day of inoculation was defined as day 0. When the mean tumor volume was 142.96 $mm^3$ (on day 13 after cell inoculation), the mice were randomly grouped according to tumor size as shown in Table 6 below (dosing volume: 10 μL/g).

Table 6. Experimental design for antitumor effect of drugs in subcutaneous tumor graft model of human lung cancer cell A549

| Group | Number of animals | Administration group | Dose (mg/kg) | Route of administration | Period of planned administration | Period of actual administration |
|---|---|---|---|---|---|---|
| 1 | 6 | Vehicle control group (G1) | 0 | i.v. | QW × 3-4 doses | QW × 4 doses |
| 2 | 9 | Docetaxel (G2) | 20 | i.v. | QW × 3-4 doses | QW × 4 doses |
| 3 | 12 | 1-Z00 (G3) | 20 | i.v. | QW × 3-4 doses | QW × 4 doses |
| 4 | 12 | 1-Z00-J (G4) | 10 | i.v. | QW × 3-4 doses | QW × 4 doses |

3.3. Observation and data collection

After tumor inoculation, routine monitoring items included the effect of tumor growth and treatment on the normal behavior of animals, including activity, food intake and water intake, weight gain or loss (body weight was measured twice a week), abnormalities in eyes, hair, etc. Clinical symptoms observed during the experiment were all recorded in the raw data. Tumor volume calculation formula: tumor volume ($mm^3$) = 1/2 × (a × $b^2$) (where a represents long diameter and b represents short diameter). In the experiment, data collection, including measurement of tumors' long and short diameters and animals' body weight, was performed using StudyDirectorTM (version: 3.1.399.19; supplier: Studylog System, Inc., S.San Francisco, CA, USA) software. Raw data measured by scales and vernier calipers were directly imported into the software. Any changes in the data were recorded in the software.

3.4. Treatment of weight loss in animals during experiment

When the body weight of an individual animal decreased by more than 15% (BWL ≥ 15%), medication for the individual animal/the group of animals was stopped until its/their body weight loss returned to within 15% (BWL < 15%).

When the body weight of an individual animal decreased by >20%, it was euthanized according to animal welfare.

3.5. Evaluation criteria for drug efficacy

Relative tumor proliferation rate, T/C (%), refers to the percentage value of the relative tumor volume or tumor weight of the treatment group and the control group at a certain time point. The calculation formula is: T/C% = TRTV / CRTV × 100% (TRTV: mean RTV of treatment group; CRTV: mean RTV of control group; RTV = Vt / V0, V0 is the tumor volume of the animal at the time of grouping, and Vt is the tumor volume of the animal after treatment).

The calculation formula for relative tumor inhibition, TGI (%), is TGI% = (1 - T/C) × 100% (T and C are the relative tumor volume (RTV) or tumor weight (TW) of the treatment group and control group, respectively, at a particular time point).

3.6. Endpoint

After the last administration, plasma was collected at the time points designated in Table 7 (EDTA anticoagulant tubes were used (the esterase inhibitor DDVP was added at the time of blood collection)). After plasma collection at the last time point was complete, the mice were euthanized, and tumors were collected, snap frozen and stored.

Table 7. Sampling time points in the experiment

| Group | Number of animals | Time point | Plasma | Tumor | Complete blood count |
|---|---|---|---|---|---|
| G1 | 6 | 24 h | √ | √(24 h) | √(24 h) |
| G2 | 2 | 5 min, 1 h | √√ | √(1h) | NA |
| | 2 | 4 h, 24 h | √√ | √(24 h) | √(24 h) |
| G3,4 | 3 | 5 min, 4 h | √√ | √(4 h) | NA |
| | 3 | 1 h, 24 h | √√ | √(24 h) | √(24 h) |
| | 3 | 8 h, 96 h | √√ | √(96 h) | √(96 h) |

(continued)

| Group | Number of animals | Time point | Plasma | Tumor | Complete blood count |
|---|---|---|---|---|---|
| | 3 | 48 h, 168 h | √√ | √(168 h) | NA |

3.7. Statistical analysis

All the results are expressed as mean tumor volume ± SEM (standard error of the mean). The best point of drug treatment (usually after the last administration) was selected for statistical analysis between different groups. A two-way ANOVA was used to determine whether there was a significant difference in tumor volume between a treatment group and the control group. $p < 0.05$ indicates a significant difference. Statistical analysis and graphing were both performed in the R language environment (version 3.3.1). All tests were two-tailed unless otherwise specified. A p value less than 0.05 was considered statistically significant.

4. Results

4.1. Results of the study of antitumor effect of drugs in subcutaneous tumor graft model of human lung cancer cell A549

The mice in the vehicle control group had a mean tumor volume of 1620.93 $mm^3$ on day 21 after administration began.

The docetaxel 20 mg/kg group had a mean tumor volume of 564.93 $mm^3$ on day 21 after administration began, which is statistically significantly different ($p < 0.001$) from that of the control group; the relative tumor inhibition TGI (%) was 66.24%.

The 1-Z00 20 mg/kg group had a mean tumor volume of 576.02 $mm^3$ on day 21 after administration began, which is statistically significantly different ($p < 0.001$) from that of the control group; the relative tumor inhibition TGI (%) was 64.85 %.

The 1-Z00-J 10 mg/kg group had a mean tumor volume of 285.12 $mm^3$ on day 21 after administration began, which is statistically significantly different ($p < 0.001$) from that of the control group; the relative tumor inhibition TGI (%) was 83.02 %.

The tumor growth of each treatment group and the control group is shown in Table 8 and Table 9 below and in FIG. 4.

Table 8. Changes over time in tumor volume of each group of mice in the subcutaneous tumor graft model of human lung cancer cell A549

| Days after administration began | Tumor volume ($mm^3$) ($\bar{x}$ ±S) | | | |
|---|---|---|---|---|
| | Group 1, vehicle control group | Group 2, docetaxel, 20 mg/kg | Group 3, 1-Z00, 20 mg/kg | Group 4, 1-Z00-J, 10 mg/kg |
| 0 | 143.34±7.82 | 142.49±6.17 | 142.84±7.03 | 142.52±7.5 |
| 3 | 231.32±29.22 | 187.27±18.12 | 215.84±17.99 | 223.45±25.73 |
| 7 | 472.18±66.41 | 294.09±32.19 | 313.97±22.48 | 330.43±45.11 |
| 10 | 637.59±53.01 | 362.73±41.13 | 403.11±31.07 | 369.83±46.8 |
| 14 | 960.02±63.06 | 460.59±68.72 | 527.36±39.14 | 383.93±44.18 |
| 17 | 1280.58±45.5 | 502.25±84.97 | 568.88±43.78 | 365.17±42.85 |
| 21 | 1620.93±97.74 | 564.93±108.61 | 576.02±46.05 | 285.12±31.79 |
| Note: data are expressed as "mean ± standard error". | | | | |

Table 9. Analysis of drug efficacy of each group in the subcutaneous tumor graft model of human lung cancer cell A549

| Experimental group | Day 21 after administration began | | | | |
|---|---|---|---|---|---|
| | Tumor volume ($\bar{x}$ ± S) | Relative tumor volume ($\bar{x}$ ± S) | TGI (%) | T/C (%) | P value (relative to control group) |
| Group 1, vehicle control group | 1620.93±97.74 | 11.45±0.88 | - | - | - |
| Group 2, docetaxel, 20 mg/kg | 564.93±108.61 | 3.87±0.61 | 66.24 | 33.76 | <0.001 |
| Group 3, 1-Z00, 20 mg/kg | 576.02±46.05 | 4.02±0.25 | 64.85 | 35.15 | <0.001 |
| Group 4, 1-Z00-J, 10 mg/kg | 285.12±31.79 | 1.94±0.12 | 83.02 | 16.98 | <0.001 |

Note: 1. data are expressed as "mean ± standard error";
2. T/C % = TRTV / CRTV × 100%; TGI% = (1-T/C) × 100%.
3. P value: obtained by using a two-way ANOVA to compare the tumor volume of a treatment group to the tumor volume of the control group.

4.2. Drug efficacy accompanied by hematological study results of drugs in subcutaneous tumor graft model of human lung cancer cell A549

As shown in Table 10 below, the white blood cells, lymphocytes and mononuclear cells in the blood of the group of mice intravenously injected with 20 mg/kg docetaxel were decreased by a factor of 2-3 compared to the vehicle control group, which indicates the significant lymphatic toxicity of docetaxel. Intravenous injection of 10 mg/kg 1-Z00-J was significantly safer for the lymphatic system than intravenous injection of 20 mg/kg docetaxel.

Table 10. Drug efficacy accompanied by hematological analysis in subcutaneous tumor graft model of human lung cancer cell A549

| Group | | White blood cell count WBC (10^9/L) | Lymphocyte LYMPH# (10^9/L) | Mononuclear cell MONO# (10^9/L) | Neutrophil NEUT# (10^9/L) | Eosinophil EO# (10^9/L) |
|---|---|---|---|---|---|---|
| Vehicle control group | 6 | 6.57±1.34 | 4.58±1.15 | 0.39±0.12 | 1.5±0.41 | 0.10±0.02 |
| Docetaxel 20 mg/kg | 2 | 2.52±0.08 | 1.58±0.39 | 0.11±0.02 | 0.81±0.47 | 0.02±0.02 |
| 1-Z00 20 mg/kg | 3 | 5.25±1.33 | 3.26±1.10 | 0.37±0.14 | 1.52±0.32 | 0.09±0.03 |
| 1-Z00-J 10 mg/kg | 3 | 5.45±2.08 | 3.85±1.63 | 0.40±0.25 | 1.41±0.24 | 0.07±0.04 |

Note: data are expressed as "mean ± standard deviation".

5. Conclusion

1-Z00-J had a significantly better effect than the common docetaxel injection and 1-Z00 when it was injected at half of the dose. Meanwhile, the 1-Z00-J molecule exhibited significantly reduced hematological toxicities such as reductions in lymphocytes and neutrophils compared to the docetaxel injection.

Test Example 4: Pharmacokinetic Study of Administering Different Compounds to Beagle Dogs by Single Intravenous Injections

1. Test samples

The compounds of the present disclosure: 3-L00, 3-M00, 3-N00, 3-O00, 3-R00 and 3-T00.

Sample preparation:

To the test compounds 3-L00, 3-M00, 3-N00, 3-O00 and 3-R00 (80 mg/vial) were added 5% (final volume) DMSO and 95% normal saline, and the test compounds were dissolved by vortexing to obtain 1.5 mg/mL formulations for administration.
3-T00: to the test compound (120 mg/vial) were added 5% (final volume) DMSO and 95% normal saline, and the test compound was dissolved by vortexing to obtain 1.5 mg/mL formulations for administration.

2. Test animals

Beagle dogs, non-naïve, sourced from Medicilon: 999M-004.

3. Method

Method of administration: the weight was measured and the dosing amount was calculated based on the weight; the administration regimen is shown in Table 11 below. The blood sampling time points were 0.083 h, 1 h, 4 h, 8 h, 24 h, 48 h, 72 h and 96 h after administration. Blood was collected from the jugular vein or by other suitable means at particular time points after administration, about 1 mL per sample. The collected blood samples were placed in EDTA-K2 anticoagulant blood collection tubes, and each group of blood samples needed to be specially treated with an esterase inhibitor. To the EDTA-K2 anticoagulant blood collection tubes was added 200 mM DDVP in an amount of 1/40, by volume, of the blood collected (if the amount of blood collected was 1 mL, 25 μL of 200 mM DDVP was added). The collected blood samples were placed on ice, and plasma was separated by centrifugation within 1 h (centrifugation conditions: 2200 g, 10 min, 2-8 °C). The drug concentration in the plasma was then determined and pharmacokinetic calculation was performed. The plasma samples were stored in a -70 °C freezer before testing.

Table 11. Administration regimen

| Test compound | Number Male | Dose | Concentration | Dosing volume | Route of administration | Sample type |
|---|---|---|---|---|---|---|
| | | (mg/kg) | (mg/mL) | (mL/kg) | | |
| 3-L00 | 2 | 3 | 1.5 | 2 | IV | Plasma |
| 3-M00 | 2 | 3 | 1.5 | 2 | IV | Plasma |
| 3-N00 | 2 | 3 | 1.5 | 2 | IV | Plasma |
| 3-O00 | 2 | 3 | 1.5 | 2 | IV | Plasma |
| 3-R00 | 2 | 3 | 1.5 | 2 | IV | Plasma |
| 3-T00 | 3 | 3 | 1.5 | 2 | IV | Plasma |

4. Results

The pharmacokinetic (PK) results of 3-L00, 3-M00, 3-N00, 3-O00, 3-R00 and 3-T00 in beagle dogs are shown in FIG. 5 and Table 12.

Table 12. Pharmacokinetics

| Compound | Dose (mg/kg) | $C_{max}$ (ng/ml) | $AUC_{all}$ (h*ng/ml) | $T_{1/2}$ (h) | Cl (ml/min/kg) |
|---|---|---|---|---|---|
| 3-L00 | 3 | 8740 | 67100 | 6.5 | 0.744 |
| 3-M00 | 3 | 1840 | 25800 | 13.9 | 1.92 |
| 3-N00 | 3 | 7670 | 64000 | 12.5 | 0.713 |

(continued)

| Compound | Dose (mg/kg) | $C_{max}$ (ng/ml) | $AUC_{all}$ (h*ng/ml) | $T_{1/2}$ (h) | Cl (ml/min/kg) |
|---|---|---|---|---|---|
| 3-O00 | 3 | 2710 | 55100 | 45.6 | 0.73 |
| 3-R00 | 3 | 6500 | 54600 | 6.68 | 0.911 |
| 3-T00 | 3 | 120 | 1680 | 33.4 | 23.6 |

The results show that the dendrimers 3-L00, 3-M00, 3-N00, 3-O00 and 3-R00 of the present disclosure had significantly higher exposure (AUC) than 3-T00, having significant sustained-release effect. The dendrimers of the present disclosure combines alkyl linkers which have nitrogen linkages on the side chains through the dendrimers. Compared to an all-carbon chain linker, the alkyl linkers can facilitate the release of small molecules.

Test Example 5: Effect of Single Intravenous Injection of Compound 3, 3-M00 and 3-O00 into Beagle Dogs on Platelet Count

1. Test samples

The compounds of the present disclosure: compound 3, 3-M00 and 3-O00.

Sample preparation:

To the test compound, compound 3 (120 mg/vial), were added 5% (final volume) DMSO, 5% Tween 80 and 90% normal saline, and the test compound was dissolved by vortexing to obtain 1.5 mg/mL formulations for administration.

To the test compounds 3-M00 and 3-O00 (80 mg/vial) were added 5% (final volume) DMSO and 95% normal saline, and the test compounds were dissolved by vortexing to obtain 1.5 mg/mL formulations for administration.

2. Test animals

Beagle dogs, non-naïve, sourced from Medicilon: 999M-004.

3. Method

Method of administration: the weight was measured and the dosing amount was calculated based on the weight; the administration regimen is shown in Table 13 below. The drugs were slowly administered by intravenous injection. Blood was collected from the jugular vein or by other suitable means, about 1 mL per sample. Whole blood was collected before administration and 24 h after administration and hematological tests was performed.

Table 13. Administration regimens for compound 3, 3-M00 and 3-O00

| Test compound | Number Male | Dose (mg/kg) | Concentration (mg/mL) | Dosing volume (mL/kg) | Route of administration | Sample type |
|---|---|---|---|---|---|---|
| Compound 3 | 3 | 3 | 1.5 | 2 | IV | Whole blood |
| 3-M00 | 2 | 3 | 1.5 | 2 | IV | Whole blood |
| 3-O00 | 2 | 3 | 1.5 | 2 | IV | Whole blood |

4. Results

Table 14. Effect of compounds 3, 3-M00 and 3-O00 on platelet (PLT) count in beagle dogs

| Compound | Day 1 (before administration) | Day 2 (24 h after administration) | Day 1 (before administration) | Day 2 (24 h after administration) | Difference (%) between PLT count in dogs at 24 h after administration and that before administration |
|---|---|---|---|---|---|
| | PLT $10^9$/L | PLT $10^9$/L | PLT $10^9$/L mean value | PLT $10^9$/L mean value | |
| Compound 3 | 355.00 | 34.00 | 355.00 | 34.00 | -90.42 |
| 3-M00 | 460.00 | 255.00 | 405.5 | 226 | -44.27 |
| | 351.00 | 197.00 | | | |
| 3-O00 | 330.00 | 230.00 | 306.5 | 193.5 | -36.87 |
| | 283.00 | 157.00 | | | |

The results show that the dendrimers 3-M00 and 3-O00 of the present disclosure can significantly reduce platelet reductions targeted by the small molecule compound 3. In this experiment, 3 beagle dogs in the small molecule compound 3 treatment group were intolerant to the single intravenous injection of 3 mg/kg compound 3, and 2 animals died. However, the beagle dogs in the groups treated with dendrimers 3-M00 and 3-O00 were not dead, which further indicates that the dendrimers of the present disclosure significantly improved the safety of the small molecule compound 3.

Test Example 6: Therapeutic Effect of 3-L00, 3-M00, 3-N00, 3-O00, 3-R00 and 3-T00 on Subcutaneously Grafted Tumors in Mice with Human Acute Lymphoblastic Leukemia RS4;11

1. Test samples

3-L00, 3-M00, 3-N00, 3-000, 3-R00 and 3-T00.

Sample preparation:

3-L00, 3-M00, 3-N00, 3-O00, 3-R00 and 3-T00: to each vial was added 0.25 mL DMSO to dissolve the compounds to obtain 20 mg/mL working stock solutions, and then 4.75 mL of normal saline was added; the compounds were dissolved by vortexing to obtain 1 mg/mL formulations for administration.

2. Test cells and animals

Human acute lymphoblastic leukemia RS4;11 was purchased from American Type Culture Collection. RS4;11 cells were cultured in a 10 cm petri dish with an RPMI 1640 culture medium (Gibco) containing 10% fetal bovine serum (Gibco) and penicillin and streptomycin in an incubator at 37 °C containing 5% $CO_2$. The cells were passaged twice a week, and then collected, counted and inoculated when in the exponential growth phase.

NOD-Scid mice, 5 weeks old, female, purchased from Beijing Huafukang Biotechnology Co., Ltd. with production license No.: SCXK (Beijing) 2019-0008, and certificate Nos.: 110322211100992176 and 110322211101069023. Housing environment: SPF.

The use and welfare of the laboratory animals were carried out in compliance with the provisions of Association for Assessment and Accreditation of Laboratory Animal Care, International (AAALAC). The health and death of the animals were monitored daily. Routine examinations included observation of the effect of the test compounds and drugs on the daily performance of the animals, such as behavioral activities, weight changes and appearance.

3. Method

Each mouse was inoculated subcutaneously with $1 \times 10^7$ RS4;11 cells. When tumors grew to 150~200 $mm^3$, the mice were grouped according to tumor volume and intravenously (IV) injected with the drugs once a week (QW), and a total of 4 doses were injected. The injection volume was 0.1 mL/10 g body weight; the doses and administration regimen are shown in Table 15 below. The experimental index was to investigate the influence of the drug on the tumor growth, and the specific index was T/C% or tumor growth inhibition (TGI%).

Tumor diameters were measured twice weekly with a vernier caliper and tumor volume (V) was calculated according to the following formula:

$$V = 1/2 \times a \times b^2$$

where a and b represent length and width, respectively.

$$T/C\ (\%) = (T - T_0) / (C - C_0) \times 100$$

where T and C are tumor volumes at the end of the experiment; $T_0$ and $C_0$ are tumor volumes at the beginning of the experiment.

$$\text{Tumor growth inhibition \% (TGI\%)} = 100 - T/C\ (\%).$$

Table 15. Administration regimen

| Group | Route | of administration |
|---|---|---|
| 3-L00, 10 mg/kg | D0, 7, 14, 20 | IV |
| 3-M00, 10 mg/kg | D0, 7, 14, 20 | IV |
| 3-N00, 10 mg/kg | D0, 7, 14, 20 | IV |
| 3-O00, 10 mg/kg | D0, 7, 14, 20 | IV |
| 3-R00, 10 mg/kg | D0, 7, 14, 20 | IV |
| 3-T00, 10 mg/kg | D0, 7, 14, 20 | IV |

Note: the mice were randomly grouped, and the time of the first administration was defined as D0; IV: intravenous injection.

4. Results

Table 16. Therapeutic effect of compounds on subcutaneously grafted tumors in mice bearing human acute lymphoblastic leukemia RS4; 11

| Group | Tumor growth inhibition (TGI) % |
|---|---|
| 3-L00, 10 mg/kg | 70.9 |
| 3-M00, 10 mg/kg | 63.2 |
| 3-N00, 10 mg/kg | 71.5 |
| 3-O00, 10 mg/kg | 67.6 |
| 3-R00, 10 mg/kg | 73.3 |
| 3-T00, 10 mg/kg | 0.36 |

The results show that in the subcutaneous tumor graft model of mice bearing human acute lymphoblastic leukemia RS4;11, 3-T00 failed to effectively release the small molecule compound 3, having no antitumor activity, while 3-L00, 3-M00, 3-N00, 3-O00 and 3-R00 had linkers that could effectively facilitate the release of the small molecule compound 3, having significant antitumor activity.

## Claims

**1.** A macromolecule, comprising:

i) a dendrimer D having surface amino groups, wherein at least two different terminal groups are covalently linked to the surface amino groups of the dendrimer;
ii) a first terminal group, which is a pharmaceutically active agent comprising a hydroxy, amino or sulfhydryl group or is a residue A thereof; and
iii) a second terminal group, which is a pharmacokinetic modifier;
wherein the first terminal group is covalently linked to a surface amino group of the dendrimer by a linker $-X_1-L-X_2-$; $X_1$ is a linking group of the linker to the pharmaceutically active agent or residue A thereof, $X_2$ is a linking group of the linker to dendrimer D, and $X_1$ and $X_2$ are both -C(O)-; L is a linear or branched $C_{1-10}$ alkylene, wherein the linear or branched $C_{1-10}$ alkylene is substituted with one or more substituents selected from the group consisting of deuterium, hydroxy, $C_{3-7}$ cycloalkyl, $C_{1-6}$ alkoxy, haloalkyl, haloalkoxy, halogen, nitro, cyano, acyl, sulfhydryl, sulfinyl, sulfonyl, $-NR_1R_2$, aryl, heteroaryl and heterocyclyl;
$R_1$ and $R_2$ are each independently selected from the group consisting of hydrogen, hydroxy, $C_{1-6}$ alkyl, cycloalkyl and $C_{1-6}$ alkoxy.

**2.** A macromolecule, comprising:

i) a dendrimer D having surface amino groups, wherein at least two different terminal groups are covalently linked to the surface amino groups of the dendrimer;
ii) a first terminal group, which is a pharmaceutically active agent comprising a hydroxy, amino or sulfhydryl group or is a residue A thereof; and
iii) a second terminal group, which is a pharmacokinetic modifier;
wherein the first terminal group is covalently linked to a surface amino group of the dendrimer by a linker $-X_1-L-X_2-$; $X_1$ is a linking group of the linker to the pharmaceutically active agent, $X_2$ is a linking group of the linker to the dendrimer, and $X_1$ and $X_2$ are both -C(O)-; L is a linear or branched $C_{1-10}$ alkylene, wherein the linear or branched $C_{1-10}$ alkylene is substituted with one or more substituents selected from the group consisting of deuterium, halogen, $-OR_1$, $-SR_1$, $-NR_1R_2$ and $-C(O)R_3$;
$R_1$ and $R_2$ are each independently selected from the group consisting of hydrogen, hydroxy, $C_{1-6}$ alkyl, $C_{3-7}$ cycloalkyl, $C_{1-6}$ alkoxy and $C(O)R_4$, and the $C_{1-6}$ alkyl, $C_{3-7}$ cycloalkyl and $C_{1-6}$ alkoxy are optionally substituted with one or more substituents selected from the group consisting of hydroxy, halogen, $C_{1-6}$ alkyl, $C_{1-6}$ alkoxy, $C_{2-6}$ alkenyl, $C_{2-6}$ alkynyl, $C_{1-6}$ haloalkyl, $C_{1-6}$ haloalkoxy, nitro, cyano, amino and $C_{1-6}$ alkylamino;
$R_3$ and $R_4$ are each independently selected from the group consisting of hydrogen, $C_1$-$_6$ alkyl, $C_1$-$_6$ haloalkyl and $C_1$-$_6$ alkoxy.

**3.** The macromolecule according to claim 2, wherein the linear or branched $C_{1-10}$ alkylene is substituted with one or more substituents selected from the group consisting of halogen, -ORi, -SRi and $-NR_1R_2$, wherein $R_1$ and $R_2$ are each independently selected from the group consisting of hydrogen, $C_{1-6}$ alkyl, $C_{3-7}$ cycloalkyl, $C_{1-6}$ alkoxy and $C(O)R_4$, and the $C_{1-6}$ alkyl, $C_{3-7}$ cycloalkyl and $C_{1-6}$ alkoxy are optionally substituted with one or more substituents selected from the group consisting of hydroxy, halogen, $C_{1-6}$ alkyl, $C_{1-6}$ alkoxy, $C_{1-6}$ haloalkyl, $C_{1-6}$ haloalkoxy, amino and $C_{1-6}$ alkylamino; $R_4$ is selected from the group consisting of hydrogen, $C_1$-$_6$ alkyl, $C_1$-$_6$ haloalkyl and $C_{1-6}$ alkoxy.

**4.** The macromolecule according to any one of claims 1 to 3, wherein the linear or branched $C_{1-10}$ alkylene is substituted with one or more $-NR_1R_2$, wherein $R_1$ and $R_2$ are each independently selected from the group consisting of hydrogen, $C_{1-6}$ alkyl, $C_{3-7}$ cycloalkyl, $C_{1-6}$ alkoxy and $C(O)R_4$, and the $C_{1-6}$ alkyl, $C_{3-7}$ cycloalkyl and $C_{1-6}$ alkoxy are optionally substituted with one or more substituents selected from the group consisting of hydroxy, halogen, $C_{1-6}$ alkyl, $C_{1-6}$ alkoxy, $C_{1-6}$ haloalkyl, $C_{1-6}$ haloalkoxy, amino and $C_{1-6}$ alkylamino; $R_4$ is selected from the group consisting of hydrogen, $C_{1-6}$ alkyl, $C_1$-$_6$ haloalkyl and $C_1$-$_6$ alkoxy.

**5.** The macromolecule according to any one of claims 1 to 4, wherein the linear or branched $C_{1-10}$ alkylene is substituted with one or more $-NR_1R_2$, wherein $R_1$ and $R_2$ are each independently selected from the group consisting of hydrogen, $C_{1-6}$ alkyl and $C(O)R_4$, and $R_4$ is selected from the group consisting of hydrogen, $C_1$-$_6$ alkyl, $C_1$-$_6$ haloalkyl and $C_{1-6}$ alkoxy; preferably $R_1$ and $R_2$ are each independently selected from the group consisting of hydrogen and $C_{1-6}$ alkyl, and $R_1$ and $R_2$ are not both hydrogen.

**6.** The macromolecule according to any one of claims 1 to 5, wherein the linear or branched $C_{1-10}$ alkylene is linear or branched $C_1$-$_6$ alkylene.

**7.** The macromolecule according to claim 1, wherein in linker $-X_1-L-X_2-$, $X_1$ is -C(O)- and is linked to the pharmaceutically

active agent or residue A thereof; $X_2$ is -C(O)- and is linked to a surface amino group of dendrimer D to form an amide bond; the macromolecule has a structure as shown below:

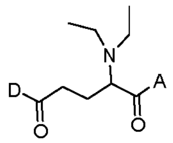 , 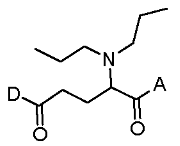 , 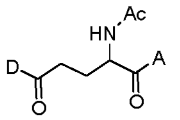 , 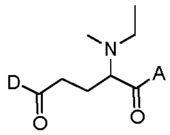 ,

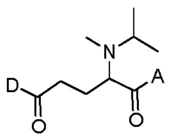 , 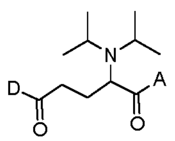 , 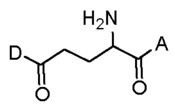 , 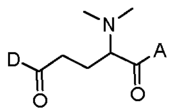

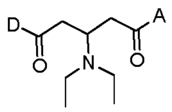 , 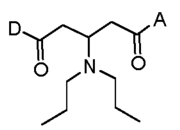 , 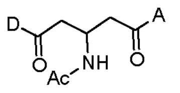 , 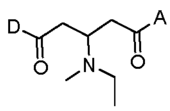 ,

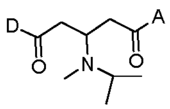 , 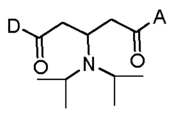 , 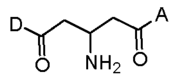 , 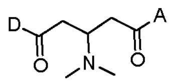

, 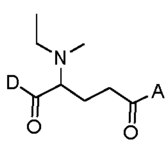 , 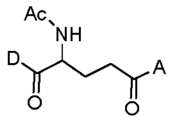 , 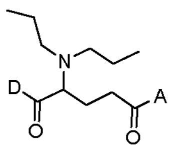 , 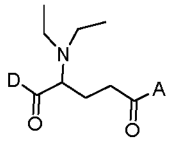

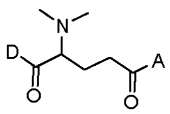 , 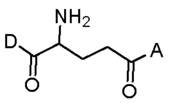 , 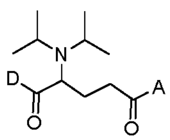 , 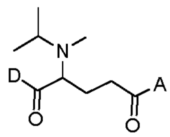

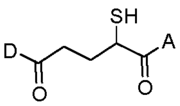 , 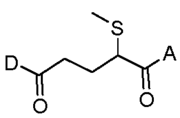 , 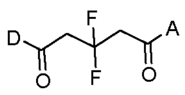 , 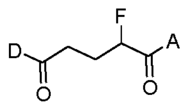 ,

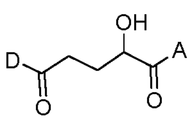 , 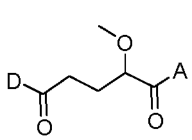 , 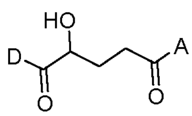 , 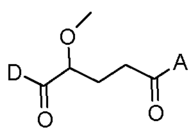

or

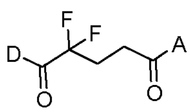 .

**8.** The macromolecule according to any one of claims 1 to 7, wherein the pharmaceutically active agent is selected from the group consisting of anesthetics, antacids, antibodies, anti-infectives, biologics, cardiovascular drugs, contrast agents, diuretics, hematinics, immunosuppressants, hormones and analogs, nutraceuticals, ophthalmic drugs, pain-treating agents, respiratory drugs, adjuvants, anabolics, antiarthritics, anticonvulsants, antihistamines, anti-inflammatory drugs, antiulcer drugs, behavior-modifying drugs, oncology drugs, central nervous system drugs, contraceptives, diabetes-treating drugs, fertility drugs, growth promoters, hemostatics, immunostimulants, muscle relaxants, obesity-treating agents, osteoporosis drugs, peptides, sedatives and tranquilizers, urinary tract acidifiers and vitamins, preferably oncology drugs.

**9.** The macromolecule according to any one of claims 1 to 7, wherein the pharmaceutically active agent is selected from the group consisting of taxanes, camptothecin derivatives, nucleosides, anthracyclines, ecteinascidin derivatives, proteasome inhibitors, microtubule inhibitors, BCL-2 inhibitors, BCL-$X_L$ inhibitors, selective inhibitor of nuclear export, antimetabolites, tyrosine kinase inhibitors, PLK1 inhibitors, CDK4/6 inhibitors, BTK inhibitors, non-steroidal hormone receptor antagonists and steroids, preferably from the group consisting of taxanes, camptothecin derivatives, BCL-2 inhibitors and BCL-$X_L$ inhibitors.

**10.** The macromolecule according to any one of claims 1 to 7, wherein the pharmaceutically active agent is selected from the group consisting of docetaxel, irinotecan, gemcitabine, capecitabine, decitabine, azacitidine, doxorubicin, epirubicin, trabectedin, lurbinectedin, bortezomib, eribulin, selinexor, venetoclax, tesetaxel, pemetrexed, cabazitaxel, cabozantinib, onvansertib, compound 2 and compound 3 below, and structural modifications of these drug molecules,

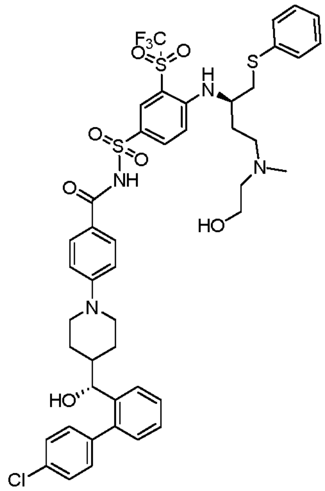

2

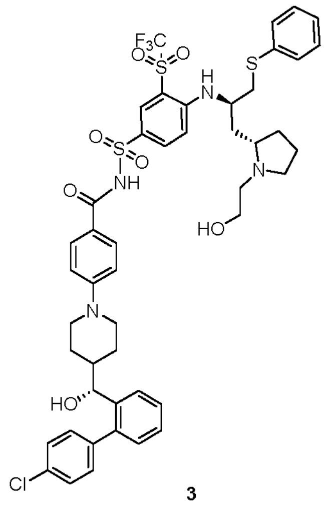

3

.

**11.** The macromolecule according to any one of claims 1 to 7, wherein the pharmaceutically active agent is docetaxel, compound 2 or compound 3, or a structural modification thereof.

**12.** The macromolecule according to any one of claims 1 to 11, wherein the pharmacokinetic modifier is selected from the group consisting of polyethylene glycol, polyethyloxazoline, polyvinylpyrrolidone, polypropylene glycol, a folate or a folate derivative of a ligand for a cell surface receptor, preferably polyethylene glycol.

**13.** The macromolecule according to claim 12, wherein the polyethylene glycol has a molecular weight in the range of 220 to 5500 Da, preferably 1000-5500 Da, more preferably 1000-2500 Da, and most preferably 1000-2300 Da.

**14.** The macromolecule according to any one of claims 1 to 13, wherein the dendrimer D is selected from a polylysine, polylysine analog, polyamidoamine (PAMAM), polyethyleneimine (PEI) or polyether hydroxylamine (PEHAM) dendrimer, preferably from a polylysine or polylysine analog.

**15.** The macromolecule according to claim 14, wherein the polylysine or polylysine analog comprises a core and 2-7 generations of lysine or a lysine analog.

**16.** The macromolecule according to claim 14, wherein the dendrimer D is selected from the group consisting of BHA-Lys[Lys]$_{16}$, BHALys[Lys]$_{32}$ and BHALys[Lys]$_{64}$.

**17.** A pharmaceutical composition, comprising the macromolecule according to any one of claims 1 to 16 and a pharmaceutically acceptable carrier.

**18.** Use of the macromolecule according to any one of claims 8 to 11 in manufacturing a medicament for treating tumors.

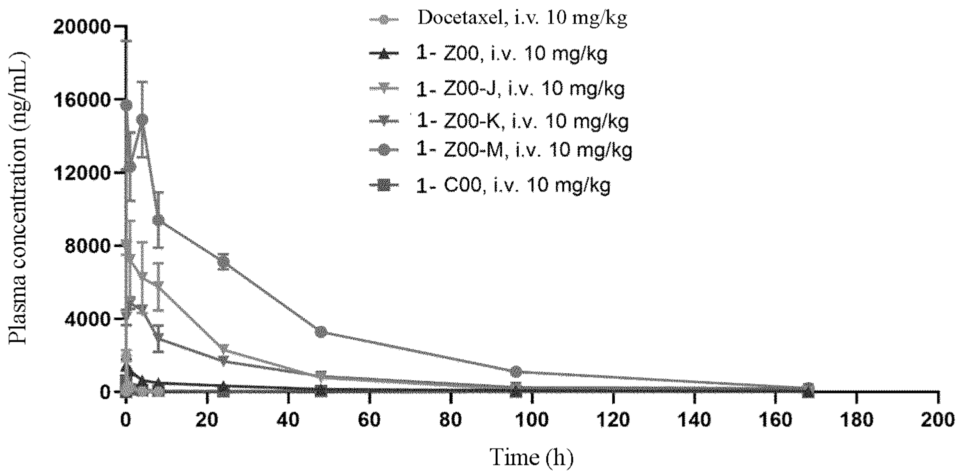


FIG. 1

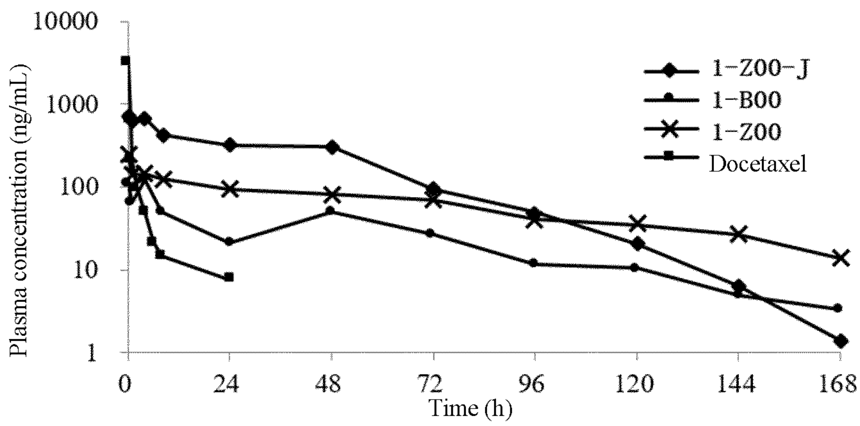


FIG. 2

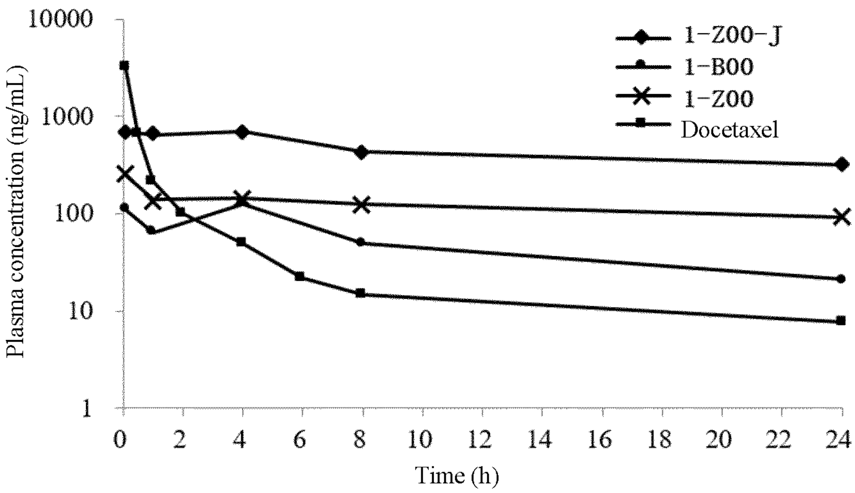


FIG. 3

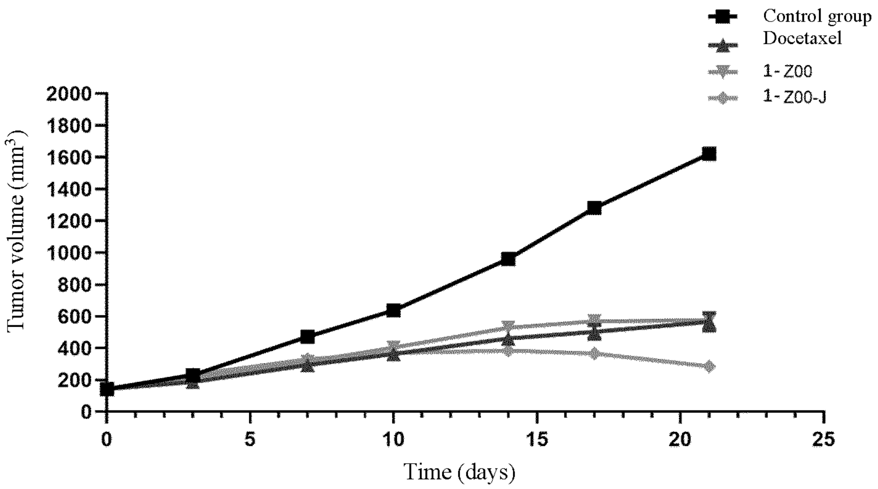


FIG. 4

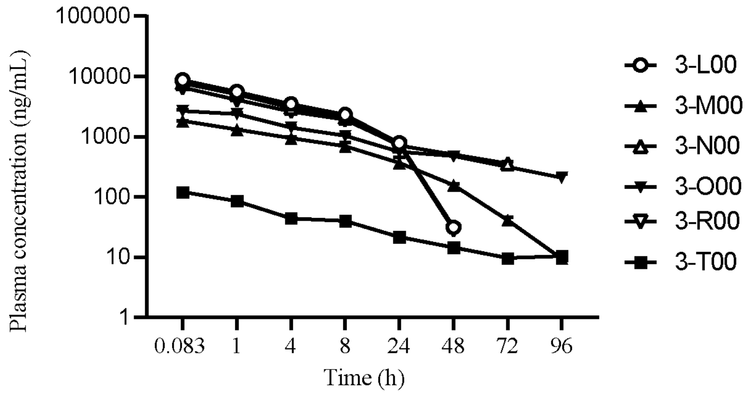


FIG. 5

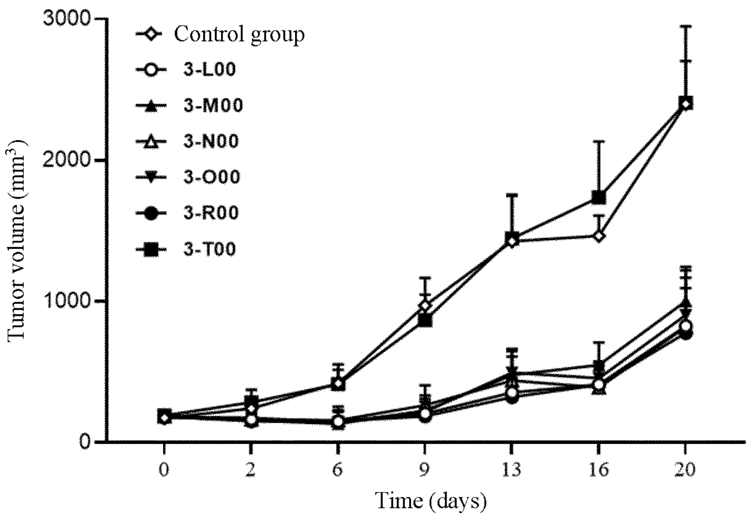


FIG. 6

**INTERNATIONAL SEARCH REPORT**

International application No.
**PCT/CN2021/114474**

**A. CLASSIFICATION OF SUBJECT MATTER**

A61K 47/64(2017.01)i; A61K 47/62(2017.01)i; A61P 35/00(2006.01)i; C08G 69/10(2006.01)i; A61K 47/60(2017.01)i; A61K 31/13(2006.01)i; A61K 31/195(2006.01)i

According to International Patent Classification (IPC) or to both national classification and IPC

**B. FIELDS SEARCHED**

Minimum documentation searched (classification system followed by classification symbols)

A61K 47/-, A61P 35/-, C08G 69/-, A61K 31/-

Documentation searched other than minimum documentation to the extent that such documents are included in the fields searched

Electronic data base consulted during the international search (name of data base and, where practicable, search terms used)

WPI, EPODOC, STN, CNKI, CNAPT: 树枝状, 树状, 超支化, 多支化, 赖氨酸, 缓释, 药物, 靶向, 连接子, 氨基, 胺基, 侧链, 酸性氨基酸, 谷氨酸, 氨基? 二酸, 取代, NR1R2, 星法马, 多西他赛, 喜树碱, 肿瘤, 缀合物, STARPHARMA, PAMAM, dendritic, dendrimer, hyperbranched, Lysine, glutamate, drug release, drug delivery, pharmaceutical, linker, targeting, amino, side chain, substitution, cancer, tumor

**C. DOCUMENTS CONSIDERED TO BE RELEVANT**

| Category* | Citation of document, with indication, where appropriate, of the relevant passages | Relevant to claim No. |
|---|---|---|
| X | CN 103796684 A (STARPHARMA PTY LTD.) 14 May 2014 (2014-05-14) description paragraphs 13-32, 40, 131, 307 | 1-3, 6-18 |
| A | CN 103796684 A (STARPHARMA PTY LTD.) 14 May 2014 (2014-05-14) description paragraphs 13-32, 40, 131, 307 | 4-5 |
| X | WO 2020102852 A1 (STARPHARMA PTY LTD.) 28 May 2020 (2020-05-28) description page 18 line 20- page 19 line 11, page 93 embodiment 2c | 1-3, 6-18 |
| A | WO 2020102852 A1 (STARPHARMA PTY LTD.) 28 May 2020 (2020-05-28) description page 18 line 20- page 19 line 11, page 93 embodiment 2c | 4-5 |
| A | CN 102917699 A (THE REGENTS OF THE UNIVERSITY OF MICHIGAN) 06 February 2013 (2013-02-06) description paragraphs [0011]-[0035], [0163]-[0165] | 1-18 |
| A | US 2010158850 A1 (THE REGENTS OF THE UNIVERSITY OF MICHIGAN) 24 June 2010 (2010-06-24) description paragraphs [0035-0037], [0054] | 1-18 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 November 2021** | **01 December 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

**INTERNATIONAL SEARCH REPORT**
**Information on patent family members**

International application No.
**PCT/CN2021/114474**

| Patent document cited in search report | | | Publication date (day/month/year) | Patent family member(s) | | | Publication date (day/month/year) |
|---|---|---|---|---|---|---|---|
| CN | 103796684 | A | 14 May 2014 | ES | 2834992 | T3 | 21 June 2021 |
| | | | | HU | E052285 | T2 | 28 April 2021 |
| | | | | CA | 2837979 | C | 30 March 2021 |
| | | | | US | 9744246 | B2 | 29 August 2017 |
| | | | | CA | 2837979 | A1 | 13 December 2012 |
| | | | | SI | 2729179 | T1 | 29 January 2021 |
| | | | | KR | 101941318 | B1 | 22 January 2019 |
| | | | | JP | 6158177 | B2 | 05 July 2017 |
| | | | | IN | 101DEN2014 | A | 15 May 2015 |
| | | | | EP | 2729179 | A1 | 14 May 2014 |
| | | | | US | 2017354739 | A1 | 14 December 2017 |
| | | | | US | 10265409 | B2 | 23 April 2019 |
| | | | | KR | 20140041678 | A | 04 April 2014 |
| | | | | HK | 1198356 | A1 | 10 April 2015 |
| | | | | AU | 2012267200 | A1 | 09 May 2013 |
| | | | | EP | 2729179 | A4 | 28 January 2015 |
| | | | | CN | 110124051 | A | 16 August 2019 |
| | | | | US | 2014171375 | A1 | 19 June 2014 |
| | | | | LT | 2729179 | T | 28 December 2020 |
| | | | | HR | P20201998 | T1 | 05 February 2021 |
| | | | | AU | 2012267200 | B2 | 25 February 2016 |
| | | | | CN | 103796684 | B | 25 June 2019 |
| | | | | PT | 2729179 | T | 25 November 2020 |
| | | | | JP | 2014520105 | A | 21 August 2014 |
| | | | | EP | 2729179 | B1 | 23 September 2020 |
| | | | | DK | 2729179 | T3 | 21 December 2020 |
| | | | | WO | 2012167309 | A1 | 13 December 2012 |
| WO | 2020102852 | A1 | 28 May 2020 | SG | 11202104239 X | A | 29 June 2021 |
| | | | | AU | 2019383466 | A1 | 27 May 2021 |
| | | | | CA | 3119993 | A1 | 28 May 2020 |
| | | | | CN | 113056469 | A | 29 June 2021 |
| CN | 102917699 | A | 06 February 2013 | EP | 2488172 | A2 | 22 August 2012 |
| | | | | US | 2012259098 | A1 | 11 October 2012 |
| | | | | EP | 2488172 | A4 | 13 August 2014 |
| | | | | WO | 2011059609 | A9 | 13 October 2011 |
| | | | | CA | 2777682 | A1 | 19 May 2011 |
| | | | | CA | 2777682 | C | 24 February 2015 |
| | | | | AU | 2010318637 | A1 | 31 May 2012 |
| | | | | US | 8945508 | B2 | 03 February 2015 |
| | | | | WO | 2011059609 | A3 | 25 August 2011 |
| | | | | WO | 2011059609 | A2 | 19 May 2011 |
| US | 2010158850 | A1 | 24 June 2010 | WO | 2010075423 | A3 | 18 November 2010 |
| | | | | WO | 2010075423 | A2 | 01 July 2010 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103796684 A
- CN 110312531 A
- WO 08017122 A
- WO 2012167309 A
- WO 2018154004 A
- WO 2012017251 A
- CN 103153954 B


### Non-patent literature cited in the description


- **XIANGYANG SHI et al.** *Sci China Mater,* 2018, vol. 61 (11), 1387-1403
- **V GAJBHIYE et al.** *Journal of Pharmacy and Pharmacology,* 2009, vol. 61, 989-1003
- **GREENE ; WUTS.** Protecting Groups in Organic Synthesis. 1999
- **KAMINSKAS et al.** *J Control. Release,* 2011
- *Journal of Controlled Release,* 2011, vol. 152, 241-248